(19) Europäisches Patentamt / European Patent Office / Office européen des brevets

(11) **EP 3 377 647 B1**

(12) **EUROPEAN PATENT SPECIFICATION**

(45) Date of publication and mention of the grant of the patent:
**11.03.2020 Bulletin 2020/11**

(51) Int Cl.:
*C12Q 1/6811* (2018.01)    *C12Q 1/6827* (2018.01)
*C12Q 1/6883* (2018.01)    *C07H 21/04* (2006.01)

(21) Application number: **16805259.5**

(22) Date of filing: **16.11.2016**

(86) International application number:
**PCT/US2016/062339**

(87) International publication number:
**WO 2017/087560 (26.05.2017 Gazette 2017/21)**

(54) **NUCLEIC ACIDS AND METHODS FOR DETECTING METHYLATION STATUS**

NUKLEINSÄUREN UND VERFAHREN ZUM NACHWEIS DES METHYLIERUNGSSTATUS

ACIDES NUCLÉIQUES ET PROCÉDÉS DE DÉTECTION DE L'ÉTAT DE MÉTHYLATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.11.2015 US 201562255947 P**

(43) Date of publication of application:
**26.09.2018 Bulletin 2018/39**

(73) Proprietor: **Progenity, Inc.**
**San Diego CA 92122 (US)**

(72) Inventors:
• **BUIS, Jeffrey**
  **Ann Arbor, MI 48102 (US)**
• **MANN, Tobias**
  **San Diego, CA 92122 (US)**
• **LALIBERTE, Julie, Catherine**
  **Ypsilanti, MI 48198 (US)**
• **KRUGER, Adele**
  **Livonia, MI 48154 (US)**

(74) Representative: **Glawe, Delfs, Moll**
**Partnerschaft mbB von**
**Patent- und Rechtsanwälten**
**Postfach 13 03 91**
**20103 Hamburg (DE)**

(56) References cited:
**WO-A1-2014/160736**

• **PALANISAMY RAMKUMAR ET AL: "Accurate detection of methylated cytosine in complex methylation landscapes.", ANALYTICAL CHEMISTRY 16 JUL 2013, vol. 85, no. 14, 16 July 2013 (2013-07-16), pages 6575-6579, XP002767396, ISSN: 1520-6882**
• **PALANISAMY RAMKUMAR ET AL: "Epiallele quantification using molecular inversion probes.", ANALYTICAL CHEMISTRY 1 APR 2011, vol. 83, no. 7, 1 April 2011 (2011-04-01), pages 2631-2637, XP002767397, ISSN: 1520-6882**
• **KHODOSEVICH KONSTANTIN ET AL: "Large-scale determination of the methylation status of retrotransposons in different tissues using a methylation tags approach.", NUCLEIC ACIDS RESEARCH 2004, vol. 32, no. 3, E31, 1 February 2004 (2004-02-01), pages 1-8, XP002767398, ISSN: 1362-4962**

**Description**

**Cross Reference to Related Application**

**Field of the Invention**

This disclosure relates to systems and methods for determining, *inter alia*, whether a subject has a predisposition to a disease state that is associated with the methylation state of a nucleic acid. This disclosure also relates to systems and methods for diagnosing a disease or condition in a subject, where the disease or condition is associated with the methylation state of a nucleic acid. This disclosure also relates to systems and methods for detecting the state of a disease or condition in a subject, where the disease or condition is associated with the methylation state of a nucleic acid.

**Background of the Invention**

The methylation state of an individual's genome can be correlated to diseases or conditions associated with methylation state. However, current methods are both labor-intensive and expensive. For example, whole genome bisulfite sequencing (WGBS) requires a large amount of input DNA for library preparation, and further requires deep sequencing for a high level of genomic coverage. Alternatively, targeted DNA methylation methods require an extensive, multiplex design that introduces bias and has limited breadth of genomic coverage. Moreover, current methods provide a picture of only the methylation state, ignoring other parameters, such as mutational landscape and genomic instability, which can be used in combination to diagnose an individual or determine whether the individual is predisposed to a disease or condition. Accordingly, there is a need for developing comprehensive genomic and epigenomic tests that are cost-effective, efficient, and have high sensitivities and specificities.

**Summary of the Disclosure**

Some embodiments are:

1. A method of determining the methylation state of a nucleic acid in a subject, the method comprising:

a) obtaining a nucleic acid sample isolated from a blood sample from the subject;
b) performing bisulfite conversion of the nucleic acid sample;
c) capturing a plurality of target sequences of interest in the nucleic acid sample obtained in step b) by using one or more populations of molecular inversion probes (MIPs) to produce a plurality of replicons,
wherein each of the MIPs in the population of MIPs comprises in sequence the following components:
first targeting polynucleotide arm - first unique molecular tag - polynucleotide linker - second unique molecular tag - second targeting polynucleotide arm;
wherein the first targeting polynucleotide arm in each of the MIPs is substantially complementary to a first repeat region, and the second targeting polynucleotide arm in each of the MIPs is substantially complementary to a second repeat region, further wherein the first and second repeat regions, respectively, flank each sequence in the plurality of target sequences of interest;
wherein the first and second unique targeting molecular tags in each of the MIPs in combination are distinct in each of the MIPs;
wherein the target sequence of interest in the plurality of target sequences of interest has one or more CpG sites;
wherein each CpG site has a corresponding known position within the target sequence of interest;
d) sequencing a plurality of MIPs amplicons that are amplified from the replicons obtained in step c); and
e) determining the number of occurrences of cytosine nucleotides at each corresponding CpG site within the MIPs amplicons sequenced at step d) to determine the methylation state of a nucleic acid.

2. A method of determining whether a subject has a predisposition to a disease or condition that is associated with the methylation state of a nucleic acid, the method comprising:

a) obtaining a nucleic acid sample isolated from a blood sample from the subject;
b) performing bisulfite conversion of the nucleic acid sample;
c) capturing a plurality of target sequences of interest in the nucleic acid sample obtained in step b) by using one or more populations of molecular inversion probes (MIPs) to produce a plurality of replicons,
wherein each of the MIPs in the population of MIPs comprises in sequence the following components:
first targeting polynucleotide arm - first unique molecular tag - polynucleotide linker - second unique molecular tag - second targeting polynucleotide arm;
wherein the first targeting polynucleotide arm in each of the MIPs is substantially complementary to a first repeat region, and the second targeting polynucleotide arm in each of the MIPs is substantially complementary to a second repeat region, further wherein the first and second repeat regions, respectively, flank each sequence in the plurality of target sequences of interest;
wherein the first and second unique targeting molecular tags in each of the MIPs in combination are distinct in each of the MIPs;
wherein each target sequence of interest in the plurality of target sequences of interest has one or more CpG sites;
wherein each CpG site has a corresponding known position within the target sequence of interest;
d) sequencing a plurality of MIPs amplicons that are amplified from the replicons obtained in step c);
e) determining the number of occurrences of cytosine nucleotides at each corresponding CpG site within the MIPs amplicons sequenced at step d);
f) determining a test ratio based on a first sum of the numbers of occurrences of cytosine nucleotides determined at step e) and a second sum of the known numbers of CpG sites in the plurality of target sequences of interest;
g) comparing the test ratio to a plurality of reference ratios that are computed based on reference nucleic acid samples isolated from reference subjects with the disease or condition that is associated with the methylation state of the nucleic acid; and
h) determining whether a subject is predisposed to the disease or condition based on the comparing in step g).

3. A method of diagnosing a disease or condition in a subject, said disease or condition being associated with the methylation state of a nucleic acid, the method comprising:

a) obtaining a nucleic acid sample isolated from a blood sample from the subject;
b) performing bisulfite conversion of the nucleic acid sample;
c) capturing a plurality of target sequences of interest in the nucleic acid sample obtained in step b) by using one or more populations of molecular inversion probes (MIPs) to produce a plurality of replicons,
wherein each of the MIPs in the population of MIPs comprises in sequence the following components:
first targeting polynucleotide arm - first unique molecular tag - polynucleotide linker - second unique molecular tag - second targeting polynucleotide arm;
wherein the first targeting polynucleotide arm in each of the MIPs is substantially complementary to a first repeat region, and the second targeting polynucleotide arm in each of the MIPs is substantially complementary to a second repeat region, further wherein the first and second repeat regions, respectively, flank each sequence in the plurality of target sequences of interest;
wherein the first and second unique targeting molecular tags in each of the MIPs in combination are distinct in each of the MIPs;
wherein each target sequence of interest in the plurality of target sequences of interest has one or more CpG sites;
wherein each CpG site has a corresponding known position within the target sequence of interest;
d) sequencing a plurality of MIPs amplicons that are amplified from the replicons obtained in step c);
e) determining the number of occurrences of cytosine nucleotides at each corresponding CpG site within the MIPs amplicons sequenced at step d);
f) determining a test ratio based on a first sum of the numbers of occurrences of cytosine nucleotides determined at step e) and a second sum of the known numbers of CpG sites in the plurality of target sequences of interest;
g) comparing the test ratio to a plurality of reference ratios that are computed based on reference nucleic acid samples isolated from reference subjects with the disease or condition that is associated with the methylation state of the nucleic acid; and
h) diagnosing the disease or condition in the subject based on the comparing in step g).

4. A method of detecting the state of a disease or condition in a subject, said disease or condition being associated with the methylation state of a nucleic acid, the method comprising:

a) obtaining a nucleic acid sample isolated from a blood sample from the subject;
b) performing bisulfite conversion of the nucleic acid sample;
c) capturing a plurality of target sequences of interest in the nucleic acid sample obtained in step b) by using one or more populations of molecular inversion probes (MIPs) to produce a plurality of replicons,
wherein each of the MIPs in the population of MIPs comprises in sequence the following components:
first targeting polynucleotide arm - first unique molecular tag - polynucleotide linker - second unique molecular tag - second targeting polynucleotide arm;
wherein the first targeting polynucleotide arm in each of the MIPs is substantially complementary to a first repeat region, and the second targeting polynucleotide arm in each of the MIPs is substantially complementary to a second repeat region, further wherein the first and second repeat regions, respectively, flank each sequence in the plurality of target sequences of interest;

wherein the first and second unique targeting molecular tags in each of the MIPs in combination are distinct in each of the MIPs;

wherein each target sequence of interest in the plurality of target sequences of interest has one or more CpG sites;

wherein each CpG site has a corresponding known position within the target sequence of interest;

d) sequencing a plurality of MIPs amplicons that are amplified from the replicons obtained in step c);

e) determining the number of occurrences of cytosine nucleotides at each corresponding CpG site within the MIPs amplicons sequenced at step d);

f) determining a test ratio based on a first sum of the numbers of occurrences of cytosine nucleotides determined at step e) and a second sum of the known numbers of CpG sites in the plurality of target sequences of interest;

g) comparing the test ratio to a plurality of reference ratios that are computed based on reference nucleic acid samples isolated from reference subjects with the disease or condition that is associated with the methylation state of the nucleic acid; and

h) detecting the state of the disease or condition in the subject based on the comparing in step g).

5. A method of differentiating nucleic acid species originating from a subject and one or more additional individuals, said subject and one or more additional individuals having differing methylation states of a nucleic acid, the method comprising:

a) obtaining a nucleic acid sample isolated from a blood sample from the subject, said blood sample comprising nucleic acids originating from the subject and the one or more additional individuals;

b) performing bisulfite conversion of the nucleic acid sample;

c) capturing a plurality of target sequences of interest in the nucleic acid sample obtained in step b) by using one or more populations of molecular inversion probes (MIPs) to produce a plurality of replicons, wherein each of the MIPs in the population of MIPs comprises in sequence the following components: first targeting polynucleotide arm - first unique molecular tag - polynucleotide linker - second unique molecular tag - second targeting polynucleotide arm;

wherein the first targeting polynucleotide arm in each of the MIPs is substantially complementary to a first repeat region, and the second targeting polynucleotide arm in each of the MIPs is substantially complementary to a second repeat region, further wherein the first and second repeat regions, respectively, flank each sequence in the plurality of target sequences of interest;

wherein the first and second unique targeting molecular tags in each of the MIPs in combination are distinct in each of the MIPs;

wherein each target sequence of interest in the plurality of target sequences of interest has one or more CpG sites;

wherein each CpG site has a corresponding known position within the target sequence of interest;

d) sequencing a plurality of MIPs amplicons that are amplified from the replicons obtained in step c);

e) determining the number of occurrences of cytosine nucleotides at each corresponding CpG site within the MIPs amplicons sequenced at step d);

f) determining a test ratio based on a first sum of the numbers of occurrences of cytosine nucleotides determined at step e) and a second sum of the known numbers of CpG sites in the plurality of target sequences of interest;

g) comparing the test ratio to a plurality of reference ratios that are computed based on reference nucleic acid samples isolated from individuals having differing methylation states of the nucleic acid; and

h) differentiating nucleic acid species originating from the subject and the one or more additional individuals based on the comparing in step g).

6. The method of embodiment 5, wherein the subject is a pregnant female and the one or more additional individuals is an unborn fetus.

7. The method of embodiment 5 or 6, wherein the blood sample is maternal plasma or maternal serum.

8. The method of embodiment 5, wherein the subject is a tissue transplant recipient.

9. The method of embodiment 8, wherein the one or more additional individuals is a tissue transplant donor.

10. A method of differentiating nucleic acid species originating from a first tissue and one or more additional tissues in a subject, said first tissue and one or more additional tissues having differing methylation states of a nucleic acid, the method comprising:

a) obtaining a nucleic acid sample isolated from a cell-free bodily fluid sample from the subject, said cell-free bodily fluid sample comprising nucleic acids originating from the first tissue and the one or more additional tissues;

b) performing bisulfite conversion of the nucleic acid sample;

c) capturing a plurality of target sequences of interest in the nucleic acid sample obtained in step b) by using one or more populations of molecular inversion probes (MIPs) to produce a plurality of replicons,

wherein each of the MIPs in the population of MIPs comprises in sequence the following components:

first targeting polynucleotide arm - first unique molecular tag - polynucleotide linker - second unique molecular tag - second targeting polynucleotide arm;

wherein the first targeting polynucleotide arm in each of the MIPs is substantially complementary to a first repeat region, and the second targeting polynucleotide arm in each of the MIPs is substantially complementary to a second repeat region, further wherein the first and second repeat regions, respectively, flank each sequence in the plurality of target sequences of interest;

wherein the first and second unique targeting molecular tags in each of the MIPs in combination are distinct in each of the MIPs;

wherein each target sequence of interest in the plurality of target sequences of interest has one or more CpG sites;

wherein each CpG site has a corresponding known position within the target sequence of interest;

d) sequencing a plurality of MIPs amplicons that are amplified from the replicons obtained in step c);

e) determining the number of occurrences of cytosine nucleotides at each corresponding CpG site within the MIPs amplicons sequenced at step d);

f) determining a test ratio based on a first sum of the numbers of occurrences of cytosine nucleotides determined at step e) and a second sum of the known numbers of CpG sites in the plurality of target sequences of interest;

g) comparing the test ratio to a plurality of reference ratios that are computed based on reference nucleic acid samples isolated from reference tissues having differing methylation states of the nucleic acid;

h) differentiating nucleic acid species originating from the first tissue and the one or more additional tissues based on the comparing in step g).

11. The method of embodiment 10, wherein the bodily fluid sample is a blood sample.

12. The method of embodiment 10 or 11, wherein the method further comprises (i) determining the percentage contribution of a particular tissue type to the nucleic acid; (ii) comparing the percentage contribution of the particular tissue type to a reference percentage contribution of the tissue type computed based on reference nucleic acid samples isolated from reference subjects with a disease or condition; and (iii) detecting the state of the disease or condition in the subject based on the comparing in step (ii).

13. The method of any one of embodiments 10-12, wherein the first tissue is selected from the group consisting of: liver, kidney, uterus, ovary, placenta, pancreas, colon, stomach, lung, and bladder.

14. A method of determining the methylation state of a nucleic acid and detecting copy number variation in a subject, the method comprising:

a) obtaining a nucleic acid sample isolated from a blood sample from the subject;

b) performing bisulfite conversion of the nucleic acid sample;

c) capturing a plurality of target sequences of interest in the nucleic acid sample obtained in step b) by using one or more populations of molecular inversion probes (MIPs) to produce a plurality of replicons,

wherein each of the MIPs in the population of MIPs comprises in sequence the following components:

first targeting polynucleotide arm - first unique molecular tag - polynucleotide linker - second unique molecular tag - second targeting polynucleotide arm;

wherein the first targeting polynucleotide arm in each of the MIPs is substantially complementary to a first repeat region, and the second targeting polynucleotide arm in each of the MIPs is substantially complementary to a second repeat region, further wherein the first and second repeat regions, respectively, flank each sequence in the plurality of target sequences of interest;

wherein the first and second unique targeting molecular tags in each of the MIPs in combination are distinct in each of the MIPs;

wherein the target sequence of interest in the plurality of target sequences of interest has one or more CpG sites;

wherein each CpG site has a corresponding known position within the target sequence of interest;

d) sequencing a plurality of MIPs amplicons that are amplified from the replicons obtained in step c);

e) determining the number of occurrences of cytosine nucleotides at each corresponding CpG site within the MIPs amplicons sequenced at step d) to determine the methylation state of a nucleic acid; and

f) determining the number of the unique MIPs amplicons sequenced at step d); determining a read density based at least in part on the number of unique MIP amplicon sequences; and detecting copy number variation by comparing the read density to a plurality of reference read densities that are computed based on reference nucleic acid samples isolated from reference subjects.

15. The method of embodiment 14, further comprising using the targeting molecular tags to remove duplicates to improve analysis.

16. The method of embodiment 14, further comprising determining the number of unique MIP amplicon sequences in defined regions to determine the read density.

17. A method of determining the methylation age of a subject, the method comprising:

a) obtaining a nucleic acid sample isolated from a blood sample from the subject;

b) performing bisulfite conversion of the nucleic acid sample;

c) capturing a plurality of target sequences of interest in the nucleic acid sample obtained in step b) by using one or more populations of molecular inversion probes (MIPs) to produce a plurality of replicons, wherein each of the MIPs in the population of MIPs comprises in sequence the following components: first targeting polynucleotide arm - first unique molecular tag - polynucleotide linker - second unique molecular tag - second targeting polynucleotide arm; wherein the first targeting polynucleotide arm in each of the MIPs is substantially complementary to a first repeat region, and the second targeting polynucleotide arm in each of the MIPs is substantially complementary to a second repeat region, further wherein the first and second repeat regions, respectively, flank each sequence in the plurality of target sequences of interest; wherein the first and second unique targeting molecular tags in each of the MIPs in combination are distinct in each of the MIPs; wherein each target sequence of interest in the plurality of target sequences of interest has one or more CpG sites; wherein each CpG site has a corresponding known position within the target sequence of interest;

d) sequencing a plurality of MIPs amplicons that are amplified from the replicons obtained in step c);

e) determining the number of occurrences of cytosine nucleotides at each corresponding CpG site within the MIPs amplicons sequenced at step d);

f) determining a test ratio based on a first sum of the numbers of occurrences of cytosine nucleotides determined at step e) and a second sum of the known numbers of CpG sites in the plurality of target sequences of interest;

g) comparing the test ratio to a plurality of reference ratios that are computed based on reference nucleic acid samples isolated from reference subjects; and

h) determining the methylation age of the subject based on the comparing in step g).

18. A method of determining the methylation age of a tissue in a subject, the method comprising:

a) obtaining a nucleic acid sample isolated from a cell-free bodily fluid sample from the subject, said cell-free bodily fluid sample comprising nucleic acids originating from the tissue;

b) performing bisulfite conversion of the nucleic acid sample;

c) capturing a plurality of target sequences of interest in the nucleic acid sample obtained in step b) by using one or more populations of molecular inversion probes (MIPs) to produce a plurality of replicons, wherein each of the MIPs in the population of MIPs comprises in sequence the following components: first targeting polynucleotide arm - first unique molecular tag - polynucleotide linker - second unique molecular tag - second targeting polynucleotide arm; wherein the first targeting polynucleotide arm in each of the MIPs is substantially complementary to a first repeat region, and the second targeting polynucleotide arm in each of the MIPs is substantially complementary to a second repeat region, further wherein the first and second repeat regions, respectively, flank each sequence in the plurality of target sequences of interest; wherein the first and second unique targeting molecular tags in each of the MIPs in combination are distinct in each of the MIPs; wherein each target sequence of interest in the plurality of target sequences of interest has one or more CpG sites; wherein each CpG site has a corresponding known position within the target sequence of interest;

d) sequencing a plurality of MIPs amplicons that are amplified from the replicons obtained in step c);

e) determining the number of occurrences of cytosine nucleotides at each corresponding CpG site within the MIPs amplicons sequenced at step d);

f) determining a test ratio based on a first sum of the numbers of occurrences of cytosine nucleotides determined at step e) and a second sum of the known numbers of CpG sites in the plurality of target sequences of interest;

g) comparing the test ratio to a plurality of reference ratios that are computed based on reference nucleic acid samples isolated from reference tissues; and

h) determining the methylation age of the tissue based on the comparing in step g).

19. The method of embodiment 18, wherein the bodily fluid sample is a blood sample.

20. The method of embodiment 18 or 19, wherein the subject is a pregnant female.

21. The method of any one of embodiments 18-20, wherein the tissue is placental tissue and the methylation age of the placental tissue is an indicator of the gestational age of an unborn fetus.

22. The method of any one of embodiments 1-4, 10, 11, 14 and 17, wherein the subject is a pregnant female.

23 The method of embodiment 22, wherein the nucleic acid sample comprises maternal and fetal nucleic acids.

24. The method of any one of embodiments 1-23, wherein the nucleic acid sample is DNA or RNA.

25. The method of embodiment 24, wherein the nucleic acid sample is genomic DNA.

26. The method of any one of embodiments 1-9, 11-17 and 19-25, wherein the blood sample is a whole blood sample, a plasma sample, or a serum sample.

27. The method of embodiment 24, wherein the blood sample is a plasma sample.

28. The method of any one of embodiments 1-27, wherein the length of the first targeting polynucleotide arm is between 14 and 30 bases.

29. The method of any one of embodiments 1-28, wherein the length of the second targeting polynucleotide arm is between 14 and 30 bases.

30. The method of any one of embodiments 1-29, wherein each of the targeting polynucleotide arms has a melting temperature between 45 °C and 66 °C.

31. The method of any one of embodiments 1-30, wherein each of the targeting polynucleotide arms has a GC content between 10% and 40%.

32. The method of any one of embodiments 1-31, wherein the length of the first unique molecular tag is between 4 and 15 bases.

33. The method of any one of embodiments 1-32, wherein the length of the second unique molecular tag is between 4 and 15 bases.

34. The method of any one of embodiments 1-33, wherein the polynucleotide linker is not substantially complementary to any genomic region of the subject.

35. The method of any one of embodiments 1-34, wherein the polynucleotide linker has a length of between 14 and 50 bases.

36. The method of any one of embodiments 1-35, wherein the polynucleotide linker has a melting temperature of between 45 °C and 85 °C.

37. The method of any one of embodiments 1-36, wherein the polynucleotide linker has a GC content between 30% and 66%.

38. The method of any one of embodiments 1-37, wherein the polynucleotide linker comprises at least one amplification primer binding site.

39. The method of embodiment 38, wherein the polynucleotide linker comprises a forward amplification primer binding site.

40. The method of embodiment 39, wherein the sequence of the forward amplification primer comprises the nucleotide sequence of
CCGTAATCGGGAAGCTGAAG (SEQ ID NO: 1).

41. The method of any one of embodiments 1-29, wherein the sequence of a reverse amplification primer comprises the nucleotide sequence of
GCACGATCCGACGGTAGTGT (SEQ ID NO:2).

42. The method of any one of embodiments 1-41, wherein the polynucleotide linker comprises the nucleotide sequence of
CTTCAGCTTCCCGATTACGGGCACGATCCGACGGTAGTGT (SEQ ID NO:3).

43. The method of any one of embodiments 1-42, wherein the first targeting polynucleotide arm comprises the nucleotide sequence of
CACTACACTCCAACCTAA (SEQ ID NO:4) or TTCTCCTACCTCAACCTC (SEQ ID NO:5).

44. The method of any one of embodiments 1-43, wherein the second targeting polynucleotide arm comprises the nucleotide sequence of
CAAAAAACTAAAACAAAA (SEQ ID NO:6) or CCAAACTAAAATACAATA (SEQ ID NO:7).

45. The method of any one of embodiments 1-44, wherein the MIP comprises the nucleotide sequence of CACTACACTCCAACCTAA(N1) CTTCAGCTTCCCGATTACGGGCACGATCCGACGGTAGTGT(N2) CAAAAAACTAAAACAAAA (SEQ ID NO:8) or TTCTCCTACCTCAACCTC(N1). CTTCAGCTTCCCGATTACGGGCACGATCCGACGGTAGTGT(N2) CCAAACTAAAATACAATA (SEQ ID NO:9), wherein (N1) represents the first unique molecular tag and (N2) represents the second unique molecular tag.

46. The method of any one of embodiments 1-45, wherein the population of MIPs has a concentration between 10 fM and 100 nM.

47. The method of any one of embodiments 1-46, wherein each of the MIPs replicons obtained in step c) is produced by:

i) the first and second targeting polynucleotide arms, respectively, hybridizing to the first and second repeat regions in the nucleic acid sample, respectively, wherein the first and second repeat regions flank a target sequence of interest; and

ii) after the hybridization, using a ligation/extension mixture to extend and ligate the gap region between the two targeting polynucleotide arms to form single-stranded circular nucleic acid molecules.

48. The method of any one of embodiments 1-47, wherein each of the MIPs replicons is a single-stranded circular nucleic acid molecule.

49. The method of any one of embodiments 1-48, wherein the sequencing step of d) comprises a next generation sequencing method.

50. The method of embodiment 49, wherein the next generation sequencing method comprises a massively parallel sequencing method.

51. The method of any one of embodiments 1-50, wherein the method comprises, before the sequencing step of d), a PCR reaction to amplify the MIPs replicons for sequencing.

52. The method of embodiment 51, wherein the PCR reaction is an indexing PCR reaction.

53. The method of embodiment 52, wherein the indexing PCR reaction introduces into each of the MIPs amplicons the following components: a pair of sequencing adapters comprising a unique molecular tag for multiplexed sequencing.

54. The method of embodiment 53, wherein the barcoded MIPs amplicons comprise in sequence the following components:

a first sequencing adaptor - a first sequencing primer binding site - the first unique targeting molecular tag - the first targeting polynucleotide arm - captured nucleic acid - the second targeting polynucleotide arm - the second unique targeting molecular tag - a second sequencing primer binding site - a unique sample barcode - a second sequencing adaptor.

55. The method of any one of embodiments 1-54, said method further comprising obtaining sequencing information from the one or more populations of MIPs.

56. The method of embodiment 55, wherein said sequencing information is used in the determination of the methylation state of a nucleic acid in a subject, or used in the determination of whether the subject has the predisposition to the disease or condition, or used in diagnosing the disease or condition, or used in detecting the state of the disease or condition, or used in differentiating the nucleic acid species originating from the subject and the one or more additional individuals, or used in differentiating the nucleic acid species originating from the first tissue and the one or more additional tissues, or used in the determination of the methylation state of a nucleic acid and detection of copy number variation in the subject, or used in the determination of the methylation age of the subject, or used in the determination of the methylation age of the tissue, respectively.

57. A method of determining the methylation state of a nucleic acid in a subject, the method comprising:

a) obtaining a genomic DNA sample from a blood sample from the subject;

b) performing bisulfite conversion of the genomic DNA sample;

c) adding the bisulfite-converted genomic DNA sample into each well of a multi-well plate, wherein each well of the multi-well plate comprises a probe mixture, wherein the probe mixture comprises a population of molecular inversion probes (MIPs) and a buffer;

wherein each MIP in the population of MIPs comprises in sequence the following components:

first targeting polynucleotide arm - first unique molecular tag - polynucleotide linker - second unique molecular tag - second targeting polynucleotide arm;

wherein the first targeting polynucleotide arm in each of the MIPs is substantially complementary to a first repeat region, and the second targeting polynucleotide arm in each of the MIPs is substantially complementary to a second repeat region, further wherein the first and second repeat regions, respectively, flank each sequence in a plurality of target sequences of interest;

wherein the first and second unique targeting molecular tags in each of the MIPs in combination are distinct in each of the MIPs;

wherein each target sequence of interest in the plurality of target sequences of interest has one or more CpG sites;

wherein each CpG site has a corresponding known position within the target sequence of interest;

d) incubating the bisulfite-converted genomic DNA sample with the probe mixture for the MIPs to capture the plurality of target sequences of interest;

e) adding an extension/ligation mixture to the sample of d) for the MIPs and the plurality of target sequences of interest to form a plurality of MIPs replicons, wherein the extension/ligation mixture comprises a polymerase, a plurality of dNTPs, a ligase, and buffer;

f) adding an exonuclease mixture to the targeting and control MIPs replicons to remove excess probes or excess genomic DNA;

g) adding an indexing PCR mixture to the sample of f) to add a pair of sequencing adapters comprising a unique molecular tag for multiplexed sequencing to the plurality of amplicons; and

h) using a massively parallel sequencing method to determine the number of occurrences of cytosine nucleotides at each corresponding CpG site within the MIPs amplicons to determine the methylation state of a nucleic acid.

58. A method of determining whether a subject has a predisposition to a disease or condition that is associated with the methylation state of a nucleic acid, the method comprising:

a) obtaining a genomic DNA sample from a blood sample from the subject;

b) performing bisulfite conversion of the genomic DNA sample;

c) adding the bisulfite-converted genomic DNA sample into each well of a multi-well plate, wherein each well of the multi-well plate comprises a probe mixture, wherein the probe mixture comprises a population of molecular inversion probes (MIPs) and a buffer;

wherein each MIP in the population of MIPs comprises in sequence the following components:

first targeting polynucleotide arm - first unique molecular tag - polynucleotide linker - second unique molecular tag - second targeting polynucleotide arm;

wherein the first targeting polynucleotide arm in each of the MIPs is substantially complementary to a first repeat region, and the second targeting polynucleotide arm in each of the MIPs is substantially complementary to a second repeat region, further wherein the first and second repeat regions, respectively, flank each sequence in a plurality of target sequences of interest;

wherein the first and second unique targeting molecular tags in each of the MIPs in combination are distinct in each of the MIPs;

wherein each target sequence of interest in the plurality of target sequences of interest has one or more CpG sites;

wherein each CpG site has a corresponding known position within the target sequence of interest;

d) incubating the bisulfite-converted genomic DNA sample with the probe mixture for the MIPs to capture the plurality of target sequences of interest;

e) adding an extension/ligation mixture to the sample of d) for the MIPs and the plurality of target sequences of interest to form a plurality of MIPs replicons, wherein the extension/ligation mixture comprises a polymerase, a plurality of dNTPs, a ligase, and buffer;

f) adding an exonuclease mixture to the targeting and control MIPs replicons to remove excess probes or excess genomic DNA;

g) adding an indexing PCR mixture to the sample of f) to add a pair of sequencing adapters comprising a unique molecular tag for multiplexed sequencing to the plurality of amplicons;

h) using a massively parallel sequencing method to determine the number of occurrences of cytosine nucleotides at each corresponding CpG site within the MIPs amplicons ;

i) determining a test ratio based on a first sum of the numbers of occurrences of cytosine nucleotides determined at step h) and a second sum of the known numbers of CpG sites in the plurality of target sequences of interest;

j) comparing the test ratio to a plurality of reference ratios that are computed based on reference nucleic acid samples isolated from reference subjects with the disease or condition that is associated with the methylation state of the nucleic acid; and

k) determining whether the subject is predisposed to the disease or condition based on the comparing in step j).

59. A method of diagnosing a disease or condition in a subject, the method comprising:

a) obtaining a genomic DNA sample from a blood sample from the subject;

b) performing bisulfite conversion of the genomic DNA sample;

c) adding the bisulfite-converted genomic DNA sample into each well of a multi-well plate, wherein each well of the multi-well plate comprises a probe mixture, wherein the probe mixture comprises a population of molecular inversion probes (MIPs) and a buffer;

wherein each MIP in the population of MIPs comprises in sequence the following components:

first targeting polynucleotide arm - first unique molecular tag - polynucleotide linker - second unique molecular tag - second targeting polynucleotide arm;

wherein the first targeting polynucleotide arm in each of the MIPs is substantially complementary to a first repeat region, and the second targeting polynucleotide arm in each of the MIPs is substantially complementary to a second repeat region, further wherein the first and second repeat regions, respectively, flank each sequence in a plurality of target sequences of interest;

wherein the first and second unique targeting molecular tags in each of the MIPs in combination are distinct in each of the MIPs;

wherein each target sequence of interest in the plurality of target sequences of interest has one or more CpG sites;

wherein each CpG site has a corresponding known position within the target sequence of interest;

d) incubating the bisulfite-converted genomic DNA sample with the probe mixture for the MIPs to capture the plurality of target sequences of interest;

e) adding an extension/ligation mixture to the sample of d) for the MIPs and the plurality of target sequences of interest to form a plurality of MIPs replicons, wherein the extension/ligation mixture comprises a polymerase, a plurality of dNTPs, a ligase, and buffer;

f) adding an exonuclease mixture to the targeting and control MIPs replicons to remove excess probes or excess genomic DNA;

g) adding an indexing PCR mixture to the sample of f) to add a pair of sequencing adapters comprising a unique molecular tag for multiplexed sequencing to the plurality of amplicons;

h) using a massively parallel sequencing method to determine the number of occurrences of cytosine nucleotides at each corresponding CpG site within the MIPs amplicons;

i) determining a test ratio based on a first sum of the numbers of occurrences of cytosine nucleotides determined at step h) and a second sum of the known numbers of CpG sites in the plurality of target sequences of interest;

j) comparing the test ratio to a plurality of reference ratios that are computed based on reference nucleic acid samples isolated from reference subjects with the disease or condition that is associated with the methylation state of the nucleic acid; and

k) diagnosing the disease or condition in the subject based on the comparing in step j).

60. A method of detecting the state of a disease or condition in a subject, said disease or condition being associated with the methylation state of a nucleic acid, the method comprising:

a) obtaining a genomic DNA sample from a blood sample from the subject;

b) performing bisulfite conversion of the genomic DNA sample;

c) adding the bisulfite-converted genomic DNA sample into each well of a multi-well plate, wherein each well of the multi-well plate comprises a probe mixture, wherein the probe mixture comprises a population of molecular inversion probes (MIPs) and a buffer;

wherein each MIP in the population of MIPs comprises in sequence the following components:

first targeting polynucleotide arm - first unique molecular tag - polynucleotide linker - second unique molecular tag - second targeting polynucleotide arm;

wherein the first targeting polynucleotide arm in each of the MIPs is substantially complementary to a first repeat region, and the second targeting polynucleotide arm in each of the MIPs is substantially complementary to a second repeat region, further wherein the first and second repeat regions, respectively, flank each sequence in a plurality of target sequences of interest;

wherein the first and second unique targeting molecular tags in each of the MIPs in combination are distinct in each of the MIPs;

wherein each target sequence of interest in the plurality of target sequences of interest has one or more CpG sites;

wherein each CpG site has a corresponding known position within the target sequence of interest;

d) incubating the bisulfite-converted genomic DNA sample with the probe mixture for the MIPs to capture the plurality of target sequences of interest;

e) adding an extension/ligation mixture to the sample of d) for the MIPs and the plurality of target sequences of interest to form a plurality of MIPs replicons, wherein the extension/ligation mixture comprises a polymerase, a plurality of dNTPs, a ligase, and buffer;

f) adding an exonuclease mixture to the targeting and control MIPs replicons to remove excess probes or excess genomic DNA;

g) adding an indexing PCR mixture to the sample of f) to add a pair of sequencing adapters comprising a unique molecular tag for multiplexed sequencing to the plurality of amplicons;

h) using a massively parallel sequencing method to determine the number of occurrences of cytosine nucleotides at each corresponding CpG site within the MIPs amplicons;

i) determining a test ratio based on a first sum of the numbers of occurrences of cytosine nucleotides determined at step h) and a second sum of the known numbers of CpG sites in the plurality of target sequences of interest;

j) comparing the test ratio to a plurality of reference ratios that are computed based on reference nucleic acid samples isolated from reference subjects with the disease or condition that is associated with the methylation state of the nucleic acid; and

k) detecting the state of the disease or condition in the subject based on the comparing in step j).

61. A method of differentiating nucleic acid species originating from a subject and one or more additional individuals, said subject and one or more additional individuals having differing methylation states of a nucleic acid, the method comprising:

a) obtaining a genomic DNA sample from a blood sample from the subject, said blood sample comprising nucleic acids originating from the subject and one or more additional individuals

b) performing bisulfite conversion of the genomic DNA sample;

c) adding the bisulfite-converted genomic DNA sample into each well of a multi-well plate, wherein each well of the multi-well plate comprises a probe mixture, wherein the probe mixture comprises a population of molecular inversion probes (MIPs) and a buffer;

wherein each MIP in the population of MIPs comprises in sequence the following components:

first targeting polynucleotide arm - first unique molecular tag - polynucleotide linker - second unique molecular tag - second targeting polynucleotide arm;

wherein the first targeting polynucleotide arm in each of the MIPs is substantially complementary to a first repeat region, and the second targeting polynucleotide arm in each of the MIPs is substantially complementary to a second repeat region, further wherein the first and second repeat regions,, respectively, flank each sequence in a plurality of target sequences of interest;

wherein the first and second unique targeting molecular tags in each of the MIPs in combination are distinct in each of the MIPs;

wherein each target sequence of interest in the plurality of target sequences of interest has one or more CpG sites;

wherein each CpG site has a corresponding known position within the target sequence of interest;

d) incubating the bisulfite-converted genomic DNA sample with the probe mixture for the MIPs to capture the plurality of target sequences of interest;

e) adding an extension/ligation mixture to the sample of d) for the MIPs and the plurality of target sequences of interest to form a plurality of MIPs replicons, wherein the extension/ligation mixture comprises a polymerase, a plurality of dNTPs, a ligase, and buffer;

f) adding an exonuclease mixture to the targeting and control MIPs replicons to remove excess probes or excess genomic DNA;

g) adding an indexing PCR mixture to the sample of f) to add a pair of sequencing adapters comprising a unique molecular tag for multiplexed sequencing to the plurality of amplicons;

h) using a massively parallel sequencing method to determine the number of occurrences of cytosine nucleotides at each corresponding CpG site within the MIPs amplicons;

i) determining a test ratio based on a first sum of the numbers of occurrences of cytosine nucleotides determined at step h) and a second sum of the known numbers of CpG sites in the plurality of target sequences of interest;

j) comparing the test ratio to a plurality of reference ratios that are computed based on reference nucleic acid samples isolated from reference subjects with the disease or condition that is associated with the methylation state of the nucleic acid; and

k) differentiating nucleic acid species originating from the subject and the one or more additional individuals based on the comparing in step j).

62. The method of embodiment 61, wherein the subject is a pregnant female and the one or more additional individuals is an unborn fetus.

63. The method of embodiment 61 or 62, wherein the blood sample is maternal plasma or maternal serum.

64. The method of embodiment 61, wherein the subject is a tissue transplant recipient.

65. The method of embodiment 64, wherein the one or more additional individuals is a tissue transplant donor.

66. A method of differentiating nucleic acid species originating from a first tissue and one or more additional tissues in a subject, said first tissue and one or more additional tissues having differing methylation states of a nucleic acid, the method comprising:

a) obtaining a DNA sample from a cell-free bodily fluid from the subject, said cell-free bodily fluid sample comprising nucleic acids originating from the first tissue and the one or more additional tissues;

b) performing bisulfite conversion of the DNA sample;

c) adding the bisulfite-converted DNA sample into each well of a multi-well plate, wherein each well of the multi-well plate comprises a probe mixture, wherein the probe mixture comprises a population of molecular inversion probes (MIPs) and a buffer;

wherein each MIP in the population of MIPs comprises in sequence the following components:

first targeting polynucleotide arm - first unique molecular tag - polynucleotide linker - second unique molecular tag - second targeting polynucleotide arm;

wherein the first targeting polynucleotide arm in each of the MIPs is substantially complementary to a first repeat region, and the second targeting polynucleotide arm in each of the MIPs is substantially complementary to a second repeat region, further wherein the first and second repeat regions, respectively, flank each sequence in a plurality of target sequences of interest;

wherein the first and second unique targeting molecular tags in each of the MIPs in combination are distinct in each of the MIPs;

wherein each target sequence of interest in the plurality of target sequences of interest has one or more CpG sites;

wherein each CpG site has a corresponding known position within the target sequence of interest;

d) incubating the bisulfite-converted DNA sample with the probe mixture for the MIPs to capture the plurality of target sequences of interest;

e) adding an extension/ligation mixture to the sample of d) for the MIPs and the plurality of target sequences of interest to form a plurality of MIPs replicons, wherein the extension/ligation mixture comprises a polymerase, a plurality of dNTPs, a ligase, and buffer;

f) adding an exonuclease mixture to the targeting and control MIPs replicons to remove excess probes or excess genomic DNA;

g) adding an indexing PCR mixture to the sample of f) to add a pair of sequencing adapters comprising a unique molecular tag for multiplexed sequencing to the plurality of amplicons;

h) using a massively parallel sequencing method to determine the number of occurrences of cytosine nucleotides at each corresponding CpG site within the MIPs amplicons;

i) determining a test ratio based on a first sum of the numbers of occurrences of cytosine nucleotides determined at step h) and a second sum of the known numbers of CpG sites in the plurality of target sequences of interest;

j) comparing the test ratio to a plurality of reference ratios that are computed based on reference nucleic acid samples isolated from reference tissues having differing methylation states of the nucleic acid; and

k) differentiating nucleic acid species originating from the first tissue and the one or more additional tissues based on the comparing in step j).

67. The method of embodiment 66, wherein the bodily fluid sample is a blood sample.

68. The method of embodiment 66 or 67, wherein the method further comprises (i) determining the percentage contribution of a particular tissue type to the nucleic acid; (ii) comparing the percentage contribution of the particular tissue type to a reference percentage contribution of the tissue type computed based on reference nucleic acid samples isolated from reference subjects with a disease or condition; and (iii) detecting the state of the disease or condition in the subject based on the comparing in step (ii).

69. The method of any one of embodiments 66-68, wherein the first tissue is selected from the group consisting of: liver, kidney, uterus, ovary, placenta, pancreas, colon, stomach, lung, and bladder.

70. A method of determining the methylation state of a nucleic acid and detecting copy number variation in a subject, the method comprising:

a) obtaining a genomic DNA sample from a blood sample from the subject;

b) performing bisulfite conversion of the genomic DNA sample;

c) adding the bisulfite-converted genomic DNA sample into each well of a multi-well plate, wherein each well of the multi-well plate comprises a probe mixture, wherein the probe mixture comprises a population of molecular inversion probes (MIPs) and a buffer;
wherein each MIP in the population of MIPs comprises in sequence the following components:

first targeting polynucleotide arm - first unique molecular tag - polynucleotide linker - second unique molecular tag - second targeting polynucleotide arm;
wherein the first targeting polynucleotide arm in each of the MIPs is substantially complementary to a first repeat region, and the second targeting polynucleotide arm in each of the MIPs is substantially complementary to a second repeat region, further wherein the first and second repeat regions, respectively, flank each sequence in a plurality of target sequences of interest;
wherein the first and second unique targeting molecular tags in each of the MIPs in combination are distinct in each of the MIPs;
wherein each target sequence of interest in the plurality of target sequences of interest has one or more CpG sites;
wherein each CpG site has a corresponding known position within the target sequence of interest;

d) incubating the bisulfite-converted genomic DNA sample with the probe mixture for the MIPs to capture the plurality of target sequences of interest;
e) adding an extension/ligation mixture to the sample of d) for the MIPs and the plurality of target sequences of interest to form a plurality of MIPs replicons, wherein the extension/ligation mixture comprises a polymerase, a plurality of dNTPs, a ligase, and buffer;
f) adding an exonuclease mixture to the targeting and control MIPs replicons to remove excess probes or excess genomic DNA;
g) adding an indexing PCR mixture to the sample of f) to add a pair of sequencing adapters comprising a unique molecular tag for multiplexed sequencing to the plurality of amplicons;
h) using a massively parallel sequencing method to determine the number of occurrences of cytosine nucleotides at each corresponding CpG site within the MIPs amplicons to determine the methylation state of a nucleic acid; and
i) using the massively parallel sequencing method to i) further determine the number of unique MIPs amplicons; ii) determining a first read density based at least in part on the number of unique MIP amplicon sequences; and iii) comparing the first read density to a plurality of reference read densities that are computed based on reference nucleic acid samples isolated from reference subjects; and iv) detecting copy number variation based on the comparing in step iii).

71. The method of embodiment 70, further comprising using the targeting molecular tags to remove duplicates to improve analysis.
72. The method of embodiment 70, further comprising determining the number of unique MIP amplicon sequences in defined regions to determine the read density.
73. A method of determining the methylation age of a subject, the method comprising:

a) obtaining a genomic DNA sample from a blood sample from the subject;
b) performing bisulfite conversion of the genomic DNA sample;
c) adding the bisulfite-converted genomic DNA sample into each well of a multi-well plate, wherein each well of the multi-well plate comprises a probe mixture, wherein the probe mixture comprises a population of molecular inversion probes (MIPs) and a buffer;
wherein each MIP in the population of MIPs comprises in sequence the following components:

first targeting polynucleotide arm - first unique molecular tag - polynucleotide linker - second unique molecular tag - second targeting polynucleotide arm;
wherein the first targeting polynucleotide arm in each of the MIPs is substantially complementary to a first repeat region, and the second targeting polynucleotide arm in each of the MIPs is substantially complementary to a second repeat region, further wherein the first and second repeat regions, respectively, flank each sequence in a plurality of target sequences of interest;
wherein the first and second unique targeting molecular tags in each of the MIPs in combination are distinct in each of the MIPs;
wherein each target sequence of interest in the plurality of target sequences of interest has one or more CpG sites;
wherein each CpG site has a corresponding known position within the target sequence of interest;

d) incubating the bisulfite-converted genomic DNA sample with the probe mixture for the MIPs to capture the plurality of target sequences of interest;

e) adding an extension/ligation mixture to the sample of d) for the MIPs and the plurality of target sequences of interest to form a plurality of MIPs replicons, wherein the extension/ligation mixture comprises a polymerase, a plurality of dNTPs, a ligase, and buffer;

f) adding an exonuclease mixture to the targeting and control MIPs replicons to remove excess probes or excess genomic DNA;

g) adding an indexing PCR mixture to the sample of f) to add a pair of sequencing adapters comprising a unique molecular tag for multiplexed sequencing to the plurality of amplicons;

h) using a massively parallel sequencing method to determine the number of occurrences of cytosine nucleotides at each corresponding CpG site within the MIPs amplicons ;

i) determining a test ratio based on a first sum of the numbers of occurrences of cytosine nucleotides determined at step h) and a second sum of the known numbers of CpG sites in the plurality of target sequences of interest;

j) comparing the test ratio to a plurality of reference ratios that are computed based on reference nucleic acid samples isolated from reference subjects; and

k) determining the methylation age of the subject based on the comparing in step j).

74. A method of determining the methylation age of a tissue in a subject, the method comprising:

a) obtaining a DNA sample from a cell-free bodily fluid sample from the subject;

b) performing bisulfite conversion of the DNA sample;

c) adding the bisulfite-converted DNA sample into each well of a multi-well plate, wherein each well of the multi-well plate comprises a probe mixture, wherein the probe mixture comprises a population of molecular inversion probes (MIPs) and a buffer;

wherein each MIP in the population of MIPs comprises in sequence the following components:

first targeting polynucleotide arm - first unique molecular tag - polynucleotide linker - second unique molecular tag - second targeting polynucleotide arm;

wherein the first targeting polynucleotide arm in each of the MIPs is substantially complementary to a first repeat region, and the second targeting polynucleotide arm in each of the MIPs is substantially complementary to a second repeat region, further wherein the first and second repeat regions, respectively, flank each sequence in a plurality of target sequences of interest;

wherein the first and second unique targeting molecular tags in each of the MIPs in combination are distinct in each of the MIPs;

wherein each target sequence of interest in the plurality of target sequences of interest has one or more CpG sites;

wherein each CpG site has a corresponding known position within the target sequence of interest;

d) incubating the bisulfite-converted genomic DNA sample with the probe mixture for the MIPs to capture the plurality of target sequences of interest;

e) adding an extension/ligation mixture to the sample of d) for the MIPs and the plurality of target sequences of interest to form a plurality of MIPs replicons, wherein the extension/ligation mixture comprises a polymerase, a plurality of dNTPs, a ligase, and buffer;

f) adding an exonuclease mixture to the targeting and control MIPs replicons to remove excess probes or excess genomic DNA;

g) adding an indexing PCR mixture to the sample of f) to add a pair of sequencing adapters comprising a unique molecular tag for multiplexed sequencing to the plurality of amplicons;

h) using a massively parallel sequencing method to determine the number of occurrences of cytosine nucleotides at each corresponding CpG site within the MIPs amplicons ;

i) determining a test ratio based on a first sum of the numbers of occurrences of cytosine nucleotides determined at step h) and a second sum of the known numbers of CpG sites in the plurality of target sequences of interest;

j) comparing the test ratio to a plurality of reference ratios that are computed based on reference nucleic acid samples isolated from reference tissues; and

k) determining the methylation age of the tissue based on the comparing in step j).

75. The method of embodiment 74, wherein the bodily fluid sample is a blood sample.

76. The method of embodiment 74 or 75, wherein the subject is a pregnant female.

77. The method of any one of embodiments 74-76, wherein the tissue is placental tissue and the methylation age of the placental tissue is an indicator of the gestational age of an unborn fetus.

78. The method of any one of embodiments 57-65, 67-73 and 75-77, wherein the blood sample is a whole blood sample, a plasma sample, or a serum sample.

79. The method of embodiment 78, wherein the blood sample is a plasma sample.

80. The method of any one of embodiments 57-79, wherein the length of the first targeting polynucleotide arm is between 14 and 30 bases.

81. The method of any one of embodiments 57-80, wherein the length of the second targeting polynucleotide arm is between 14 and 30 bases.

82. The method of any one of embodiments 57-81, wherein each of the targeting polynucleotide arms has a melting temperature between 45 °C and 80 °C.

83. The method of any one of embodiments 57-82, wherein each of the targeting polynucleotide arms has a GC content between 10% and 50%.

84. The method of any one of embodiments 57-83, wherein the length of the first unique molecular tag is between 4 and 15 bases.

85. The method of any one of embodiments 57-84, wherein the length of the second unique molecular tag is between 4 and 15 bases.

86. The method of any one of embodiments 57-85, wherein the polynucleotide linker is not substantially complementary to any genomic region of the subject.

87. The method of any one of embodiments 57-86, wherein the polynucleotide linker has a length of between 20 and 1,000 bases.

88. The method of any one of embodiments 57-87, wherein the polynucleotide linker has a melting temperature of between 45 °C and 80 °C.

89. The method of any one of embodiments 57-88, wherein the polynucleotide linker has a GC content between 30% and 80%.

90. The method of any one of embodiments 57-89, wherein the polynucleotide linker comprises at least one amplification primer binding site.

91. The method of embodiment 90, wherein the polynucleotide linker comprises a forward amplification primer binding site.

92. The method of embodiment 91, wherein the sequence of the forward amplification primer comprises the nucleotide sequence of CCGTAATCGGGAAGCTGAAG (SEQ ID NO: 1).

93. The method of any one of embodiments 57-92, wherein the sequence of a reverse amplification primer comprises the nucleotide sequence of GCACGATCCGACGGTAGTGT (SEQ ID NO:2).

94. The method of any one of embodiments 57-93, wherein the polynucleotide linker comprises the nucleotide sequence of CTTCAGCTTCCCGATTACGGGCACGATCCGACGGTAGTGT (SEQ ID NO:3).

95. The method of any one of embodiments 57-94, wherein the first targeting polynucleotide arm comprises the nucleotide sequence of CACTACACTCCAACCTAA (SEQ ID NO:4) or TTCTCCTACCTCAACCTC (SEQ ID NO:5).

96. The method of any one of embodiments 57-95, wherein the second targeting polynucleotide arm comprises the nucleotide sequence of GAGGCTGAGGCAGGAGAA (SEQ ID NO:10) or CCAAACTAAAATACAATA (SEQ ID NO:7).

97. The method of any one of embodiments 57-96, wherein the MIP comprises the nucleotide sequence of CACTACACTCCAACCTAA(N1) CTTCAGCTTCCCGATTACGGGCACGATCCGACGGTAGTGT(N2) CAAAAAACTAAAACAAAA (SEQ ID NO:8) or TTCTCCTACCTCAACCTC(N1) CTTCAGCTTCCCGATTACGGGCACGATCCGACGGTAGTGT(N2) CCAAACTAAAATACAATA (SEQ ID NO:9), wherein (N1) represents the first unique molecular tag and (N2) represents the second unique molecular tag.

98. The method of any one of embodiments 57-97, wherein the population of MIPs has a concentration between 10 fM and 100 nM.

99. A method of selecting a molecular inversion probe (MIP) from a plurality of candidate MIPs for using to detect methylation in a subject, the method comprising:

a) receiving nucleic acid sequences of the plurality of candidate MIPs, wherein each of the MIPs in the plurality of candidate MIPs comprises in sequence the following components:
first targeting polynucleotide arm - first unique molecular tag - polynucleotide linker - second unique molecular tag - second targeting polynucleotide arm;
b) for each respective MIP in the plurality of candidate MIPs,

i) computing a first number (A) of unique CpG sites predicted, with no mismatch on the binding arm sequence, to be captured by the respective MIP;
ii) computing a second number (C) of unique CpG sites predicted, with one mismatch on the binding arm sequence, to be captured by the respective MIP;

iii) computing a third number (E) of unique sites predicted, with no mismatch on the binding arm sequence, to be captured by the respective MIP across a genome;

iv) computing a fourth number (G) of unique sites predicted, with one mismatch on the binding arm sequence, to be captured by the respective MIP across the genome;

v) computing a fifth number (F) of non-unique sites predicted, with no mismatch on the binding arm sequence, to be captured by the respective MIP across the genome;

vi) computing a sixth number (H) of non-unique sites predicted, with one mismatch on the binding arm sequence, to be captured by the respective MIP across the genome;

vii) computing a seventh number (I) of CpG sites present on the first targeting polynucleotide arm;

viii) computing an eighth number (J) of CpG sites present on the second targeting polynucleotide arm;

ix) computing a performance metric for the respective MIP based at least in part on the first, second, third, fourth, fifth, sixth, seventh, and eighth numbers;

c) selecting a MIP, based at least in part on the performance metric computed in step b)ix) for each MIP in the plurality of candidate MIPs.

100. The method of embodiment 99, wherein the MIP at step c) is selected such that a sum of the seventh number (I) and the eighth number (J) is smaller than the corresponding sum for a remaining set of the candidate MIPs.

101. The method of embodiment 99 or 100, wherein:

a first sum is a sum of the first number (A) and the second number (C);

a second sum is a sum of the third number (E), the fourth number (G), the fifth number (F), and the sixth number (H); and

the MIP at step c) is selected such that a ratio between the first sum and the second sum is larger than the ratio for a remaining set of the candidate MIPs.

102. The method of any one of embodiments 99-101, wherein:

a third sum is a sum of the third number (E) and the fourth number (G);

a fourth sum is a sum of the third number (E), the fourth number (G), the fifth number (F), and the sixth number (H); and

the MIP at step c) is selected such that a ratio between the third sum and the fourth sum is larger than the ratio for a remaining set of the candidate MIPs.

103. The method of any one of embodiments 99-102, wherein the MIP at step c) is selected based on a ratio ($K_e$) of an average capture coefficient of one mismatch sites ($K_1$) and an average capture coefficient of zero mismatch sites ($K_0$):

$$K_e = \frac{K_1}{K_0}$$

and wherein the ratio ($K_e$) is experimentally estimated.

104. The method of any of embodiments 99-103, wherein the performance metric at step b)ix includes a factor corresponding to a weighted sum of the first number (A) and the second number (C).

105. The method of embodiment 104, wherein the weighted sum corresponds to $A + Ke \times C$.

106. The method of any of embodiments 99-015, wherein the performance metric at step b)ix includes a factor corresponding to a weighted sum of the third number (E) and the fourth number (G).

107. The method of embodiment 106, wherein the weighted sum corresponds to $E + Ke \times G$.

108. The method of any of embodiments 99-107, wherein the MIP at step c) is selected such that a product between a first weighted sum of $A + Ke \times C$ and a second weighted sum of $E + Ke \times G$ is larger than the product for a remaining set of the candidate MIPs.

109. A nucleic acid molecule comprising a nucleotide sequence of CACTACACTCCAACCTAA(N1) CTTCAGCTTCCCGATTACGGGCACGATCCGACGGTAGTGT(N2) CAAAAAACTAAAACAAAA (SEQ ID NO: 8) or TTCTCCTAC-CTCAACCTC(N1) CTTCAGCTTCCCGATTACGGGCACGATCCGACGGTAGTGT(N2) CCAAACTAAAATACAA-TA (SEQ ID NO:9), wherein (N1) represents a first unique molecular tag and (N2) represents a second unique molecular tag.

110. The nucleic acid of embodiment 109, wherein the length of the first unique molecular tag is between 4 and 15 bases.

111. The nucleic acid of embodiment 109 or 110, wherein the length of the second unique molecular tag is between 4 and 15 bases.

112. The method of any one of embodiments 2-5, 10, 17, 18, 58, 59-61, 66, 73 and 74, wherein step e) further comprises determining the number of occurrences of cytosine nucleotides at each corresponding CpG site within a subset of the MIPs amplicons sequenced at step d), wherein the subset is previously determined to be differentially methylated.

113. The method of embodiment 112, wherein the subset is differentially methylated between different individuals or between different tissues or between a test sample and a reference sample or between a disease sample and a healthy control sample.

**Brief Description of the Drawings**

**FIG. 1** is an illustrative embodiment of a computing device for performing any of the processes as described in accordance with the methods of the invention.

**FIG. 2** is a representative process flow diagram for designing and selecting a probe according to the methods of the invention.

**FIG. 3** is a representative process flow diagram for predicting a methylation state in a test subject according to the methods of the invention.

**FIG. 4** is another representative and more detailed process flow diagram for predicting a disease state of a test subject according to the methods of the invention.

**FIG. 5** depicts an exemplary molecular inversion probe (MIP).

**FIG. 6** depicts a process by which a MIP can capture a target sequence of interest.

**FIG. 7** depicts a process by which a captured target sequence of interest is indexed and amplified.

**FIG. 8** is a Circos plot depicting a graphical representation of the data obtained from a methylation status assay.

**FIG. 9** depicts data corresponding to repeat methylation scores for various percentage mixes of two DNA samples.

**FIG. 10** provides an example of how to calculate a methylation score across multiple CpG sites in multiple samples.

**FIGS. 11A and 11B** depict a process by which a MIP can capture a bisulfite-converted target sequence of interest, followed by an extension and ligation reaction which creates a replicon. The replicon is subsequently amplified by PCR to create a PCR product comprising the target sequence of interest.

**FIG. 12** depicts a process for amplifying the PCR product from FIG. 11B for further amplification.

**FIG. 13** is a representative process flow diagram for processing the amplification products from FIG. 12 for further methylation analysis.

**FIG. 14** shows two Circos plots depicting a graphical representation of the methylation data obtained from colorectal samples.

**FIGS. 15A and 15B** show two plots of copy number alterations (CNAs) across the genome in a colorectal cancer sample depicting genomic instability associated with the disease.

**FIG. 16** shows the percentage of CpG sites across the genome with hypomethylation at CNA sites in normal versus tumor tissue.

**FIG. 17** is a representative process flow diagram for determining tissue-of-origin based on methylation status.

**FIG. 18** shows separation between blood-derived DNA (B) and liver DNA L) based on pattern of methylation.

**FIG. 19** is a dendrogram showing clustering of 10 different tissue types based on methylation.

**FIG. 20** is a chart showing that overall methylation decreases with age of a subject.

**FIG. 21** is a chart showing that overall methylation decreases with gestational age of a fetus.

**Detailed Description of the Invention**

This disclosure provides a system and method for detecting diseases or conditions. There is a need for informative, non-invasive tools facilitating improved diagnosis, prognosis, and surveillance of human disease. Several complex diseases including carcinogenesis display altered CpG methylation at specific loci and more broadly across the genome. Both targeted and whole genome bisulfite sequencing methods have been used to discern these changes in tissue and in cell-free DNA, but have significant cost and complexity drawbacks. To this end, the inventors have developed a single-probe capture method for sequencing ready libraries from input of DNA as low as 200 pg of tissue or circulating genetic material. This method simultaneously assesses >200,000 sites across the genome, which can be analyzed simultaneously to determine one or more of methylation status, genomic instability (e.g. copy number variation), or mutational landscape. Further, the inventors have developed methods to identify methylated regions and patterns, areas of genomic instability, and mutational landscapes that are significantly different between sample types.

The following detailed description is set forth as an aid to understanding various embodiments.

Unless otherwise defined herein, scientific and technical terms used in this disclosure shall have the meanings that are commonly understood by those of ordinary skill in the art to which this invention belongs. Generally, nomenclature used in connection with, and techniques of, cell and tissue culture, molecular biology, cell biology, cancer biology, neurobiology, neurochemistry, virology, immunology, microbiology, genetics, protein and nucleic acid chemistry, chemistry, and pharmacology described herein, are those well-known and commonly used in the art. Each embodiment of the inventions described herein may be taken alone or in combination with one or more other embodiments of the invention which is defined in the appended claims.

The methods and techniques of the various embodiments are generally performed, unless otherwise indicated, according to methods of molecular biology, cell biology, biochemistry, microarray and sequencing technology well known in the art and as described in various general and more specific references that are cited and discussed throughout this specification. See, e.g. Motulsky, "Intuitive Biostatistics", Oxford University Press, Inc. (1995); Lodish et al., "Molecular Cell Biology, 4th ed.", W. H. Freeman & Co., New York (2000); Griffiths et al., "Introduction to Genetic Analysis, 7th ed.", W. H. Freeman & Co., N.Y. (1999); Gilbert et al., "Developmental Biology, 6th ed.", Sinauer Associates, Inc., Sunderland, MA (2000).

Chemistry terms used herein are used according to conventional usage in the art, as exemplified by "The McGraw-Hill Dictionary of Chemical Terms", Parker S., Ed., McGraw-Hill, San Francisco, C.A. (1985).

In case of conflict, the present specification, including its specific definitions, will control.

Throughout this specification, the word "comprise" or variations such as "comprises" or "comprising" will be understood to imply the inclusion of a stated integer (or components) or group of integers (or components), but not the exclusion of any other integer (or components) or group of integers (or components).

The singular forms "a," "an," and "the" include the plurals unless the context clearly dictates otherwise.

The term "including" is used to mean "including but not limited to". "Including" and "including but not limited to" are used interchangeably.

The following terms and definitions are provided herein.

**Definitions**

The term "DNA methylation" as used herein refers to the addition of a methyl group to the 5' carbon of cytosine residues (i.e. 5-methylcytosines) among CpG dinucleotides. DNA methylation may occur in cytosines in other contexts, for example CHG and CHH, where H is adenine, cytosine or thymine. Cytosine methylation may also be in the form of 5-hydroxymethylcytosine. DNA methylation may include non-cytosine methylation such as N6-methyladenine.

The term "methylation state" or "methylation status" as used herein, refers to the state of a nucleic acid molecule or population of nucleic acid molecules with respect to the methylation of certain nucleotides. For example, genomic DNA is methylated at certain sites (e.g., CpG sites) at cytosine nucleotides. Thus, the methylation state of a nucleic acid may refer to the ratio of CpG sites in a genome that is methylated, or the ratio of CpG sites in a genome that is unmethylated.

The term "methylation score" as used herein refers to a ratio calculated from the number of cytosine sites (C's) observed at CpG sites. It may also be referred to as a "test ratio" or "reference ratio". The methylation score provides the ratio of unconverted (i.e., methylated) cytosine nucleotides at one or more CpG sites usually across a region or the entire genome. The methylation score can be calculated using the following ratio:

Methylated C's at CpG site / (Methylated C's at CpG site + Unmethylated C's at CpG site).

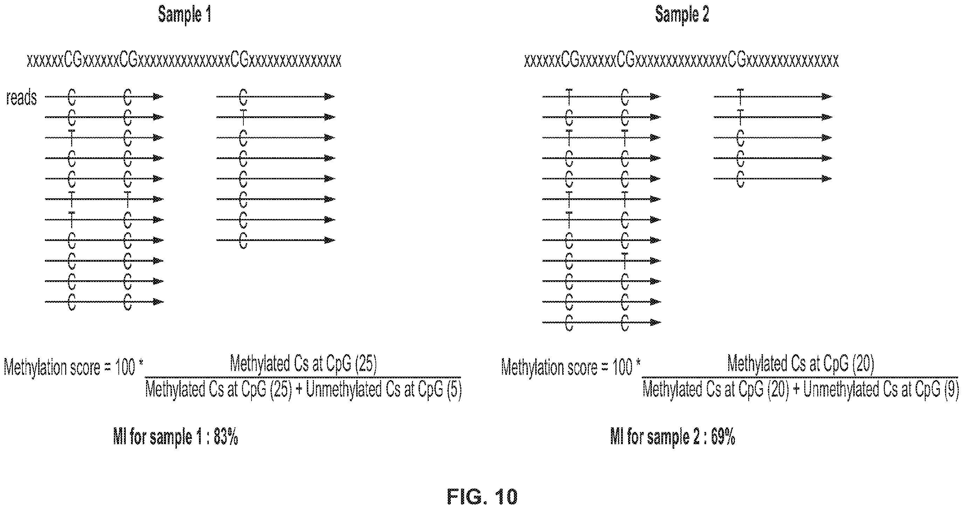

While a single CpG site in a nucleic acid molecule can be methylated or unmethylated, it is more common, and clinically useful, to determine the methylation status of a population of cells with each cell containing a unique diploid genome. In some embodiments, the compositions and methods described herein provide a methylation score for a subset of the total diploid genomes within a selected sample. Thus, the binary methylation status of a collection of single CpG sites is being summed over the population of cells to give a methylation score across many CpG sites in a sample. In some embodiments, the methylation score of a region (e.g., block, gene, chromosome, or globally) can be calculated as a median, mean or average of the individual site ratios. The methylation score can also be expressed as a ratio or a percentage. In some embodiments that employ a sequencing readout such as next generation sequencing, the methylation score is calculated from the nucleic acid sequence information contained in sequencing reads comprising CpG sites. Thus, a methylation score can be thought of as the proportion of sequence reads showing methylation at CpG sites over the total number of reads covering the CpG sites - whether methylated or not. In some embodiments, a single read can generate multiple counts if it comprises multiple CpG sites. For example in FIG. 10, the number of CpG sites covered by the assay does not necessarily dictate the methylation score. If that was the case, as illustrated in FIG. 10, the three CpG sites in both Samples 1 and 2 would be methylated and the methylation index would be 100% for both samples. In some embodiments, the methylation score can be used to determine the methylation state of a single CpG site, or a series of individual CpG sites. In some embodiments, CpG sites can be selectively filtered out of the analysis or the CpG sites can be grouped and the methylation density can be calculated. The methylation score can also be the average of the methylation score at individual CpG sites in order to correct for different sequencing coverage at each individual site. For example, without averaging, a CpG with 100x coverage might be weighted more in the methylation score than a site with 20x coverage. However, by averaging the methylation score at individual cytosines, every cytosine with a methylation score is given a methylation score regardless of sequencing coverage.

In some embodiments, the methylation score can be expressed as the "methylation density", which is the methylation score for the CpG sites in a defined region (e.g., a particular CpG site, CpG sites within a CpG island, or a larger region such as a block). For example, the methylation density for a 1Mb bin in the human genome can be determined from the number of counts showing CpG methylation divided by the total number of counts covering CpG sites in the 1Mb region. This analysis can also be performed for other bin sizes, e.g. 50 kb, 100 kb, 200kb, 250 kb, 300kb, 400 kb, 500 kb, 750 kb, etc.

In some embodiments, the methylation score can be expressed as the "proportion of methylated cytosines", which includes cytosines outside of the CpG context in the region.

In some embodiments, the methylation score can be expressed as a "global methylation score" or "global methylation index". The global methylation index refers to the methylation score for all of the CpG sites interrogated by the compositions and methods described herein, which includes CpG sites across the genome (e.g., greater than 50,000, 60,000, 70,000, 80,000, 100,000, 150,000, 200,000, 300,000, 400,000, 500,000 or more CpG sites distributed throughout the genome); thus allowing one to generate a global methylation index with a single assay. The skilled worker will appreciate that not every CpG site in a genome needs to be studied to determine the global methylation index. For example, the methylation score of a subset of CpG sites may be determined as an indication for the global methylation state of the entire genome, and given as a "global methylation index".

In some embodiments, a methylation score is determined for a test subject, sample, tissue or portion thereof, in which case it is referred to as a "test methylation score" or "test ratio". A test ratio can be compared to a "reference methylation score" or "reference ratio" from a corresponding known (reference) subject, sample or tissue. For example, the methylation score from a population of cells (e.g., from a tumor, or from a particular tissue type), multiple or mixed populations of cells (e.g., maternal and fetal cells), or multiple subjects (e.g., smoker vs. non-smoker) can be determined and compared to corresponding well-characterized, reference samples or subjects.

In some embodiments, the regions with methylation differences between test samples and reference samples are referred to as "differentially methylated regions" (DMRs), which are regions or blocks having different methylation scores. A differentially methylated region (e.g., block, chromosome, gene, island, etc.) is identified by a difference in the methylation score between a test and reference sample across a sufficient number of samples to be significant.

The term "site" as used herein refers to a single site, which may be a single base position or a group of correlated base positions, e.g., a CpG site; whereas a "block" or "region" refers to a portion of the genome that includes multiple sites. A block may include one or more CpG islands, genes, chromatin regions such as large organized chromatin lysine-modifications, or nuclear organization regions such as lamin associated domains. A block may contain one or more repeat elements. The compositions and methods described herein offer improved methods for identifying large scale phenomenon of methylation dysregulation in diseases such as cancer. Rather than a specific targeting of methylation change at particular sites, the compositions and methods described are able to assay the regions of the genome believed to be pathologically important for cell differentiation and disease. Furthermore, in the case of cancer, there is evidence that epigenetic dysregulation is occurring early in cancer - even before full cancer development (see Timp et al. Genome Medicine 2014, 6:61) and is more likely to occur at CpG sites that reside in Alu repeat elements (see Luo et al. BioMed research international 2014); thereby adding to the clinical utility of the methods and compositions described herein.

The term "methylome" as used herein refers to the amount or pattern of methylation at different sites or regions within a population of cells. Thus, methylome can be thought of as the methylation score for a particular population of cells. For example, a disease state may have a methylome, such as the healthy liver methylome versus the necrotic liver. A tissue type may have a methylome, such as a liver methylome versus a blood methylome. A cellular phenotype may have a methylome, such as senescent cells versus dividing cells. The methylome may correspond to all of the genome, a subset of the genome (e.g., repeat elements in the genome), or a portion of the subset (e.g., those areas found to be associated with disease). A "fetal methylome" corresponds to a methylome of a fetus of a pregnant female. The fetal methylome can be determined using a variety of fetal tissues or sources of fetal DNA, including placental tissues and cell-free fetal DNA in maternal plasma. A "tumor methylome" corresponds to a methylome of a tumor of an organism (e.g., a human). The tumor methylome can be determined using tumor tissue or cell-free tumor DNA. In certain embodiments, cell-free tumor DNA is present in plasma. The fetal methylome and the tumor methylome are examples of a methylome of interest. Other examples of methylomes of interest are the methylomes of organs (e.g. methylomes of the liver, lungs, prostate, gastrointestinal tract, bladder etc.) that can contribute DNA into a bodily fluid (e.g. plasma, serum, sweat, saliva, urine, genital secretions, semen, stools fluid, diarrheal fluid, cerebrospinal fluid, secretions of the gastrointestinal tract, ascitic fluid, pleural fluid, intraocular fluid, fluid from a hydrocele (e.g. of the testis), fluid from a cyst, pancreatic secretions, intestinal secretions, sputum, tears, aspiration fluids from breast and thyroid, etc.). The organs may be transplanted organs. A methylome from plasma may be referred to a "plasma methylome". The plasma methylome is an example of a cell-free methylome since plasma and serum include cell-free DNA (cfDNA). The plasma methylome is also an example of a mixed population methylome since it is a mixture of fetal/maternal methylome or tumor/non-tumor methylome or DNA derived from different tissues or organs.

The term "read" as used herein refers to the raw or processed output of sequencing systems, such as massively parallel sequencing. In some embodiments, the output of the methods and compositions described herein is reads. In some embodiments, these reads may need to be trimmed, filtered, and aligned, resulting in raw reads, trimmed reads, aligned reads. The term "count" as used herein refers to a uniquely aligned read within target sequence of interest. In the context of the methylation score, a count will correspond to the information retrieved from the reads (methylated or unmethylated) at the CpG sites. Therefore, if a read encompassed multiple CpG site, this read can produce multiple counts.

In certain embodiments, the methods may be used to detect copy number variations. As used herein a "copy number variation" (CNV) generally is a class or type of genetic variation or chromosomal aberration. In some contexts, copy number variations refer to changes in copy number in germline cells, while copy number alterations/aberrations (CNAs) refer changes in copy number that have arisen in somatic tissue (e.g., in tumor cells). As used herein, copy number variations include copy number alterations/aberrations. A copy number variation can be a deletion (e.g. micro-deletion), duplication (e.g., a micro-duplication), or insertion (e.g., a micro-insertion). In certain embodiments, the prefix "micro" as used herein may refer to a segment of a nucleic acid less than 5 base pairs in length. A copy number variation can include one or more deletions (e.g. micro-deletion), duplications and/or insertions (e.g., a micro-duplication, micro-insertion) of a segment of a chromosome. In certain embodiments a duplication comprises an insertion. In certain embodiments an insertion is a duplication. In certain embodiments an insertion is not a duplication. For example, a duplication of a sequence in a portion increases the counts for a portion in which the duplication is found. Often a duplication of a sequence in a portion increases the elevation or level. In certain embodiments, a duplication present in portions making up a first elevation or level increases the elevation or level relative to a second elevation or level where a duplication is absent. In certain embodiments an insertion increases the counts of a portion and a sequence representing the insertion is present (i.e., duplicated) at another location within the same portion. In certain embodiments an insertion does not significantly increase the counts of a portion or elevation or level and the sequence that is inserted is not a duplication of a sequence within the same portion. In certain embodiments an insertion is not detected or represented as a duplication and a duplicate sequence representing the insertion is not present in the same portion. In some embodiments a copy number variation is a fetal copy number variation. Often, a fetal copy number variation is a copy number variation in the genome of a fetus. In some embodiments a copy number variation is a maternal and/or fetal copy number variation. In certain embodiments a maternal and/or fetal copy number variation is a copy number variation within the genome of a pregnant female (e.g., a female subject bearing a fetus), a female subject that gave birth or a female capable of bearing a fetus. A copy number variation can be a heterozygous copy number variation where the variation (e.g., a duplication or deletion) is present on one allele of a genome. A copy number variation can be a homozygous copy number variation where the variation is present on both alleles of a genome. In some embodiments a copy number variation is a heterozygous or homozygous fetal copy number variation. In some embodiments a copy number variation is a heterozygous or homozygous maternal and/or fetal copy number variation. A copy number variation sometimes is present in a maternal genome and a fetal genome, a maternal genome and not a fetal genome, or a fetal genome and not a maternal genome.

The term "aneuploidy," as used herein, refers to a chromosomal abnormality characterized by an abnormal variation in chromosome number, e.g., a number of chromosomes that is not an exact multiple of the haploid number of chromosomes. For example, a euploid individual will have a number of chromosomes equaling 2n, where n is the number of chromosomes in the haploid individual. In humans, the haploid number is 23. Thus, a diploid individual will have 46 chromosomes. An aneuploid individual may contain an extra copy of a chromosome (trisomy of that chromosome) or lack a copy of the chromosome (monosomy of that chromosome). The abnormal variation is with respect to each individual chromosome. Thus, an individual with both a trisomy and a monosomy is aneuploid despite having 46 chromosomes. Examples of aneuploidy diseases or conditions include, but are not limited to, Down syndrome (trisomy of chromosome 21), Edwards syndrome (trisomy of chromosome 18), Patau syndrome (trisomy of chromosome 13), Turner syndrome (monosomy of the X chromosome in a female), and Klinefelter syndrome (an extra copy of the X chromosome in a male). Other, non-aneuploid chromosomal abnormalities include translocation (wherein a segment of a chromosome has been transferred to another chromosome), deletion (wherein a piece of a chromosome has been lost), and other types of chromosomal damage (e.g., Fragile X syndrome, which is caused by an X chromosome that is abnormally susceptible to damage).

The terms "subject" and "patient", as used herein, refer to any animal, such as a dog, a cat, a bird, livestock, and particularly a mammal, and preferably a human. The term "reference subject" and "reference patients" refer to any subject or patient that exhibits known genotypes (e.g., known euploidy or aneuploidy), phenotypes, or age, or to a subject or patient that is known to have a disease or condition, or known to not have a disease or condition, or known to have a particular state of a disease or condition, or known to have a predisposition to a disease or condition, or known to have been exposed to drugs, toxins, a particular diet, or an agent or conditions suspected of causing methylation changes. The skilled worker will appreciate that the subject can be any human. In certain embodiments, the subject is a pregnant female. In these embodiments, the blood sample may be a maternal plasma or serum sample. In certain embodiments, the subject is an organ transplant recipient, and the subject's methylation state may be indicative of organ rejection. In certain embodiments, the methylation state of a population of target sequences of interest (e.g., of fetal, tumor, or disease origin) may be determined among a background of target sequences of interest (e.g., maternal, non-tumor, or disease-free origin). The background target sequences of interest may serve as a reference, wherein differences from the reference are indicative of a disease or condition, or to identify a nucleic acid species.

The term "mutational landscape", as used herein, is the cumulative frequency of a collection of mutations that generally span the genome. The types of mutations that make up a mutational landscape, include but are not limited to, single nucleotide variations, deletions, and insertions, and the type of mutations also inform a given mutational landscape or pattern. Examples of specific mutational landscapes associated with diseases or conditions include an increased frequency of C>A transversions associated with cigarette smoke exposure (Ding, L. et al. Somatic mutations affect key pathways in lung adenocarcinoma. Nature 455, 1069-1075 (2008)), and an increased frequency of C>T transitions and C>G transversions associated with 12 cancer types (Kandoth, Cyriac, et al. "Mutational landscape and significance across 12 major cancer types." Nature 502.7471 (2013): 333-339).

The term "tissue-of-origin" as used herein refers to the tissue source of nucleic acids in a sample, where "tissue" is used to describe a group or population of cells of a same type. Some tissue may have multiple cell types, for example hepatocytes, alveolar cells or blood cells, while other tissue may originate from different organisms, for example, mother and fetus, or from healthy vs. disease tissue. Likewise, "reference tissues" may be used to determine methylation-specific tissue patterns or levels. For example, reference tissues from multiple subjects may be used to determine the tissue components of a test sample. In some embodiments, the DNA molecules of differing origin are DNA molecules of maternal origin and DNA molecules of fetal origin. In some embodiments, the DNA molecules of differing origin are DNA molecules of a first tissue origin and DNA molecules of a second tissue origin or of leukocyte origin. In some embodiments, determining the tissue-of-origin may be indicative of the presence of disease (e.g., cancer), or may be used to determine the relative or absolute amount of DNA from a particular tissue (e.g., fetal cfDNA in a maternal sample). Thus, the compositions and methods described herein can be used to differentiate and identify the tissue source of DNA. Given an unknown source of tissue, DNA can be extracted using standard methods, the compositions and methods described herein can be used to generate tissue-specific data, and the data can be fit into the most likely tissue reference bin as described further in the Examples.

The terms "polynucleotide", "nucleic acid" and "nucleic acid molecules", as used herein, are used interchangeably and refer to DNA molecules (e.g., cDNA or genomic DNA), RNA molecules (e.g., mRNA), DNA-RNA hybrids, and analogs of the DNA or RNA generated using nucleotide analogs. The nucleic acid molecule can be a nucleotide, oligonucleotide, double-stranded DNA, single-stranded DNA, multi-stranded DNA, complementary DNA, genomic DNA, non-coding DNA, messenger RNA (mRNAs), microRNA (miRNAs), small nucleolar RNA (snoRNAs), ribosomal RNA (rRNA), transfer RNA (tRNA), small interfering RNA (siRNA), heterogeneous nuclear RNAs (hnRNA), or small hairpin RNA (shRNA). In certain embodiments, the methods can be performed on a nucleic acid sample such as DNA or RNA, e.g., genomic DNA. In some embodiments the nucleic acid molecule may be cell-free DNA (cfDNA). Cell-free DNA is thought to result from cellular necrosis or apoptosis, wherein genomic cellular DNA is digested and becomes fragmented, extracellular DNA. Cell-free DNA of apoptotic origin may be from a non-host (e.g., transplanted organ or tissue), fetus (e.g., from the placenta resulting in cell-free fetal DNA), or a diseased tissue (e.g., from a tumor resulting in circulating tumor DNA). Cell-free DNA can be detected in a range of samples including, but not limited to, blood, plasma and urine. In some embodiments, the nucleic acid molecules are associated with exosomes, which are microvesicles released from a variety of different cells, including cancer cells. In some embodiments, the compositions and methods described herein may be able to differentiate cfDNA of necrotic and apoptotic origin based on its methylation status. A nucleic acid sample may be isolated in any manner known to a person of ordinary skill in the art (e.g., by centrifugation).

The term "sample", as used herein, refers to a sample typically derived from a biological fluid, cell, tissue, organ, or organism, comprising a nucleic acid or a mixture of nucleic acids comprising at least one nucleic acid sequence that is to be screened for, e.g., cancer or aneuploidy. In some embodiments, a sample is a blood sample such as a whole blood sample, a serum sample, or a plasma sample. In some embodiments the sample comprises at least one nucleic acid sequence whose genome is suspected of having undergone variation. Such samples include, but are not limited to sputum/oral fluid, amniotic fluid, blood, a blood fraction, or fine needle biopsy samples (e.g., surgical biopsy, core needle biopsy, fine needle biopsy, etc.) urine, stool, peritoneal fluid, pleural fluid, cerebro-spinal fluid, gastrointestinal fluid, cell lines, tissue embedded in paraffin, fresh frozen tissue, and the like. Although the sample is often taken from a human subject (e.g., patient), the assays can be used to detect a disease or condition, or detect the state of a disease or condition, or determine whether a subject has a predisposition to a disease or condition, in samples from any mammal, including, but not limited to dogs, cats, horses, goats, sheep, cattle, pigs, etc. The sample may be used directly as obtained from the biological source or following a pretreatment to modify the character of the sample. For example, such pretreatment may include preparing plasma from blood, diluting viscous fluids and so forth. Methods of pretreatment may also involve, but are not limited to, bisulfite conversion, filtration, precipitation, dilution, distillation, mixing, centrifugation, freezing, lyophilization, concentration, amplification, nucleic acid fragmentation, inactivation of interfering components, the addition of reagents, lysing, etc. If such methods of pretreatment are employed with respect to the sample, such pretreatment methods are typically such that the nucleic acid(s) of interest remain in the test sample, preferably at a concentration proportional to that in an untreated test sample (e.g., namely, a sample that is not subjected to any such pretreatment method(s)). Depending on the type of sample used, additional processing and/or purification steps may be performed to obtain nucleic acid fragments of a desired purity or size, using processing methods including but not limited to sonication, nebulization, gel purification, PCR purification systems, nuclease cleavage, size-specific capture or exclusion, targeted capture or a combination of these methods. Optionally, cell-free DNA may be isolated from the sample prior to further analysis. In some embodiments, the sample is from the subject whose disease or condition is to be determined by the systems and methods of the disclosure, also referred as "a test sample."

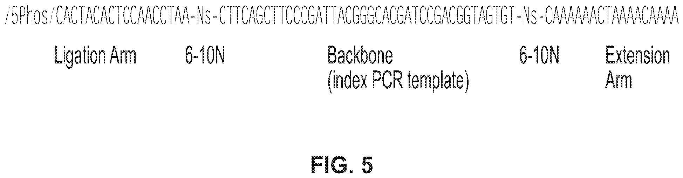

The term "MIP," as used herein, refers to a molecular inversion probe (also known as a circular capture probe). As used herein, the term "primer", "probe", or "capture probe" also may refer to a MIP in the context of their ability to selectively bind to nucleic acid molecules. Molecular inversion probes are nucleic acid molecules that contain two targeting polynucleotide arms, one or more unique molecular tags (also known as unique molecular identifiers (UMID's)), and a polynucleotide linker (e.g., a universal backbone linker). A polynucleotide linker can range from 10, 20, 30, 40, 50, 60, 70, 80, 90, 100, 125, 150, 175, 200, 225, 250, 275, 300, 400, 500, 1000, 1500, 2000 or more bases. See, for example, FIG. 5. In some embodiments, a MIP may comprise more than one unique molecular tags, such as, two unique molecular tags, three unique molecular tags, or more. In some embodiments, the polynucleotide arms in each MIP are located at the 5' and 3' ends of the MIP, while the unique molecular tag(s) and the polynucleotide linker are located in the middle. For example, in some embodiments, the MIPs comprise in sequence the following components: first targeting polynucleotide arm - first unique molecular tag - polynucleotide linker - second unique molecular tag - second targeting polynucleotide arm. In some embodiments, the polynucleotide linker (or the backbone linker) in the MIPs are universal in all the MIPs used in a method of the invention. In some embodiments, the MIPs may not comprise any unique molecular tags.

In the MIPs, the polynucleotide arms are designed to hybridize upstream and downstream of target sequences (or sites) in a genomic nucleic acid sample. These polynucleotide arms are substantially complementary to one or more repeat sequences in a genomic nucleic acid sample that flank a target sequence (herein referred to as a "gap sequence" or a "unique gap sequence". In some embodiments, the gap sequences are 1, 2, 3, 4, 5, 6, 7, 8, 9, 10, 11, 12, 13, 14, 15, 16, 17, 18, 19, 20, 25, 30, 40, 50, 60, 70, 80, 90, 100, 100, 125, 150, 175, 200, 225, 250, 275, 300, 400, 500, 1000, 1500, 2000 bases or greater in length. When interrogating cell-free DNA, the gap sequences are generally less than 150 or 200 bases in length. In some embodiments, the targeting polynucleotide arms comprise a ligation sequence and an extension sequence. A MIP may comprise targeting polynucleotide arms that are substantially complementary to a plurality of repeat sequences in a DNA sample. For example, a MIP can hybridize to tens, hundreds, thousands, hundreds of thousands, or millions of target sequences of interest in a DNA sample (e.g., a sample comprising a human genome). In some embodiments, a MIP targets, for example, greater than 1,000, greater than 10,000, greater than 20,000, greater than 30,000, greater than 40,000, greater than 50,000, greater than 60,000, greater than 70,000, greater than 80,000, greater than 90,000, greater than 100,000, greater than 200,000, greater than 300,000, greater than 400,000, greater than 500,000, greater than 600,000, greater than 700,000, greater than 800,000, greater than 900,000, and/or greater than 1,000,000 target sequences of interest. In some embodiments, "substantially complementary" refers to 0 mismatches in both arms, or at most 1 mismatch in only one arm (e.g., when the targeting polynucleotide arms hybridize to the first and second regions in the nucleic acid that, respectively, flank a site of interest). In some embodiments, "substantially complementary" refers to at most a small number of mismatches in both arms, such as 1, 2, 3, 3, 5, 6, 7, or 8.

The terms "target sequence", "sequence of interest", and "target sequence of interest" are used interchangeably to refer to the sequence bound or captured by the primes or probes of the disclosure and comprise one or more CpG sites. The target sequences of interest are selected to, among other considerations, comprise at least one CpG site; however, not every nucleic acid sequence captured by the primers or probes of the disclosure comprises a CpG site. In some embodiments, 30% or more, 31% or more, 32% or more, 33% or more, 34% or more, 35% or more, 36% or more, 37% or more, 38% or more, 39% or more, 40% or more, 41% or more, 42% or more, 43% or more, 44% or more, 45% or more, 46% or more, 47% or more, 48% or more, 49% or more, 50% or more, 51% or more, 52% or more, 53% or more, 54% or more, 55% or more, 56% or more, 57% or more, 58% or more, 59% or more, 60% or more, 61% or more, 62% or more, 63% or more, 64% or more, 65% or more, 66% or more, 67% or more, 68% or more, 69% or more, 70% or more of the nucleic acid sequences captured by the primers or probes of the disclosure comprise one or more CpG sites. These target sequences of interest may include the repeat sequences to which the targeting polynucleotide arms hybridize, as well as the gap sequences flanked by the repeat sequences. In certain embodiments, the repeat sequences have 0, 1, 2, 3, 4, or more mismatches in hybridizing with the targeting polynucleotide arms. In some embodiments the same repeat sequences are found in a target sequence of interest, in which case the target polynucleotide arms are identical. In other embodiments, two different repeat sequences (e.g., repeat A and repeat B) are found in a target sequence of interest, in which case the target polynucleotide arms are not identical. In specific embodiments, the repeat sequences have 0 or 1 mismatches in hybridizing with the targeting polynucleotide arms. In some embodiments, a MIP binds to Alu repeats. In some embodiments, a MIP does not bind long interspersed nucleotide elements (LINE) in the genome.

In some embodiments, the unique molecular tags are short nucleotide sequences that are randomly generated. In certain embodiments, the unique molecular tags are not designed to hybridize to any sequence or site located on a genomic nucleic acid fragment or in a genomic nucleic acid sample. In certain embodiments, the unique molecular tag is any tag with a suitable detectable label that can be incorporated into or attached to a nucleic acid (e.g., a polynucleotide) that allows detection and/or identification of nucleic acids that comprise or attach to the tag. In certain embodiments, unique molecular tags of sufficient length are introduced at concentrations to ensure that each MIP comprises a unique combination of molecular tags, thereby making each capture event distinct. By tracking individual capture events, one is able to identify duplicates and reduce capture bias. Although the invention described herein already allows for nearly uniform capture efficiencies since the same capture probe is used to interrogate many sites across the genome, the ability to account for capture bias (i.e., normalizing for differences in capture efficiency), further improves the quantitative aspects of the assay, for example, when doing CNV analysis. In some embodiments the tag is incorporated into or attached to a nucleic acid during a sequencing method (e.g., by a polymerase). Non-limiting examples of tags include nucleic acid tags, nucleic acid indexes or barcodes, a radiolabel (e.g., an isotope), metallic label, a fluorescent label, a chemiluminescent label, a phosphorescent label, a fluorophore quencher, a dye, a protein (e.g., an enzyme, an antibody or part thereof, a linker, a member of a binding pair), the likes or combinations thereof. In some embodiments the tag (e.g., a nucleic acid index or barcode) is a unique, known and/or identifiable sequence of nucleotides or nucleotide analogues. In some embodiments the tags or UMID's help reduce or remove amplification errors and sequencing errors by allowing for the identification of unique molecules during bioinformatics analysis. In some embodiments tags are four, five, or six or more contiguous nucleotides. Unique molecular identifiers comprising oligonucleotides are described in U.S. Patent Application No. 11/186,636, which published as US20070020640A1, and the use of oligonucleotide unique molecular identifiers with MIPs is described in U.S. Patent Application No. 12/027,039, which published as US20080269068A1. A multitude of fluorophores are available with a variety of different excitation and emission spectra. Any suitable type and/or number of fluorophores can be used as a tag. In some embodiments 1 or more, 2 or more, 3 or more, 4 or more, 5 or more, 6 or more, 7 or more, 8 or more, 9 or more, 10 or more, 20 or more, 30 or more, 50 or more, 100 or more, 500 or more, 1000 or more, 10,000 or more, 100,000 or more, $10^6$ or more, $10^7$ or more, $10^8$ or more, $10^9$ or more, $10^{10}$ or more, $10^{11}$ or more, $10^{12}$ or more different tags are utilized in a method described herein (e.g., a nucleic acid detection and/or sequencing method). In some embodiments, one or two types of tags (e.g., fluorescent labels) are linked to each nucleic acid in a library. In some embodiments, chromosome-specific tags are used to make chromosomal counting faster or easier. Detection and/or quantification of a tag can be performed by a suitable method, machine or apparatus, non-limiting examples of which include flow cytometry, quantitative polymerase chain reaction (qPCR), gel electrophoresis, a luminometer, a fluorometer, a spectrophotometer, a suitable gene-chip or microarray analysis, Western blot, mass spectrometry, chromatography, cytofluorimetric analysis, fluorescence microscopy, a suitable fluorescence or digital imaging method, confocal laser scanning microscopy, laser scanning cytometry, affinity chromatography, manual batch mode separation, electric field suspension, a suitable nucleic acid sequencing method and/or nucleic acid sequencing apparatus, the like and combinations thereof. In particular embodiments, the tag is suitable for use with microarray analysis.

The MIPs are introduced to nucleic acids (e.g., nucleic acid fragments) to perform capture of target sequences or sites located on a nucleic acid sample (e.g., a genomic DNA). In some embodiments, for example, if genomic DNA is present in a sample, fragmenting may aid in capture of target nucleic acid by molecular inversion probes. As described in greater detail herein, after capture of the target sequence (e.g., locus) of interest, the captured target may further be subjected to an enzymatic gap-filling and ligation step, such that a copy of the target sequence is incorporated into a circle, which is herein referred to as a replicon. Capture efficiency of the MIP to the target sequence on the nucleic acid fragment can be improved by lengthening the hybridization and gap-filing incubation periods. (See, e.g., Turner E H, et al., Nat Methods. 2009 Apr. 6:1-2.).

MIP technology may be used to detect or amplify particular nucleic acid sequences in complex mixtures. One of the advantages of using the MIP technology is in its capacity for a high degree of multiplexing, which allows thousands of target sequences to be captured in a single reaction containing thousands of MIPs. Various aspects of MIP technology are described in, for example, Hardenbol et al., "Multiplexed genotyping with sequence-tagged molecular inversion probes," Nature Biotechnology, 21(6): 673-678 (2003); Hardenbol et al., "Highly multiplexed molecular inversion probe genotyping: Over 10,000 targeted SNPs genotyped in a single tube assay," Genome Research, 15: 269-275 (2005);

Burmester et al., "DMET microarray technology for pharmacogenomics-based personalized medicine," Methods in Molecular Biology, 632: 99-124 (2010); Sissung et al., "Clinical pharmacology and pharmacogenetics in a genomics era: the DMET platform," Pharmacogenomics, 11(1): 89-103 (2010); Deeken, "The Affymetrix DMET platform and pharmacogenetics in drug development," Current Opinion in Molecular Therapeutics, 11(3): 260-268 (2009); Wang et al., "High quality copy number and genotype data from FFPE samples using Molecular Inversion Probe (MIP) microarrays," BMC Medical Genomics, 2:8 (2009); Wang et al., "Analysis of molecular inversion probe performance for allele copy number determination," Genome Biology, 8(11): R246 (2007); Ji et al., "Molecular inversion probe analysis of gene copy alternations reveals distinct categories of colorectal carcinoma," Cancer Research, 66(16): 7910-7919 (2006); and Wang et al., "Allele quantification using molecular inversion probes (MIP)," Nucleic Acids Research, 33(21): e183 (2005). See also in U.S. Pat. Nos. 6,858,412; 5,817,921; 6,558,928; 7,320,860; 7,351,528; 5,866,337; 6,027,889 and 6,852,487.

MIP technology has previously been successfully applied to other areas of research, including the novel identification and subclassification of biomarkers in cancers. See, e.g., Brewster et al., "Copy number imbalances between screen- and symptom-detected breast cancers and impact on disease-free survival," Cancer Prevention Research, 4(10): 1609-1616 (2011); Geiersbach et al., "Unknown partner for USP6 and unusual SS18 rearrangement detected by fluorescence in situ hybridization in a solid aneurysmal bone cyst," Cancer Genetics, 204(4): 195-202 (2011); Schiffman et al., "Oncogenic BRAF mutation with CDKN2A inactivation is characteristic of a subset of pediatric malignant astrocytomas," Cancer Research, 70(2): 512-519 (2010); Schiffman et al., "Molecular inversion probes reveal patterns of 9p21 deletion and copy number aberrations in childhood leukemia," Cancer Genetics and Cytogenetics, 193(1): 9-18 (2009); Press et al., "Ovarian carcinomas with genetic and epigenetic BRCA1 loss have distinct molecular abnormalities," BMC Cancer, 8:17 (2008); and Deeken et al., "A pharmacogenetic study of docetaxel and thalidomide in patients with castration-resistant prostate cancer using the DMET genotyping platform," Pharmacogenomics, 10(3): 191-199 (2009).

MIP technology has also been applied to the identification of new drug-related biomarkers. See, e.g., Caldwell et al., "CYP4F2 genetic variant alters required warfarin dose," Blood, 111(8): 4106-4112 (2008); and McDonald et al., "CYP4F2 Is a Vitamin K1 Oxidase: An Explanation for Altered Warfarin Dose in Carriers of the V433M Variant," Molecular Pharmacology, 75: 1337-1346 (2009). Other MIP applications include drug development and safety research. See, e.g., Mega et al., "Cytochrome P-450 Polymorphisms and Response to Clopidogrel," New England Journal of Medicine, 360(4): 354-362 (2009); Dumaual et al., "Comprehensive assessment of metabolic enzyme and transporter genes using the Affymetrix Targeted Genotyping System," Pharmacogenomics, 8(3): 293-305 (2007); and Daly et al., "Multiplex assay for comprehensive genotyping of genes involved in drug metabolism, excretion, and transport," Clinical Chemistry, 53(7): 1222-1230 (2007). Further applications of MIP technology include genotype and phenotype databasing. See, e.g., Man et al., "Genetic Variation in Metabolizing Enzyme and Transporter Genes: Comprehensive Assessment in 3 Major East Asian Subpopulations With Comparison to Caucasians and Africans," Journal of Clinical Pharmacology, 50(8): 929-940 (2010).

The term "capture" or "capturing", as used herein, refers to the binding or hybridization reaction between a primer or probe (e.g., molecular inversion probe) and the corresponding targeting site.

The term "sensitivity", as used herein, refers to a statistical measure of performance of an assay (e.g., method, test), calculated by dividing the number of true positives by the sum of the true positives and the false negatives.

The term "specificity", as used herein, refers to a statistical measure of performance of an assay (e.g., method, test), calculated by dividing the number of true negatives by the sum of true negatives and false positives.

The term "amplicon", as used herein, refers to a nucleic acid generated via capturing reactions or amplification reactions. In some embodiments, the amplicon is a single-stranded nucleic acid molecule. In some embodiments, the amplicon is a single-stranded circular nucleic acid molecule. In some embodiments, the amplicon is a double-stranded nucleic acid molecule. For example, a MIP captures or hybridizes to a target sequence or site. After the capturing reaction or hybridization, a ligation/extension mixture is introduced to extend and ligate the gap region between the two targeting polynucleotide arms to form a single-stranded circular nucleotide molecule, i.e., a MIP replicon. The gap-filled sequence in the replicon can be thought of as an "insert" or "insert sequence". The MIP replicon may be amplified through a polymerase chain reaction (PCR) to produce a plurality of MIP amplicons, which are double-stranded nucleotide molecules. MIP replicons and amplicons can be produced from a first plurality of target sequences of interest (e.g., a sequence containing known or suspected CpG sites) and a second plurality of target sequences of interest (e.g., target sequences distributed throughout the genome).

The term "sequencing", as used herein, is used in a broad sense and may refer to any technique known in the art that allows the order of at least some consecutive nucleotides in at least part of a nucleic acid to be identified, including without limitation at least part of an extension product or a vector insert. Sequencing also may refer to a technique that allows the detection of differences between nucleotide bases in a nucleic acid sequence. Exemplary sequencing techniques include targeted sequencing, single molecule real-time sequencing, electron microscopy-based sequencing, transistor-mediated sequencing, direct sequencing, random shotgun sequencing, Sanger dideoxy termination sequencing, targeted sequencing, exon sequencing, whole-genome sequencing, sequencing by hybridization (e.g., in an array such as a microarray), pyrosequencing, capillary electrophoresis, gel electrophoresis, duplex sequencing, cycle sequencing, single-base extension sequencing, solid-phase sequencing, high-throughput sequencing, massively parallel signature sequencing, emulsion PCR, co-amplification at lower denaturation temperature-PCR (COLD-PCR), multiplex PCR, sequencing by reversible dye terminator, paired-end sequencing, near-term sequencing, exonuclease sequencing, sequencing by ligation, short-read sequencing, single-molecule sequencing, sequencing-by-synthesis, real-time sequencing, reverse-terminator sequencing, ion semiconductor sequencing, nanoball sequencing, nanopore sequencing, 454 sequencing, Solexa Genome Analyzer sequencing, miSeq (Illumina), HiSeq 2000 (Illumina), HiSeq 2500 (Illumina), Illumina Genome Analyzer (Illumina), Ion Torrent PGM™ (Life Technologies), MinION™ (Oxford Nanopore Technologies), real-time SMRT™ technology (Pacific Biosciences), the Probe-Anchor Ligation (cPAL™) (Complete Genomics/BGI), SOLiD® sequencing, MS-PET sequencing, mass spectrometry, and a combination thereof. In some embodiments, sequencing comprises detecting the sequencing product using an instrument, for example but not limited to an ABI PRISM® 377 DNA Sequencer, an ABI PRISM® 310, 3100, 3100-Avant, 3730, or 373OxI Genetic Analyzer, an ABI PRISM® 3700 DNA Analyzer, or an Applied Biosystems SOLiD™ System (all from Applied Biosystems), a Genome Sequencer 20 System (Roche Applied Science), or a mass spectrometer. In certain embodiments, sequencing comprises emulsion PCR. In certain embodiments, sequencing comprises a high throughput sequencing technique, for example but not limited to, massively parallel sequencing (MPS).

The methods and compositions described herein may alternatively employ microarray technology to quantify MIPs products. "Microarray" or "array" refers to a solid phase support having a surface, preferably but not exclusively a planar or substantially planar surface, which carries an array of sites containing nucleic acids such that each site of the array comprises substantially identical or identical copies of oligonucleotides or polynucleotides and is spatially defined and not overlapping with other member sites of the array; that is, the sites are spatially discrete. The array or microarray can also comprise a non-planar interrogatable structure with a surface such as a bead or a well. The oligonucleotides or polynucleotides of the array may be covalently bound to the solid support, or may be non-covalently bound. Conventional microarray technology is reviewed in, e.g., Schena, Ed., Microarrays: A Practical Approach, IRL Press, Oxford (2000). "Array analysis", "analysis by array" or "analysis by microarray" refers to analysis, such as, e.g., sequence analysis, of one or more biological molecules using a microarray. In some embodiments each sample is hybridized individually to a single microarray. In other embodiments, processing through-put can be enhanced by physically connecting multiple microarrays onto a single multi-microarray plate for convenient high-throughput handling. In certain embodiments, custom DNA microarrays, for example from Affymetrix Inc. (Santa Clara,Calif., USA), can be manufactured to specifically quantify products of the MIPs assay.

It will be understood by one of ordinary skill in the art that the compositions and methods described herein may be adapted and modified as is appropriate for the application being addressed and that the compositions and methods described herein may be employed in other suitable applications, and that such other additions and modifications will not depart from the scope hereof.

This invention will be better understood from the Experimental Details which follow. However, one skilled in the art will readily appreciate that the specific methods and results discussed are merely illustrative of the invention as described more fully in the embodiments that follow thereafter.

Methods for detecting methylation status

Existing methods for detecting methylation status employ whole genome sequencing techniques, which inherently require a large amount of input DNA and large numbers of reads to achieve coverage of desired methylation sites. Embodiments of the present invention provide a solution to the problems of existing methylation detection methods. These embodiments replace previous library preparations with a capture method using a small number of oligonucleotide MIPs comprising targeting polynucleotide arms that hybridize to repeat sequences, said arms being arms attached to high performance universal backbone structures. These MIPs are designed to flank and incorporate uniquely aligning sequences over the entire human genome, but are enriched for targets pertinent to methylation (i.e., targets containing CpG sites). CpG sites are sites located throughout the genome where methylation occurs at the cytosine nucleotide of the site. By performing sulphonation, hydrolytic deamination and desulphonation (e.g., bisulfite conversion, or simply deamination), unmethylated cytosines are converted to uracils. By contrast, methylated cytosines are protected from this reaction, and, thus, remain cytosines. Therefore, by performing a bisulfite conversion or an alternative procedure that preserves methylated cytosines, and subsequently sequencing CpG sites, the embodiments described herein provide a method of detecting whether a CpG site was methylated or not. Contemplated methods of selecting capture molecules enable the selection of unique sequences in a desired area for quantitation, and do not rely on the presence of some unique sequences in the amplification of convenient repeat sequences.

The use of repeat sequences in the optimized capture method allows dense tiling of a target area with little or no interference of similar sequences in the production of barcoded targets for single molecule kinetics during library preparation. In some embodiments, the method has economic benefits over previous methods. In particular, these methods provide savings from the use of a small number of capture reagents (primers or probes) that still are capable of surveying genome-wide indices. Moreover, the capture reagents can provide information not only about methylation status, but also more generally about the sequences of the target sites. This information can be used to determine, for example, copy number variation or mutational landscape. This information also can be used to detect chromosomal abnormalities, e.g., aneuploidies such as trisomy, or tissue-specific methylation scores and patterns along with disease or condition-specific mutational landscapes or patterns, e.g., the presence of tissue-specific circulating tumor DNA (ctDNA) in blood.

In some embodiments, the methods also provide a rapid analysis with a low read count in an assay that is easily multiplexed. For example, multiple layers of unique molecular tags and/or barcodes can be used within the methods to identify specific primer species as well as to deconvolute multiplex data to trace signals back to individual samples. For example, a first population of MIPs can be used to obtain methylation status (and optionally, sequence information), while a second population of MIPs provides sequence information. Moreover, the methods can be used in ultra low coverage applications such as detecting trisomies in a 100% fetal sample, such as a product of conception, or a non-fetal diagnostic sample. A sample can be mixed (e.g., fetal vs. maternal or diseased vs. non-diseased) or not mixed (e.g., an individual suspected of having a disease or condition), in which case the "coverage" or read depth can be lower because the signal will be strong. In some embodiments, the methods also are fast as compared to whole genome sequencing, whole exome sequencing, and targeted sequencing. The methods described herein also offer the advantage of requiring relatively small amounts of input DNA as compared to whole genome bisulfite sequencing, which suffers from input DNA loss during the harsh bisulfite conversion process, whereas the methods described herein allow for the capture of bisulfite converted DNA after the conversion step thereby preserving input DNA and reducing bias. More specifically, most library prep kits require double-stranded input DNA for an adapter ligation step. Since bisulfite conversion denatures the DNA, the bisulfite conversion step needs to be performed after the adapter ligation step, but before PCR. The harsh bisulfite conversion can compromise some of the ligated molecules, and thereby make them unusable. Also, using conventional methods, the ligation adapters need to be methylated, otherwise the cytosines will be converted, which adds additional cost.

In some embodiments, the methods are related to the field of genetic analysis. In general, these methods can be used as a rapid and economical means to detect and quantify methylation status. Because the methylation status can be determined by sequencing, the sequence information obtained by the methods described herein allows for detection of mutations as well as detection of deletions and duplications of genetic features in a range extending from complete chromosomes and arms of chromosomes to microscopic deletions and duplications, submicroscopic deletions and deletions, and even single nucleotide features including single nucleotide polymorphisms, deletions, and insertions. In certain embodiments, these methods can be used to detect sub-chromosomal genetic lesions, e.g., microdeletions. Moreover, the methods can be used to determine mutations or other sequence elements that correlate to a disease or condition (e.g., by detecting a SNP or SNPs). Because the methods provide different types of information in a single assay, they are simpler, more efficient, and less expensive than current methods. In certain embodiments, the methods also provide a maximum likelihood estimate (k) which will allow for increased accuracy and an estimation of the probe capture efficiency and reduces need for extraneous sequencing during copy number variation (CNV) detection. This may result in a low coefficient of variation (CV) due to probe uniformity because a small number of probes (e.g., one, two or more) is used. These capture probes allow the combination of additional probes with no interference or cross assay reactions. Combining the information from several probes and their unique reads greatly reduces error in the system. Indeed, targeted probe addition can greatly enhance assay utility while reducing cost.

The methods provided by some embodiments have particular advantages as compared to targeted sequencing. In certain embodiments, the methods described herein use a simultaneous recognition of two sequence elements at the point of capture, and the two arms are limited by proximity. By contrast, a typical targeted sequencing method will allow a polymerase to initiate at a single site. The run on-product created by typical sequencing produces inefficiency, but may also produce internal or "off-target priming" with the second primer. The inherent "dual recognition" of the nucleic acids of some embodiments increases stringency, an effect which carries over into the quantitation by the molecular identifier element in the MIP structure. A unique molecular tag may be placed at one site in the MIP backbone, but in standard targeted sequencing using a molecular identifier, a random sequence is used in both primers. Also, the methods allow for lower reagent costs since coverage across the genome can be achieved with very few MIPs compared to the hundreds or thousands of multiplexed, PCR primers required for targeted sequencing. Nevertheless, the methods enjoy most, if not all, of the economic and performance advantages that targeted sequencing displays over shotgun methods.

In sum, the methods and nucleic acids of some embodiments offer clear advantages over previously described genetic methods. For example, whole genome sequencing and massively parallel signature sequencing generally require costly analysis of large, non-informative portions of the genome; whereas the present methods can produce similar answers using a fraction of the genome, thereby reducing assay costs and time. Other approaches rely on selectively assaying informative portions of the genome. While certain aspects share some similarity, the methods, in some embodiments, use a novel, comprehensive approach for identifying repeat, primer-binding sites that allow for greater assay design parameters (sequence agnostic - for example, not limited to repeat line elements), more candidate primers (e.g.,

because all potential primers are enumerated), simple, lower cost assays that are specific and sensitive enough for clinical utility, and a greater ability to multiplex.

The compositions and methods described herein can be used to assemble methylomes through the sequence analysis of plasma DNA. The ability to determine the placental or fetal methylome from maternal plasma provides a noninvasive method to determine, detect and monitor the aberrant methylation profiles associated with pregnancy-related conditions such as preeclampsia, intrauterine growth restriction, preterm labor and others. For example, the detection of a disease-specific aberrant methylation signature allows the screening, diagnosis and monitoring of such pregnancy-associated conditions. The measuring of the maternal plasma methylation level allows the screening, diagnosis and monitoring of such pregnancy-associated conditions. Besides the direct applications on the investigation of pregnancy-associated conditions, the approach could be applied to other areas of medicine where plasma DNA analysis is of interest. For example, the methylomes of cancers could be determined from plasma DNA of cancer patients. Cancer methylome analysis from plasma, as described herein, is potentially a synergistic technology to cancer genomic analysis from plasma (e.g., the detection of well-known cancer-associated somatic mutations).

For earlier cancer detection, the determination of a methylation state could be used to screen for cancer. When the methylation test ratio of a plasma sample shows aberrant levels compared with healthy controls (reference ratio), cancer may be suspected. Using the compositions and methods described herein, further confirmation and assessment of the type of cancer or tissue-of-origin of the cancer may be performed. The compositions and methods described herein also allow for the detection of tumor-associated copy number aberrations, chromosomal translocations and single nucleotide variants across the genome (mutational landscape). In some embodiments, radiological and imaging investigations (e.g. computed tomography, magnetic resonance imaging, positron emission tomography) or endoscopy (e.g. upper gastrointestinal endoscopy or colonoscopy) can be used to further investigate individuals who are suspected of having cancer based on the plasma methylation scores.

For cancer screening or detection, the determination of a methylation score of a plasma (or other biologic) sample can be used in conjunction with other modalities for cancer screening or detection such as prostate specific antigen measurement (e.g. for prostate cancer), carcinoembryonic antigen (e.g. for colorectal carcinoma, gastric carcinoma, pancreatic carcinoma, lung carcinoma, breast carcinoma, medullary thyroid carcinoma), alpha fetoprotein (e.g. for liver cancer or germ cell tumors), CA125 (e.g. for ovarian and breast cancer) and CA19-9 (e.g. for pancreatic carcinoma).

Additionally, other tissues may be sequenced to obtain a cellular methylome. For example, liver tissue can be analyzed to determine a methylation pattern specific to the liver, which may be used to identify liver pathologies. Other tissues which can also be analyzed include brain cells, bones, the lungs, the heart, the muscles and the kidneys, etc. The methylation profiles of various tissues may change from time to time, e.g. as a result of development, aging, disease processes (e.g. inflammation or cirrhosis or autoimmune processes (such as in systemic lupus erythematosus)) or treatment (e.g. treatment with demethylating agents such as 5-azacytidine and 5-azadeoxycytidine). The dynamic nature of DNA methylation makes such analysis potentially valuable for monitoring of physiological and pathological processes. For example, if one detects a change in the plasma methylome of an individual compared to a baseline value obtained when they were healthy, one could then detect disease processes in organs that contribute plasma DNA.

Also, the methylomes of transplanted organs could be determined from plasma DNA of organ transplantation recipients. As plasma DNA is generally regarded as a marker of cell death, an increase in the plasma level of DNA released from a transplanted organ could be used as a marker for increased cell death from that organ, such as a rejection episode or other pathologic processes involving that organ (e.g. infection or abscess). In the event that anti-rejection therapy is successfully instituted, the plasma level of DNA released by the transplanted organ will be expected to decrease.

Exemplary applications of the methods include the detection, diagnosis, prognosis, recurrence, minimum residual risk assessment of methylation-associated diseases and conditions. For example, applications might include a method of determining whether a subject has a predisposition to a disease or condition that is associated with the methylation state of a nucleic acid; a method of diagnosing a disease or condition in a subject, said disease or condition being associated with the methylation state of a nucleic acid; a method of detecting the state of a disease or condition in a subject, said disease or condition being associated with the methylation state of a nucleic acid. Particular diseases and conditions include, for example, cancers. A hallmark of cancer cells is that they divide more rapidly than non-cancer cells. Thus, cancer cells and non-cancer cells will have different methylation patterns. The embodiments and methods described herein provide an assay for methylation, CNV and mutational landscape status in tumor biopsy or circulating tumor DNA. In particular, the embodiments and methods can be used to provide a diagnosis, prognosis, staging, and/or likelihood of developing cancers such as, for example, prostate cancer, colorectal cancer, lung cancer, breast cancer, liver cancer, or bladder cancer. Certain embodiments provide a diagnosis, or staging or prognostic information about a cancer, or to inform a treatment decision, or to assess minimum residual risk and recurrence. Conditions known to be affected by methylation, or known to affect methylation, include but are not limited to, aging, diet, lifestyle, ethnicity, development, bipolar disorder, multiple sclerosis, diabetes, schizophrenia, cancer, neurodegenerative diseases, inflammation, lesion, infection, immune response, exposure to: drugs, alcohol, tobacco, pesticides, heavy metals, radiation, UV other environmental factors. In some embodiments, the methods provided herein may provide the methylation status and sequence information of circulating cell-free fetal DNA, for example, as a noninvasive prenatal test. A noninvasive prenatal test using the methods described herein can be used, for example, to determine a risk for preeclampsia or preterm parturition. Additional tests using the methods described herein include pediatric diagnosis of aneuploidy, testing for product of conception or risk of premature abortion, noninvasive prenatal testing (both qualitative and quantitative genetic testing, such as detecting Mendelian disorders, insertions/deletions, and chromosomal imbalances), testing preimplantation genetics, tumor characterization, postnatal testing including cytogenetics, and mutagen effect monitoring.

Another exemplary application of the methods includes a method of differentiating nucleic acid species originating from a subject and one or more additional individuals, said subject and one or more additional individuals having differing methylation states of a nucleic acid. For example, the subject may be a pregnant female and the one or more additional individuals may be an unborn fetus. In these embodiments, the blood sample may be maternal plasma or maternal serum. Alternatively, the subject may be a tissue transplant recipient, and the one or more additional individuals may be a tissue transplant donor.

Another exemplary application of the methods includes a method of differentiating nucleic acid species originating from different tissues in a subject. For example, the methods of the invention may be used to differentiate between a tissue of interest (e.g., a tissue of fetal, tumor, or disease origin) and other tissues (e.g., maternal, non-tumor, or disease-free origin). In some embodiments, the subject may be a pregnant female. In these embodiments, the blood sample may be maternal plasma or maternal serum. Alternatively, the subject may be a tissue transplant recipient or a cancer patient.

Another exemplary application of the methods includes a method of determining the age or "bio-age" or "methylation age" of a subject or group of subjects. More specifically, an individual's genetic material is known to change over time, and the methods described herein allow for the methylation-based age determination of genetic material from an individual by determining the methylation status of thousands or hundreds of thousands of CpG sites in a single assay. This has utility both for forensic purposes and for age-related pathologies such as Alzheimer's disease. The methods are also useful for determining the age of a specific tissue. As described further in the Examples, the methods are useful for determining the "bio-age" for specific tissues such as colorectal tissue or the gestational age of a fetus.

The capture primers and probes (e.g., MIPs) in some embodiments also have the benefit of increased binding stability as compared to conventional PCR primer pairs that are not part of the same molecule. In certain embodiments, the exact targeting arm sequences are somewhat short for PCR primers, and hence will have very low melting temperatures in a PCR context. However, in a MIPs configuration, the primers will enhance binding specificity by cooperating to stabilize the interaction. If one arm has a high binding efficiency, the capture is enhanced even if the opposite arm has a lower efficiency. The additive length of the pair improves the "on/off" equilibrium for capture because the lower efficiency arm is more often in proximity of its target in a MIP than it would be as a free PCR primer.

In some embodiments, a method is provided for determining whether a subject has a predisposition to a disease or condition that is associated with the methylation state of a nucleic acid. In some embodiments, the disclosure provides a method for diagnosing a disease or condition in a subject, said disease or condition being associated with the methylation state of a nucleic acid. In some embodiments, the disclosure provides a method for detecting the state of a disease or condition in a subject, said disease or condition being associated with the methylation state of a nucleic acid. In certain embodiments, these methods comprise:

a) obtaining a nucleic acid sample isolated from a blood sample from the subject;
b) performing bisulfite conversion of the nucleic acid sample;
c) capturing a plurality of target sequences of interest in the nucleic acid sample obtained in step b) by using one or more populations of molecular inversion probes (MIPs) to produce a plurality of replicons,
wherein each of the MIPs in the population of MIPs comprises in sequence the following components:

first targeting polynucleotide arm - first unique molecular tag - polynucleotide linker - second unique molecular tag - second targeting polynucleotide arm;
wherein the first targeting polynucleotide arm in each of the MIPs is substantially complementary to a first repeat region, and the second targeting polynucleotide arm in each of the MIPs is substantially complementary to a second repeat region, further wherein the first and second repeat regions, respectively, flank each sequence in the plurality of target sequences of interest;
wherein the first and second unique targeting molecular tags in each of the MIPs in combination are distinct in each of the MIPs;
wherein each target sequence of interest in the plurality of target sequences of interest has one or more CpG sites;
wherein each CpG site has a corresponding known position within the target sequence of interest;

d) sequencing a plurality of MIPs amplicons that are amplified from the replicons obtained in step c);

e) determining the number of occurrences of cytosine nucleotides at each corresponding CpG site within the MIPs amplicons sequenced at step d);

f) determining a test ratio based on a first sum of the numbers of occurrences of cytosine nucleotides determined at step e) and a second sum of the known numbers of CpG sites in the plurality of target sequences of interest;

g) comparing the test ratio to a plurality of reference ratios that are computed based on reference nucleic acid samples isolated from reference subjects with the disease or condition that is associated with the methylation state of the nucleic acid; and

h) determining whether a subject is predisposed to the disease or condition, or diagnosing the disease or condition in the subject, or detecting the state of the disease or condition in the subject, based on the comparing in step g).

In some embodiments, a method is provided of differentiating nucleic acid species originating from a subject and one or more additional individuals, said subject and one or more additional individuals having differing methylation states of a nucleic acid, the method comprising:

a) obtaining a nucleic acid sample isolated from a blood sample from the subject, said blood sample comprising nucleic acids originating from the subject and one or more additional individuals;

b) performing bisulfite conversion of the nucleic acid sample;

c) capturing a plurality of target sequences of interest in the nucleic acid sample obtained in step b) by using one or more populations of molecular inversion probes (MIPs) to produce a plurality of replicons,

wherein each of the MIPs in the population of MIPs comprises in sequence the following components:

first targeting polynucleotide arm - first unique molecular tag - polynucleotide linker - second unique molecular tag - second targeting polynucleotide arm;

wherein the first targeting polynucleotide arm in each of the MIPs is substantially complementary to a first repeat region, and the second targeting polynucleotide arm in each of the MIPs is substantially complementary to a second repeat region, further wherein the first and second repeat regions, respectively, flank each sequence in the plurality of target sequences of interest;

wherein the first and second unique targeting molecular tags in each of the MIPs in combination are distinct in each of the MIPs;

wherein each target sequence of interest in the plurality of target sequences of interest has one or more CpG sites;

wherein each CpG site has a corresponding known position within the target sequence of interest;

d) sequencing a plurality of MIPs amplicons that are amplified from the replicons obtained in step c);

e) determining the number of occurrences of cytosine nucleotides at each corresponding CpG site within the MIPs amplicons sequenced at step d);

f) determining a test ratio based on a first sum of the numbers of occurrences of cytosine nucleotides determined at step e) and a second sum of the known numbers of CpG sites in the plurality of target sequences of interest;

g) comparing the test ratio to a plurality of reference ratios that are computed based on reference nucleic acid samples isolated from individuals having differing methylation states of the nucleic acid; and

h) differentiating nucleic acid species originating from the subject and the one or more additional individuals based on the comparing in step g).

In some embodiments, a method is provided of differentiating nucleic acid species originating from a first tissue and one or more additional tissues in a subject, said first tissue and one or more additional tissues having differing methylation states of a nucleic acid, the method comprising:

a) obtaining a nucleic acid sample isolated from a cell-free bodily fluid sample from the subject, said cell-free bodily fluid sample comprising nucleic acids originating from the first tissue and the one or more additional tissues;

b) performing bisulfite conversion of the nucleic acid sample;

c) capturing a plurality of target sequences of interest in the nucleic acid sample obtained in step b) by using one or more populations of molecular inversion probes (MIPs) to produce a plurality of replicons,

wherein each of the MIPs in the population of MIPs comprises in sequence the following components:

first targeting polynucleotide arm - first unique molecular tag - polynucleotide linker - second unique molecular tag - second targeting polynucleotide arm;

wherein the first targeting polynucleotide arm in each of the MIPs is substantially complementary to a first repeat region, and the second targeting polynucleotide arm in each of the MIPs is substantially complementary to a second repeat region, further wherein the first and second repeat regions, respectively, flank each sequence in the plurality of target sequences of interest;

wherein the first and second unique targeting molecular tags in each of the MIPs in combination are distinct in each of the MIPs;

wherein each target sequence of interest in the plurality of target sequences of interest has one or more CpG sites;

wherein each CpG site has a corresponding known position within the target sequence of interest;

d) sequencing a plurality of MIPs amplicons that are amplified from the replicons obtained in step c);

e) determining the number of occurrences of cytosine nucleotides at each corresponding CpG site within the MIPs amplicons sequenced at step d);

f) determining a test ratio based on a first sum of the numbers of occurrences of cytosine nucleotides determined at step e) and a second sum of the known numbers of CpG sites in the plurality of target sequences of interest;

g) comparing the test ratio to a plurality of reference ratios that are computed based on reference nucleic acid samples isolated from reference tissues having differing methylation states of the nucleic acid;

h) differentiating nucleic acid species originating from the first tissue and the one or more additional tissues based on the comparing in step g).

In some embodiments, a method is provided of determining the methylation state of a nucleic acid and detecting copy number variation in a subject, the method comprising:

a) obtaining a nucleic acid sample isolated from a blood sample from the subject;

b) performing bisulfite conversion of the nucleic acid sample;

c) capturing a plurality of target sequences of interest in the nucleic acid sample obtained in step b) by using one or more populations of molecular inversion probes (MIPs) to produce a plurality of replicons,

wherein each of the MIPs in the population of MIPs comprises in sequence the following components:

first targeting polynucleotide arm - first unique molecular tag - polynucleotide linker - second unique molecular tag - second targeting polynucleotide arm;

wherein the first targeting polynucleotide arm in each of the MIPs is substantially complementary to a first repeat region, and the second targeting polynucleotide arm in each of the MIPs is substantially complementary to a second repeat region, further wherein the first and second repeat regions, respectively, flank each sequence in the plurality of target sequences of interest;

wherein the first and second unique targeting molecular tags in each of the MIPs in combination are distinct in each of the MIPs;

wherein the target sequence of interest in the plurality of target sequences of interest has one or more CpG sites;

wherein each CpG site has a corresponding known position within the target sequence of interest;

d) sequencing a plurality of MIPs amplicons that are amplified from the replicons obtained in step c);

e) determining the number of occurrences of cytosine nucleotides at each corresponding CpG site within the MIPs amplicons sequenced at step d) to determine the methylation state of a nucleic acid; and

f) determining the number of the unique MIPs amplicons sequenced at step d); determining a read density based at least in part on the number of unique MIP amplicon sequences; and detecting copy number variation by comparing the read density to a plurality of reference read densities that are computed based on reference nucleic acid samples isolated from reference subjects.

In a further embodiment, targeting molecular tags are used to remove duplicates and thereby improve analysis. In certain embodiments, the number of unique MIP amplicon sequences is determined for defined regions to determine read density.

In some embodiments, a method is provided of determining the methylation age of a subject, the method comprising:

a) obtaining a nucleic acid sample isolated from a blood sample from the subject;

b) performing bisulfite conversion of the nucleic acid sample;

c) capturing a plurality of target sequences of interest in the nucleic acid sample obtained in step b) by using one or more populations of molecular inversion probes (MIPs) to produce a plurality of replicons,

wherein each of the MIPs in the population of MIPs comprises in sequence the following components:

first targeting polynucleotide arm - first unique molecular tag - polynucleotide linker - second unique molecular tag - second targeting polynucleotide arm;

wherein the first targeting polynucleotide arm in each of the MIPs is substantially complementary to a first repeat region, and the second targeting polynucleotide arm in each of the MIPs is substantially complementary to a second repeat region, further wherein the first and second repeat regions, respectively, flank each sequence in the plurality of target sequences of interest;

wherein the first and second unique targeting molecular tags in each of the MIPs in combination are distinct in each of the MIPs;

wherein each target sequence of interest in the plurality of target sequences of interest has one or more CpG sites;

wherein each CpG site has a corresponding known position within the target sequence of interest;

d) sequencing a plurality of MIPs amplicons that are amplified from the replicons obtained in step c);

e) determining the number of occurrences of cytosine nucleotides at each corresponding CpG site within the MIPs amplicons sequenced at step d);

f) determining a test ratio based on a first sum of the numbers of occurrences of cytosine nucleotides determined at step e) and a second sum of the known numbers of CpG sites in the plurality of target sequences of interest;

g) comparing the test ratio to a plurality of reference ratios that are computed based on reference nucleic acid samples isolated from reference subjects; and

h) determining the methylation age of the subject based on the comparing in step g).

In some embodiments, a method is provided of determining the methylation age of a tissue in a subject, the method comprising:

a) obtaining a nucleic acid sample isolated from a cell-free bodily fluid sample from the subject, said cell-free bodily fluid sample comprising nucleic acids originating from the tissue;

b) performing bisulfite conversion of the nucleic acid sample;

c) capturing a plurality of target sequences of interest in the nucleic acid sample obtained in step b) by using one or more populations of molecular inversion probes (MIPs) to produce a plurality of replicons,

wherein each of the MIPs in the population of MIPs comprises in sequence the following components:

first targeting polynucleotide arm - first unique molecular tag - polynucleotide linker - second unique molecular tag - second targeting polynucleotide arm;

wherein the first targeting polynucleotide arm in each of the MIPs is substantially complementary to a first repeat region, and the second targeting polynucleotide arm in each of the MIPs is substantially complementary to a second repeat region, further wherein the first and second repeat regions, respectively, flank each sequence in the plurality of target sequences of interest;

wherein the first and second unique targeting molecular tags in each of the MIPs in combination are distinct in each of the MIPs;

wherein each target sequence of interest in the plurality of target sequences of interest has one or more CpG sites;

wherein each CpG site has a corresponding known position within the target sequence of interest;

d) sequencing a plurality of MIPs amplicons that are amplified from the replicons obtained in step c);

e) determining the number of occurrences of cytosine nucleotides at each corresponding CpG site within the MIPs amplicons sequenced at step d);

f) determining a test ratio based on a first sum of the numbers of occurrences of cytosine nucleotides determined at step e) and a second sum of the known numbers of CpG sites in the plurality of target sequences of interest;

g) comparing the test ratio to a plurality of reference ratios that are computed based on reference nucleic acid samples isolated from reference tissues; and

h) determining the methylation age of the tissue based on the comparing in step g).

In some embodiments, the method is a method of determining whether a subject has a predisposition to a disease or condition that is associated with the methylation state of a nucleic acid, the method comprising:

a) obtaining a genomic DNA sample from a blood sample from the subject;

b) performing bisulfite conversion of the genomic DNA sample;

c) adding the bisulfite-converted genomic DNA sample into each well of a multi-well plate, wherein each well of the multi-well plate comprises a probe mixture, wherein the probe mixture comprises a population of molecular inversion probes (MIPs) and a buffer;

wherein each MIP in the population of MIPs comprises in sequence the following components:

first targeting polynucleotide arm - first unique molecular tag - polynucleotide linker - second unique molecular tag - second targeting polynucleotide arm;

wherein the first targeting polynucleotide arm in each of the MIPs is substantially complementary to a first repeat region, and the second targeting polynucleotide arm in each of the MIPs is substantially complementary to a second repeat region, further wherein the first and second repeat regions, respectively, flank each sequence in a plurality of target sequences of interest;

wherein the first and second unique targeting molecular tags in each of the MIPs in combination are distinct in each of the MIPs;

wherein each target sequence of interest in the plurality of target sequences of interest has one or more CpG sites;

wherein each CpG site has a corresponding known position within the target sequence of interest;

d) incubating the bisulfite-converted genomic DNA sample with the probe mixture for the MIPs to capture the plurality of target sequences of interest;

e) adding an extension/ligation mixture to the sample of d) for the MIPs and the plurality of target sequences of interest to form a plurality of MIPs replicons, wherein the extension/ligation mixture comprises a polymerase, a plurality of dNTPs, a ligase, and buffer;

f) adding an exonuclease mixture to the targeting and control MIPs replicons to remove excess probes or excess genomic DNA;

g) adding an indexing PCR mixture to the sample of f) to add a pair of sequencing adapters comprising a unique molecular tag for multiplexed sequencingto the plurality of amplicons;

h) using a massively parallel sequencing method to determine the number of occurrences of cytosine nucleotides at each corresponding CpG site within the MIPs amplicons ;

i) determining a test ration based on a first sum of the numbers of occurrences of cytosine nucleotides determined at step h) and a second sum of the known numbers of CpG sites in the plurality of target sequences of interest;

j) comparing the test ratio to a plurality of reference ratios that are computed based on reference nucleic acid samples isolated from reference subjects with the disease or condition that is associated with the methylation state of the nucleic acid; and

k) determining whether the subject is predisposed to the disease or condition based on the comparing in step j).

In some embodiments, the method is a method of diagnosing a disease or condition in a subject, the method comprising:

a) obtaining a genomic DNA sample from a blood sample from the subject;

b) performing bisulfite conversion of the genomic DNA sample;

c) adding the bisulfite-converted genomic DNA sample into each well of a multi-well plate, wherein each well of the multi-well plate comprises a probe mixture, wherein the probe mixture comprises a population of molecular inversion probes (MIPs) and a buffer;

wherein each MIP in the population of MIPs comprises in sequence the following components:

first targeting polynucleotide arm - first unique molecular tag - polynucleotide linker - second unique molecular tag - second targeting polynucleotide arm;

wherein the first targeting polynucleotide arm in each of the MIPs is substantially complementary to a first repeat region, and the second targeting polynucleotide arm in each of the MIPs is substantially complementary to a second repeat region, further wherein the first and second repeat regions, respectively, flank each sequence in a plurality of target sequences of interest;

wherein the first and second unique targeting molecular tags in each of the MIPs in combination are distinct in each of the MIPs;

wherein each target sequence of interest in the plurality of target sequences of interest has one or more CpG sites;

wherein each CpG site has a corresponding known position within the target sequence of interest;

d) incubating the bisulfite-converted genomic DNA sample with the probe mixture for the MIPs to capture the plurality of target sequences of interest;

e) adding an extension/ligation mixture to the sample of d) for the MIPs and the plurality of target sequences of interest to form a plurality of MIPs replicons, wherein the extension/ligation mixture comprises a polymerase, a plurality of dNTPs, a ligase, and buffer;

f) adding an exonuclease mixture to the targeting and control MIPs replicons to remove excess probes or excess genomic DNA;

g) adding an indexing PCR mixture to the sample of f) to add a pair of sequencing adapters comprising a unique molecular tag to the plurality of amplicons;

h) using a massively parallel sequencing method to determine the number of occurrences of cytosine nucleotides at each corresponding CpG site within the MIPs amplicons;

i) determining a test ratio based on a first sum of the numbers of occurrences of cytosine nucleotides determined at step h) and a second sum of the known numbers of CpG sites in the plurality of target sequences of interest;

j) comparing the test ratio to a plurality of reference ratios that are computed based on reference nucleic acid samples isolated from reference subjects with the disease or condition that is associated with the methylation state of the nucleic acid; and

k) diagnosing the disease or condition in the subject based on the comparing in step j).

In some embodiments, the method is a method of detecting the state of a disease or condition in a subject, said disease or condition being associated with the methylation state of a nucleic acid, the method comprising:

a) obtaining a genomic DNA sample from a blood sample from the subject;

b) performing bisulfite conversion of the genomic DNA sample;

c) adding the bisulfite-converted genomic DNA sample into each well of a multi-well plate, wherein each well of the multi-well plate comprises a probe mixture, wherein the probe mixture comprises a population of molecular inversion probes (MIPs) and a buffer;

wherein each MIP in the population of MIPs comprises in sequence the following components:

first targeting polynucleotide arm - first unique molecular tag - polynucleotide linker - second unique molecular tag - second targeting polynucleotide arm;

wherein the first targeting polynucleotide arm in each of the MIPs is substantially complementary to a first repeat region, and the second targeting polynucleotide arm in each of the MIPs is substantially complementary to a second repeat region, further wherein the first and second repeat regions, respectively, flank each sequence in a plurality of target sequences of interest;

wherein the first and second unique targeting molecular tags in each of the MIPs in combination are distinct in each of the MIPs;

wherein each target sequence of interest in the plurality of target sequences of interest has one or more CpG sites;

wherein each CpG site has a corresponding known position within the target sequence of interest;

d) incubating the bisulfite-converted genomic DNA sample with the probe mixture for the MIPs to capture the plurality of target sequences of interest;

e) adding an extension/ligation mixture to the sample of d) for the MIPs and the plurality of target sequences of interest to form a plurality of MIPs replicons, wherein the extension/ligation mixture comprises a polymerase, a plurality of dNTPs, a ligase, and buffer;

f) adding an exonuclease mixture to the targeting and control MIPs replicons to remove excess probes or excess genomic DNA;

g) adding an indexing PCR mixture to the sample of f) to add a pair of sequencing adapters comprising a unique molecular tag to the plurality of amplicons;

h) using a massively parallel sequencing method to determine the number of occurrences of cytosine nucleotides at each corresponding CpG site within the MIPs amplicons;

i) determining a test ratio based on a first sum of the numbers of occurrences of cytosine nucleotides determined at step h) and a second sum of the known numbers of CpG sites in the plurality of target sequences of interest;

j) comparing the test ratio to a plurality of reference ratios that are computed based on reference nucleic acid samples isolated from reference subjects with the disease or condition that is associated with the methylation state of the nucleic acid; and

k) detecting the state of the disease or condition in the subject based on the comparing in step j).

In certain alternative embodiments, the MIPs of the disclosure may not comprise any unique molecular tags. It is possible to determine the methylation score, copy number variation, mutational landscape, etc. using MIPs that do not contain unique molecular tags, according to the methods of the disclosure.

In alternative embodiments, the bisulfite conversion of any of the methods described herein may be replaced by another type of deamination reaction.

The above methods may also be used to detect aneuploidy in a fetal or non-fetal subject. In certain embodiments, as an alternative to detecting aneuploidy, the methods can be used to detect and quantify deletions and duplications of genetic features in arms of chromosomes, as well as microscopic deletions and duplications, submicroscopic deletions and deletions, and single nucleotide features including single nucleotide polymorphisms, deletions, and insertions.

In some embodiments, the methods of the disclosure use a single species of MIP. In alternative embodiments, the methods are useful with 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, or more species of MIPs. For example, multiple species of MIPs can be used to detect different diseases or conditions (e.g., cancer, pregnancy-related conditions such as preeclampsia or preterm parturition, or chromosomal abnormalities such as aneuploidy) in a single sample. In certain embodiments, a single MIP can be used to detect different diseases or conditions (e.g., cancer, pregnancy-related conditions such as preeclampsia or preterm parturition, or chromosomal abnormalities such as aneuploidy) in a single sample.

The skilled worker will appreciate that the lengths of the first and second targeting polynucleotide arms can be varied as appropriate to provide efficient hybridization between the targeting polynucleotide and the nucleic acid sample. For example the first and/or second targeting polynucleotide arms can be between 14 and 30 bases, e.g., 18-21 bases.

In certain embodiments, the length of the first and/or second targeting polynucleotide arms is 14, 15, 16, 17, 18, 19, 20, 21, 22, 23, 24, 25, 26, 27, 28, 29, or 30 bases. In certain embodiments, the targeting polynucleotide arms have a melting temperature ($T_M$) between 45 °C and 80 °C (e.g., 45 °C, 46 °C, 47 °C, 48 °C, 49 °C, 50 °C, 51 °C, 52 °C, 53 °C, 54 °C, 55 °C, 56 °C, 57 °C, 58 °C, 59 °C, 60 °C, 61 °C, 62 °C, 63 °C, 64 °C, or 65°C) and/or a GC content between 10% and 80% (e.g., approximately 10%, 11%, 12%, 13%, 14%, 15%, 16%, 17%, 18%, 19%, 20%, 25%, 30%, 35%, 40%, 45 %, 50%, 55%, 60%, 65%, 70%, 75%, or 80%). In certain embodiments, the targeting polynucleotide arms have a $T_M$ between 45 °C and 55 °C and/or a GC content between 15% and 25%. In certain embodiments, the targeting polynucleotide arms have a $T_M$ between 45 °C and 66 °C and/or a GC content between 10% and 40%. In some embodiments, the sequence of the first targeting polynucleotide arm is CACTACACTCCAACCTAA (SEQ ID NO:4) or TTCTCCTACCTCAACCTC (SEQ ID NO:5). In some embodiments, the sequence of the second targeting polynucleotide arm is CAAAAAACTAAAACAAAA (SEQ ID NO:6) or CCAAACTAAAATACAATA (SEQ ID NO:7). In some embodiments, the targeting polynucleotide arms target, for example, greater than 1,000, greater than 10,000, greater than 20,000, greater than 30,000, greater than 40,000, greater than 50,000, greater than 60,000, greater than 70,000, greater than 80,000, greater than 90,000, greater than100,000, greater than 200,000, greater than 300,000, greater than 400,000, greater than 500,000, greater than 600,000, greater than 700,000, greater than 800,000, greater than 900,000, and/or greater than 1,000,000 sequences of interest. In some embodiments, a MIP does not bind long interspersed nucleotide elements (LINE) in the genome.

Unique molecular tags provide a way to determine the number of capture events for a given amplicon. A MIP may comprise one or more unique molecular tags, e.g., 1, 2, 3, 4, or 5 unique molecular tags. In certain embodiments, the length of the first and/or second unique molecular tag is between 4 and 15 bases, e.g., 4, 5, 6, 7, 8, 9, 10, 11, 12, 13, 14, or 15 bases.

A polynucleotide linker bridges the gap between the two targeting polynucleotide arms. In some embodiments, the polynucleotide linker is located directly between the first and second unique molecular tags. In certain embodiments, the polynucleotide linker is not substantially complementary to any genomic region of the subject. In certain embodiments, the polynucleotide linker has a length of between 20 and 1,000 bases (e.g., 20, 25, 30, 35, 40, 45, 50, 55, 60 or 65 bases) and/or a melting temperature of between 45 °C and 85 °C (e.g., 45 °C, 50 °C, 55 °C, 60 °C, 65°C, 70°C, 75°C, 80°C, or 85°C) and/or a GC content between 10% and 80% (e.g., approximately 10%, 15%, 20%, 30%, 35%, 40%, 45 %, 50%, 55%, 60%, 65%, 70%, 75%, or 80%). In certain embodiments, the polynucleotide linker comprises at least one amplification primer binding site, e.g., a forward amplification primer binding site. In some embodiments, the linker includes a reverse amplification primer binding site. In some embodiments, the reverse primer binding site will be generated after the first extension of the PCR with a forward amplification primer. For example, the sequence of the forward amplification primer can comprise the nucleotide sequence of CCGTAATCGGGAAGCTGAAG (SEQ ID NO:1) and/or the sequence of the reverse amplification primer can comprise the nucleotide sequence of GCACGATCCGACGGTAGTGT (SEQ ID NO:2). Thus, the nucleotide sequence of the polynucleotide linker can comprise the nucleotide sequence of CTTCAGCTTCCCGATTACGGGCACGATCCGACGGTAGTGT (SEQ ID NO:3).

In certain embodiments, the MIP comprises the nucleotide sequence of CACTACACTCCAACCTAA(N1) CTTCAGCTTCCCGATTACGGGCACGATCCGACGGTAGTGT(N2) CAAAAAACTAAAACAAAA (SEQ ID NO:8), wherein (N1) represents the first unique molecular tag and (N2) represents the second unique molecular tag. In some embodiments, the MIP comprises the nucleotide sequence of TTCTCCTACCTCAACCTC(N1) CTTCAGCTTCCCGATTACGGGCACGATCCGACGGTAGTGT(N2) CCAAACTAAAATACAATA (SEQ ID NO:9), wherein (N1) represents the first unique molecular tag and (N2) represents the second unique molecular tag. In some embodiments the MIP comprises a 5' phosphate to facilitate ligation.

In some embodiments, the population of MIPs has a concentration between 10 fM and 100 nM, for example, 0.5 nM. In certain embodiments, the concentration of MIPs used will vary with the number of sequences being targeted, e.g., as calculated by multiplying the number of target sequences of interest by the number of genomic equivalents in a reaction (the "total target number"). In particular embodiments, the approximate ratio of the number of MIP molecules to the total target number is 1:50, 1:100, 1:150, 1:200, 1:250, 1:300, 1:350, 1:400, 1:450, 1:500, 1:550, 1:600, 1:650, 1:700, 1:750, 1:800, 1:850, 1:900, 1:950, or 1:1,000. In certain embodiments, each of the MIPs replicons and/or amplicons is a single-stranded circular nucleic acid molecule.

In some embodiments, the MIPs replicons are produced by: i) the first and second targeting polynucleotide arms, respectively, hybridizing to the first and second regions in the nucleic acid sample, respectively, wherein the first and second regions flank a target sequence of interest; and ii) after the hybridization, using a ligation/extension mixture to extend and ligate the gap region between the two targeting polynucleotide arms to form single-stranded circular nucleic acid molecules. In certain embodiments, a MIP amplicon is produced by amplifying a MIP replicon, e.g., through PCR.

In some embodiments, the sequencing step comprises a next generation sequencing method, for example, a massively parallel sequencing method, or a short read sequencing method. In some embodiments, sequencing may be by any method known in the art, for example, targeted sequencing, single molecule real-time sequencing, electron microscopy-based sequencing, transistor-mediated sequencing, direct sequencing, random shotgun sequencing, Sanger dideoxy termination sequencing, targeted sequencing, exon sequencing, whole-genome sequencing, sequencing by hybridization, pyrosequencing, capillary electrophoresis, gel electrophoresis, duplex sequencing, cycle sequencing, single-base extension sequencing, solid-phase sequencing, high-throughput sequencing, massively parallel signature sequencing, emulsion PCR, co-amplification at lower denaturation temperature-PCR (COLD-PCR), multiplex PCR, sequencing by reversible dye terminator, paired-end sequencing, near-term sequencing, exonuclease sequencing, sequencing by ligation, short-read sequencing, single-molecule sequencing, sequencing-by-synthesis, real-time sequencing, reverse-terminator sequencing, nanopore sequencing, 454 sequencing, Solexa Genome Analyzer sequencing, SOLiD® sequencing, MS-PET sequencing, mass spectrometry, and a combination thereof. In some embodiments, sequencing comprises an detecting the sequencing product using an instrument, for example but not limited to an ABI PRISM® 377 DNA Sequencer, an ABI PRISM® 310, 3100, 3100-Avant, 3730, or 373OxI Genetic Analyzer, an ABI PRISM® 3700 DNA Analyzer, or an Applied Biosystems SOLiD™ System (all from Applied Biosystems), a Genome Sequencer 20 System (Roche Applied Science), or a mass spectrometer. In certain embodiments, sequencing comprises emulsion PCR. In certain embodiments, sequencing comprises a high throughput sequencing technique, for example but not limited to, massively parallel signature sequencing (MPSS).

A sequencing technique that can be used in various embodiments includes, for example, Illumina® sequencing. Illumina® sequencing is based on the amplification of DNA on a solid surface using fold-back PCR and anchored primers. Genomic DNA is fragmented, and adapters are added to the 5' and 3' ends of the fragments. DNA fragments that are attached to the surface of flow cell channels are extended and bridge amplified. The fragments become double stranded, and the double stranded molecules are denatured. Multiple cycles of the solid-phase amplification followed by denaturation can create several million clusters of approximately 1,000 copies of single-stranded DNA molecules of the same template in each channel of the flow cell. Primers, DNA polymerase and four fluorophore-labeled, reversibly terminating nucleotides are used to perform sequential sequencing. After nucleotide incorporation, a laser is used to excite the fluorophores, and an image is captured and the identity of the first base is recorded. The 3' terminators and fluorophores from each incorporated base are removed and the incorporation, detection and identification steps are repeated. Sequencing according to this technology is described in U.S. Pat. No. 7,960,120; U.S. Pat. No. 7,835,871; U.S. Pat. No. 7,232,656; U.S. Pat. No. 7,598,035; U.S. Pat. No. 6,911,345; U.S. Pat. No. 6,833,246; U.S. Pat. No. 6,828,100; U.S. Pat. No. 6,306,597; U.S. Pat. No. 6,210,891; U.S. Pub. 2011/0009278; U.S. Pub. 2007/0114362; U.S. Pub. 2006/0292611; and U.S. Pub. 2006/0024681.

Some embodiments comprise, before sequencing (e.g., the sequencing step of d) as described above), a PCR reaction to amplify the MIPs amplicons for sequencing. This PCR reaction may be an indexing PCR reaction. In certain embodiments, the indexing PCR reaction introduces into each of the MIPs amplicons the following components: a pair of indexing primers comprising a unique sample barcode and a pair of sequencing adaptors. In particular embodiments, the barcoded targeting MIPs amplicons comprise in sequence the following components in a 5' to 3' orientation:
a first sequencing adaptor - a first sequencing primer binding site - the first unique targeting molecular tag - the first targeting polynucleotide arm - captured nucleic acid - the second targeting polynucleotide arm - the second unique targeting molecular tag - a second sequencing primer binding site - a unique sample barcode - a second sequencing adaptor.

In some embodiments, the target sequences of interest are on a single chromosome. In alternative embodiments, the target sequences of interest are on multiple chromosomes. In particular embodiments, the target sequences of interest are selected at particular sites where methylation status correlates with a disease or condition. Because a single MIP sequence can be used to target sequences of interest across an entire genome, in certain embodiments the methods of the invention provide the benefit of being able to detect methylation status of more than one chromosome at a time. By selecting MIPs that provide ample coverage across the genome, the methylation status of the sequences of interest may serve as a proxy for the methylation status of a genome. Moreover, because the MIPs provide sequence information as well as methylation status, the MIPs may be used to detect 1, 2, 3, 4, 5, 6, 7, 8, 9, 10, or more conditions associated with methylation status and/or chromosomal or other sequence abnormalities.

In some embodiments, the methods provided include a method of selecting a molecular inversion probe (MIP) from a plurality of candidate MIPs for using to detect methylation in a subject, the method comprising:

a) receiving nucleic acid sequences of the plurality of candidate MIPs, wherein each of the MIPs in the plurality of candidate MIPs comprises in sequence the following components:
first targeting polynucleotide arm - first unique molecular tag - polynucleotide linker - second unique molecular tag - second targeting polynucleotide arm;
b) for each respective MIP in the plurality of candidate MIPs,

i) computing a first number (A) of unique CpG sites predicted, with no mismatch on the binding arm sequence, to be captured by the respective MIP;
ii) computing a second number (C) of unique CpG sites predicted, with one mismatch on the binding arm sequence to be captured by the respective MIP;

iii) computing a third number (E) of unique sites predicted, with no mismatch on the binding arm sequence, to be captured by the respective MIP across a genome;

iv) computing a fourth number (G) of unique sites predicted, with one mismatch on the binding arm sequence, to be captured by the respective MIP across the genome;

v) computing a fifth number (F) of non-unique sites predicted, with no mismatch on the binding arm sequence, to be captured by the respective MIP across the genome;

vi) computing a sixth number (H) of non-unique sites predicted, with one mismatch on the binding arm sequence, to be captured by the respective MIP across the genome;

vii) computing a seventh number (I) of CpG sites present on the first targeting polynucleotide arm;

viii) computing an eighth number (J) of CpG sites present on the second targeting polynucleotide arm;

ix) computing a performance metric for the respective MIP based at least in part on the first, second, third, fourth, fifth, sixth, seventh, and eighth numbers;

c) selecting a MIP, based at least in part on the performance metric computed in step b)ix) for each MIP in the plurality of candidate MIPs.

For example, in the method above, the MIP at step c) may be selected such that a sum of the seventh number (I) and the eighth number (J) is smaller than the corresponding sum for a remaining set of the candidate MIPs. In certain embodiments, a first sum is a sum of the first number (A) and the second number (C), a second sum is a sum of the third number (E), the fourth number (G), the fifth number (F), and the sixth number (H); and the MIP at step c) is selected such that a ratio between the first sum and the second sum is larger than the ratio for a remaining set of the candidate MIPs. In certain embodiments, a third sum is a sum of the third number (E) and the fourth number (G); a fourth sum is a sum of the third number (E), the fourth number (G), the fifth number (F), and the sixth number (H); and the MIP at step c) is selected such that a ratio between the third sum and the fourth sum is larger than the ratio for a remaining set of the candidate MIPs. In certain embodiments, the MIP at step c) is selected based on a ratio ($K_e$) of an average capture coefficient of one mismatch sites ($K_1$) on the binding arm sequence and an average capture coefficient of zero mismatch sites ($K_0$):

$$K_e = \frac{K_1}{K_0}$$

and wherein the ratio ($K_e$) is experimentally estimated. In certain embodiments, the performance metric at step b) includes a factor corresponding to a weighted sum of the first number (A) and the second number (C). In certain embodiments, the weighted sum corresponds to A + Ke × C. In certain embodiments, the performance metric at step b) includes a factor corresponding to a weighted sum of the third number (E) and the fourth number (G). In certain embodiments, the weighted sum corresponds to E + Ke × G. In certain embodiments, the MIP at step c) is selected such that a product between a first weighted sum of A + Ke × C and a second weighted sum of E + Ke × G is larger than the product for a remaining set of the candidate MIPs.

In some embodiments, a nucleic acid molecule comprising a nucleotide sequence of CACTACACTCCAACCTAA(N1) CTTCAGCTTCCCGATTACGGGCACGATCCGACGGTAGTGT(N2) CAAAAAACTAAAACAAAA (SEQ ID NO:8), wherein (N1) represents a first unique molecular tag and (N2) represents a second unique molecular tag, is provided. In some embodiments, a nucleic acid molecule comprising a nucleotide sequence of TTCTCCTACCTCAACCTC(N1) CTTCAGCTTCCCGATTACGGGCACGATCCGACGGTAGTGT(N2) CCAAACTAAAATACAATA (SEQ ID NO:9), wherein (N1) represents the first unique molecular tag and (N2) represents the second unique molecular tag, is provided. Additional MIP molecules of the disclosure include the following:

mROP208-F, TCCTACCTCAACCTCCTA(6N)BB(6N)CCAAACTAAAATACAATA (SEQ ID NO:11),

/5Phos/TCCTACCTCAACCTCCTANNNNNNCTTCAGCTTCCCGATTACGGGCA CGATCCGACGGTAGTGTNNNNNNCCAAACTAAAATACAATA (SEQ ID

NO:12)

mROP208-R,
CACTACACTCCAACCTAA(6N)BB(6N)CAAAAAACTAAAACAAAA (SEQ ID NO: 13),

/5Phos/CACTACACTCCAACCTAANNNNNNCTTCAGCTTCCCGATTACGGGC

ACGATCCGACGGTAGTGTNNNNNNCAAAAAACTAAAACAAAA (SEQ ID

NO:14)

mROP206-F, TTCTCCTACCTCAACCTC(6N)BB(6N)CCAAACTAAAATACAATA (SEQ ID NO:15,

/5Phos/TTCTCCTACCTCAACCTCNNNNNNCTTCAGCTTCCCGATTACGGGCA

CGATCCGACGGTAGTGTNNNNNNCCAAACTAAAATACAATA (SEQ ID

NO:16)

mROP206-R, CACTACACTCCAACCTAA(6N)BB(6N)AAAACTAAAACAAAAAAA (SEQ ID NO:17),

/5Phos/CACTACACTCCAACCTAANNNNNNCTTCAGCTTCCCGATTACGGGC

ACGATCCGACGGTAGTGTNNNNNNAAAACTAAAACAAAAAAA (SEQ ID

NO:18)

As used herein, "BB" refers to a backbone sequence. The skilled artisan will understand that any generic backbone sequence may be applicable here.

As described above, in particular embodiments, a) the length of the first unique molecular tag is between 4 and 15 bases; and/or b) the length of the second unique molecular tag is between 4 and 15 bases.

Methods for identifying MIPs

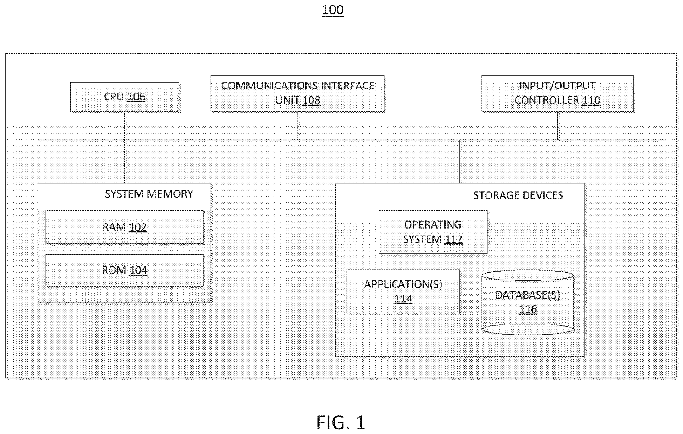

FIG. 1 is a block diagram of a computing device 100 for performing any of the processes described herein, including processes 200, 300, and 400. As used herein, the term "processor" or "computing device" refers to one or more computers, microprocessors, logic devices, servers, or other devices configured with hardware, firmware, and software to carry out one or more of the computerized techniques described herein. Processors and processing devices may also include one or more memory devices for storing inputs, outputs, and data which is currently being processed. The computing device 100 may include a "user interface," which may include, without limitation, any suitable combination of one or more input devices (*e.g.*, keypads, touch screens, trackballs, voice recognition systems, *etc.*) and/or one or more output devices (*e.g.*, visual displays, speakers, tactile displays, printing devices, *etc.*). The computing device 100 may include, without limitation, any suitable combination of one or more devices configured with hardware, firmware, and software to carry out one or more of the computerized techniques described herein. Each of the components described herein may be implemented on one or more computing devices 100. In certain aspects, a plurality of the components of these systems may be included within one computing device 100. In certain embodiments, a component and a storage device may be implemented across several computing devices 100.

The computing device 100 comprises at least one communications interface unit 108, an input/output controller 110, system memory, and one or more data storage devices. The system memory includes at least one random access memory (RAM 102) and at least one read-only memory (ROM 104). All of these elements are in communication with a central processing unit (CPU 106) to facilitate the operation of the computing device 100. The computing device 100 may be configured in many different ways. For example, the computing device 100 may be a conventional standalone computer or alternatively, the functions of computing device 100 may be distributed across multiple computer systems and architectures. In FIG. 1, the computing device 100 is linked, via network or local network, to other servers or systems.

The computing device 100 may be configured in a distributed architecture, wherein databases and processors are housed in separate units or locations. Some units perform primary processing functions and contain at a minimum a general controller or a processor and a system memory. In distributed architecture embodiments, each of these units may be attached via the communications interface unit 108 to a communications hub or port (not shown) that serves as a primary communication link with other servers, client or user computers and other related devices. The communications hub or port may have minimal processing capability itself, serving primarily as a communications router. A variety of communications protocols may be part of the system, including, but not limited to: Ethernet, SAP, SAS™, ATP, BLUETOOTH™, GSM and TCP/IP.

The CPU 106 comprises a processor, such as one or more conventional microprocessors and one or more supplementary co-processors such as math co-processors for offloading workload from the CPU 106. The CPU 106 is in communication with the communications interface unit 108 and the input/output controller 110, through which the CPU 106 communicates with other devices such as other servers, user terminals, or devices. The communications interface unit 108 and the input/output controller 110 may include multiple communication channels for simultaneous communication with, for example, other processors, servers or client terminals.

The CPU 106 is also in communication with the data storage device. The data storage device may comprise an appropriate combination of magnetic, optical or semiconductor memory, and may include, for example, RAM 102, ROM 104, flash drive, an optical disc such as a compact disc or a hard disk or drive. The CPU 106 and the data storage device each may be, for example, located entirely within a single computer or other computing device; or connected to each other by a communication medium, such as a USB port, serial port cable, a coaxial cable, an Ethernet cable, a telephone line, a radio frequency transceiver or other similar wireless or wired medium or combination of the foregoing. For example, the CPU 106 may be connected to the data storage device via the communications interface unit 108. The CPU 106 may be configured to perform one or more particular processing functions.

The data storage device may store, for example, (i) an operating system 112 for the computing device 100; (ii) one or more applications 114 (e.g., computer program code or a computer program product) adapted to direct the CPU 106 in accordance with the systems and methods described here, and particularly in accordance with the processes described in detail with regard to the CPU 106; or (iii) database(s) 116 adapted to store information that may be utilized to store information required by the program.

The operating system 112 and applications 114 may be stored, for example, in a compressed, an uncompiled and an encrypted format, and may include computer program code. The instructions of the program may be read into a main memory of the processor from a computer-readable medium other than the data storage device, such as from the ROM 104 or from the RAM 102. While execution of sequences of instructions in the program causes the CPU 106 to perform the process steps described herein, hard-wired circuitry may be used in place of, or in combination with, software instructions for embodiment of the processes of the present invention. Thus, the systems and methods described are not limited to any specific combination of hardware and software.

Suitable computer program code may be provided for performing one or more functions as described herein. The program also may include program elements such as an operating system 112, a database management system and "device drivers" that allow the processor to interface with computer peripheral devices (*e.g.*, a video display, a keyboard, a computer mouse, *etc.*) via the input/output controller 110.

The term "computer-readable medium" as used herein refers to any non-transitory medium that provides or participates in providing instructions to the processor of the computing device 100 (or any other processor of a device described herein) for execution. Such a medium may take many forms, including but not limited to, non-volatile media and volatile media. Non-volatile media include, for example, optical, magnetic, or opto-magnetic disks, or integrated circuit memory, such as flash memory. Volatile media include dynamic random access memory (DRAM), which typically constitutes the main memory. Common forms of computer-readable media include, for example, a floppy disk, a flexible disk, hard disk, magnetic tape, any other magnetic medium, a CD-ROM, DVD, any other optical medium, punch cards, paper tape, any other physical medium with patterns of holes, a RAM, a PROM, an EPROM or EEPROM (electronically erasable programmable read-only memory), a FLASH-EEPROM, any other memory chip or cartridge, or any other non-transitory medium from which a computer can read.

Various forms of computer readable media may be involved in carrying one or more sequences of one or more instructions to the CPU 106 (or any other processor of a device described herein) for execution. For example, the instructions may initially be borne on a magnetic disk of a remote computer (not shown). The remote computer can load the instructions into its dynamic memory and send the instructions over an Ethernet connection, cable line, or even telephone line using a modem. A communications device local to a computing device 100 (*e.g.*, a server) can receive the data on the respective communications line and place the data on a system bus for the processor. The system bus carries the data to main memory, from which the processor retrieves and executes the instructions. The instructions received by main memory may optionally be stored in memory either before or after execution by the processor. In addition, instructions may be received via a communication port as electrical, electromagnetic or optical signals, which are exemplary forms of wireless communications or data streams that carry various types of information.

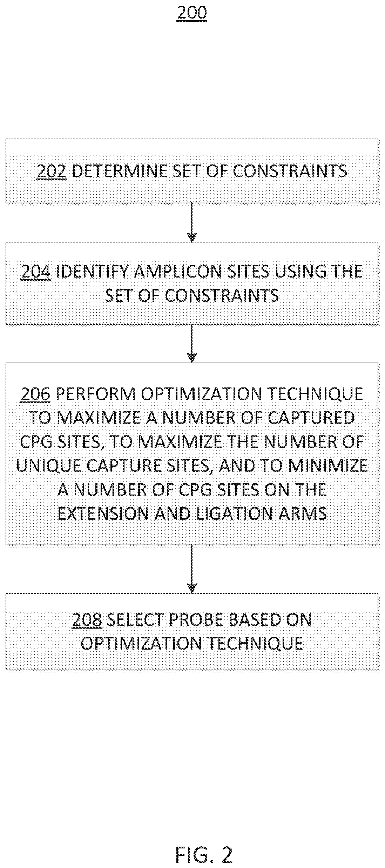

FIG. 2 is a flowchart of a process 200 for designing and selecting a probe (e.g., a MIP), according to an illustrative embodiment. The process 200 includes the steps of determining a set of constraints (step 202), identifying primers (e.g., target polynucleotide arms) using the set of constraints (step 204), performing an optimization technique to minimize a number of CpG sites on the extension and ligation arms of the MIP, maximize a total number of captured CpG sites, and maximize a number of uniquely mappable sites (step 206), and selecting a probe based on the optimization technique (step 208). As used herein, "primer" can refer to the hybridizing portion of a capture probe such as a molecular inversion probe. For example, "primer" can refer to the targeting polynucleotide arm or arms of a MIP.

At step 202, a set of constraints is determined. The set of constraints may be determined, for example, by CPU 106 using software or application(s) implemented thereon. In some embodiments, the software or application(s) may also be used by CPU 106 to perform any one or more of the subsequent steps in process 200. For example, the software and application(s) may be used by CPU 106 to find abundant primer pairs in a given reference genome (e.g., HG19) based on the determined constraints, and to automatically create suffix-array-based index for the genome file.

In some embodiments, the set of constraints may alternatively be referred to as algorithm flags. For example, the constraints (or algorithm flags) may include a length of the left primer and right primers (or ligation and extension arms, respectively), a minimum frequency of the primer-pair, a maximum distance between primers (e.g., amplicon length), a minimum and/or maximum total frequency of the primer, a minimum GC-content per primer in percent, a minimum amount of non-identical amplicons in percent, a distribution of primers in genome, or any suitable combination thereof. In an illustrative embodiment, the following set of constraints may be used in designing primer pairs:

- Length of the left primer or ligation arm: 18, 19, 20, 21 bases
- Length of the right primer or extension arm: 18, 19, 20 21 bases
- Frequency of primer-pair: 100, 250, 500, 2500, 5000, 10,000, 100,000, 500,000, 1,000,000
- Amplicon Length: 50-150 base pairs, e.g., less than 85 base pairs
- Minimum GC content per primer: 10%, 15%, 20%, 30%, 40%
- Amplicon uniqueness (percent of target sequences of interest that are unique): greater than about 40%
- Distribution of primers in genome: iteratively ran, with each bucket size (bs) ranging from 1 to 50%, and bucket-fill (bf) ranging from 1 to bs-1, wherein bucket size (bs) refers to bs% of genome long, and each bucket must contain bf% of all hits.

It is understood that the above set of constraints is merely illustrative in nature, and other sets of constraints may be used in probe selection processes.

At step 204, a set of primers are identified using the set of constraints determined at step 202. In particular, for each primer design, any combination of the following parameters may be provided: the left primer sequences (e.g., as well as the number of their occurrences on the positive and negative strands of the genome), the right primer sequences (e.g., as well as the number of their occurrences on the positive and negative strands of the genome), the frequency of the pair (e.g., the left primer sequence and the right primer sequence paired together with the amplicon length limited by a constraint) including both unique and non-unique pairs, the frequency and percentage of the uniquely occurring amplicons, and the amplicon sequences from unique and non-unique pairs. In some embodiments, each primer pair may be able to amplify multiple regions on the genome (e.g., more than hundreds, more than thousands, more than tens of thousands, more than hundreds of thousands, or more than millions).

In some embodiments, the predicted primer pairs are converted to target bisulfite converted genome. The generated primer pairs may identify or predict amplicon sites without allowing for any mismatches to occur in either the left primer sequence or in the right primer sequence (i.e., the left or right arms) on bisulfite converted genome. Alternatively, in order for additional amplicon sites to be identified or predicted, a small number of mismatches may be allowed, such as allowing for:

1 mismatch in the left arm and 0 mismatches in the right arm
0 mismatches in the left arm and 1 mismatch in the right arm
1 mismatch in the left arm and 1 mismatch in the right arm
2 mismatches in the left arm and 0 mismatches in the right arm, or
0 mismatches in the left arm and 2 mismatch in the right arm.

In some embodiments, the amplicon prediction scheme described above provides the genomic coordinates of the predicted amplicons in the bisulfite converted genome. However, in some embodiments, it may be computationally intensive for the scheme that identifies the amplicon sites without allowing for any mismatches to occur to also provide the genomic coordinates of the predicted amplicons. In this case, the scheme may be divided into two parts. In a first part, the amplicon sites are identified without allowing for any mismatches to occur, and the genomic coordinates of the identified amplicon sites are not provided. In a second part, the amplicon sites that include a small number of mismatches (e.g., the set of mismatches enumerated above) are identified, and the genomic coordinates of these amplicon sites are provided, as well as the genomic coordinate of the no-mismatch amplicon sites. Splitting up the scheme into these two modular parts may save computational complexity. However, in general, it will be understood that the two parts may be combined to provide the set of no-mismatch amplicon sites, mismatch amplicon sites, and their genomic coordinates in a single function.

In some embodiments, one or more of the amplicon sites identified at step 204 may be removed (e.g., by a filtering operation). For example, in some embodiments, arm sequences containing CG dinucleotides are removed. The amplicon sites of those primers that passed the filtering operation (hereinafter referred to as "candidate primers") should target multiple regions of the reference genome (e.g., typically 2500 or more). Additionally, in some embodiments, both the left and right arm sequences of the candidate primers have melting temperatures ($T_M$) ranging from 40° C to the high 60's C as computed by the nearest neighbor model of DNA binding stability, wherein empirical stability parameters are summed according to the nucleic acid sequence. See, e.g., Santa Lucia and Hicks 2004.

After the removal (or filtering) operation, the remaining amplicon sites will be further processed in order to generate a set of parameter values for each candidate primer. In some embodiments, the number of CpGs and the total number of amplicon sites that have passed the filtering operation will be calculated. For each candidate primer, the enrichment information (e.g., the calculated proportion), the associated amplicon sites information, and any other parameter values may be saved in a database, such as database 116.

At step 206, an optimization technique is performed to identify a primer with an optimal predicted performance. The optimization technique involves evaluating an objective function for each candidate primer. In particular, it may be desirable to use an objective function that minimizes a number of CpG sites on the extension and ligation arms of the MIP, maximizes a total number of captured CpG sites, maximizes a number of uniquely mappable sites, or any suitable combination thereof.

The objective function for each candidate MIP may, in some embodiments, be established based on the following matrices:

| # CpG counts | Unique | Non-unique |
|---|---|---|
| 0 mismatch | A | B |
| 1 mismatch | C | D |

**Table 1: Predicted CpG counts from predicted sites**

| Site Counts | Unique | Non-unique |
|---|---|---|
| 0 mismatch | E | F |
| 1 mismatch | G | H |

**Table 2: Predicted site count across the genome**

| # CpG counts | Extension arm | Ligation Arm |
|---|---|---|
| # CpG counts | I | J |

**Table 3: Number of CpG counts on each arm**

In the probe matrices above, rows labeled as "0 mismatch" indicates MIPs with perfect matches in both arms, and rows labeled as "1 mismatch" indicates primers that tolerates at most 1 mismatch in one of its arms in reference to bisulfite converted genome. Several intuitive objective functions can be readily deduced from these probe matrices. For example, an objective function that minimizes I+J (e.g., the sum of I and J is 0) would ensure that probe performance is not a function of an individual's methylation status (e.g., because there are CpG sites present in the arm sequence binding sites. In a second example, an objective function that maximizes (A+C)/(E+F+G+H) may produce reads that specifically target CpG sites. As a third example, an objective function that maximizes (E+G)/(E+F+G+H) selects primers that have significantly more unique capture sites than non-unique capture sites on bisulfite converted genome. To further illustrate this concept, three exemplary objective functions are explained in detail below.

**A. Total Number of CpG sites on the extension and ligation arms (P1)**

An exemplary objective function for each candidate primer or probe may be defined as the total number CpG sites on the extension arm and ligation arm of the probe:

$$P1 = I+J \qquad (1)$$

**B. Total Number of Useful CpG sites (P2)**

Another exemplary objective function for each candidate primer or probe may be defined as the total number of useful CpG sites that are captured:

$$P2 = g(A,B,C\ldots H; K_0, K_1) \qquad (2)$$

where $K_0$ is the average capture coefficient of 0 mismatch sites and $K_1$ is the average capture coefficient of 1 mismatch sites. More specifically:

$$P2 = A + K_e C \qquad (3)$$

where $K_e$ can be estimated from experimental data, and:

$$K_e = \frac{K_1}{K_0}, where\ 0 < K_e < 1 \qquad (4)$$

**C. Total number of uniquely mappable reads (P3)**

Another exemplary objective function for each candidate primer or probe may be defined as the total number of uniquely mappable reads across the bisulfite converted genome:

$$P3 = g(A,B,C\ldots H; K_0, K_1) \qquad (5)$$

where $K_0$ and $K_1$ are defined above. More specifically, P3 may be defined as:

$$P3 = E + K_e G \qquad (6)$$

where $K_e$ is defined in Equation (4).

## D. Comprehensive Probe Performance Function

A comprehensive way to evaluate an objective function for each candidate primer or probe is to first remove any candidate primer or probe where PI is not equal to zero. In other words, only candidate primers or probes where P1=0 may be considered, such that no CpG sites on the extension or ligation arms exist. In a second step, a performance function may correspond to:

$$P=P2\times P3 \tag{7}$$

Incorporating Equations (3) and (6), Equation (7) can be rewritten as:

$$P = ( (A+K_eC) \times (E+K_eG) ) / ( (E+F) + K_e(G+H) ) \tag{8}$$

Note that, as described above in relation to Equation (4), the value of $K_e$ can be estimated using experimental data. More particularly:

$$K_e = \frac{K_1}{K_0} = \frac{\frac{\text{molecular tag counts on 1 mismatch sites}}{\text{1 mismatch site count}}}{\frac{\text{molecular tag counts on 0 mismatch sites}}{\text{0 mismatch site count}}} \tag{10}$$

At step 208, a primer is selected from the set of candidate primers based on the optimization technique performed at step 206. For example, the selected primer may correspond to the primer with the optimal predicted performance, i.e., the primer that had P1=0 and that maximized the objective function as described in relation to step 206.

It is contemplated that the steps or descriptions of Process 200 may be used with any other embodiment of this disclosure. In addition, the steps and descriptions described in relation to FIG. 2 may be done in alternative orders or in parallel to further the purpose of this disclosure. For example, each of these steps may be performed in any order or in parallel or substantially simultaneously to reduce lag or increase the speed of the system or method. Furthermore, it should be noted that Process 200 may be carried out using computing device 100, and more particularly, CPU 106 of computing device 100.

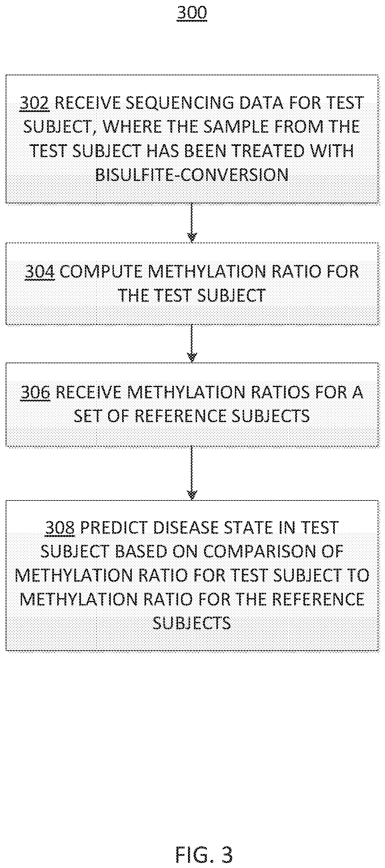

FIG. 3 is a flowchart of a process 300 for predicting a disease state in a test subject, according to an illustrative embodiment. The process 300 includes the steps of receiving sequencing data for a test subject (step 302), computing a methylation ratio for the test subject (step 304), receiving methylation ratios for a set of reference subjects (step 306), and predicting a disease state in the test subject based on comparison of the methylation ratio for the test subject to methylation ratios for the reference subjects (step 308).

The methylation score is calculated from the information contained in the sequencing reads encompassing CpG sites. Every time a CpG site is covered by a read, the information retrieved is considered as a count (methylated or unmethylated) in the formula below. A single read can generate multiple counts if it encompasses multiple CpG sites. Programs like the Bismark Methylation Extractor calculate methylation ratios as follows:

$$\% \text{ methylation at CpG sites} = 100 * [\text{Methylated Cs at CpG/( Methylated Cs at} \tag{11}$$

$$\text{CpG + Unmethylated Cs at CpG)}]$$

At step 302, sequencing data for a test subject is received. In particular, the test subject may have a disease state that is unknown, or a predisposition to a particular disease state. The received sequencing data is obtained by obtaining a nucleic acid sample from the test subject, treating the sample with bisulfite conversion, and using a population of primers, such as molecular inversion probes (MIPs), to capture a set of sites in the nucleic acid sample. As is described in detail in relation to FIG. 5, each MIP includes in sequence a first targeting polynucleotide arm, a first unique targeting molecular tag, a polynucleotide linker, a second unique targeting molecular tag, and a second targeting polynucleotide arm. The first and second targeting polynucleotide arms are the same across the MIPs in the population, while the first and second unique targeting molecular tags are distinct across the MIPs in the population. MIPs amplicons result from the capture of the sites, and the amplicons are sequenced to obtain the sequencing data.

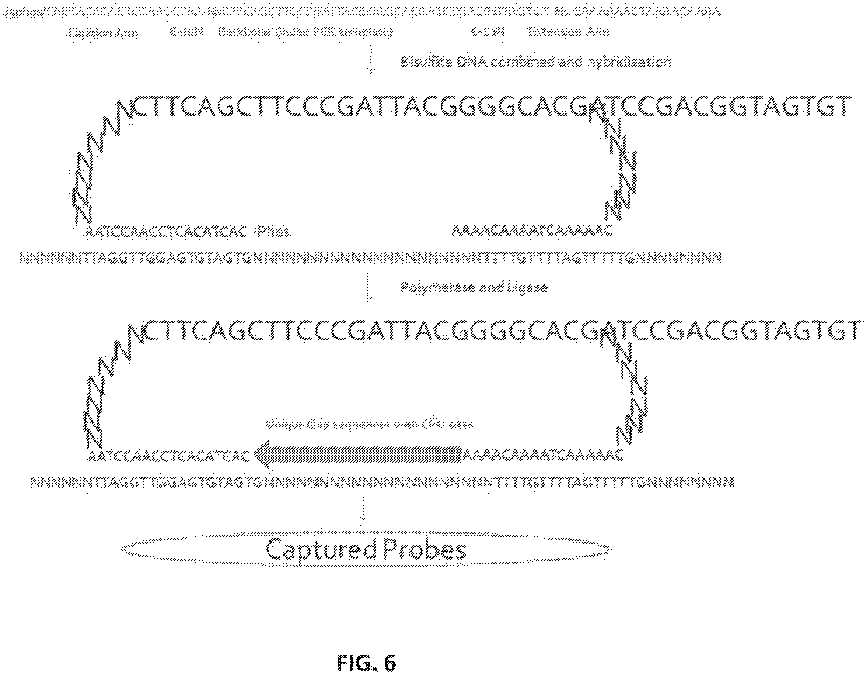

At step 304, a methylation ratio is computed for the test subject by evaluating a ratio between a number of methylated cytosine nucleotides within the target regions and a total number of known CpG sites. As is described in detail in relation to FIG. 6, the process of bisulfite-conversion converts un-methylated cytosine nucleotides to uracil nucleotides (which are subsequently converted to thymine nucleotides during PCR), and does not have an effect on methylated cytosine nucleotides. Accordingly, after a sample has been treated with bisulfite-conversion, the presence of remaining cytosine nucleotides at a CpG site indicates that those cytosine nucleotides are methylated. The methylation ratio provides a proportional measure of the methylated cytosine nucleotides, compared to a total number of CpG sites.

At step 306, a set of methylation ratios for a set of reference subjects is received. In particular, the reference subjects may correspond to a group of people that exhibit a known disease state or a known predisposition to have a disease. The methylation ratios for the reference subjects are computed in the same manner as was described in relation to step 304, but for each reference subject.

At step 308, the methylation ratio for the test subject (computed at step 304) is compared to the methylation ratios for the reference subjects (obtained at step 306), and the disease state or predisposition for a particular disease of the test subject is predicted based on this comparison. In particular, a statistical test may be used to compare the test methylation ratio to the population of reference methylation ratios, and determine whether the test methylation ratio belongs in any cluster of reference methylation ratios associated with the same disease state or predisposition.

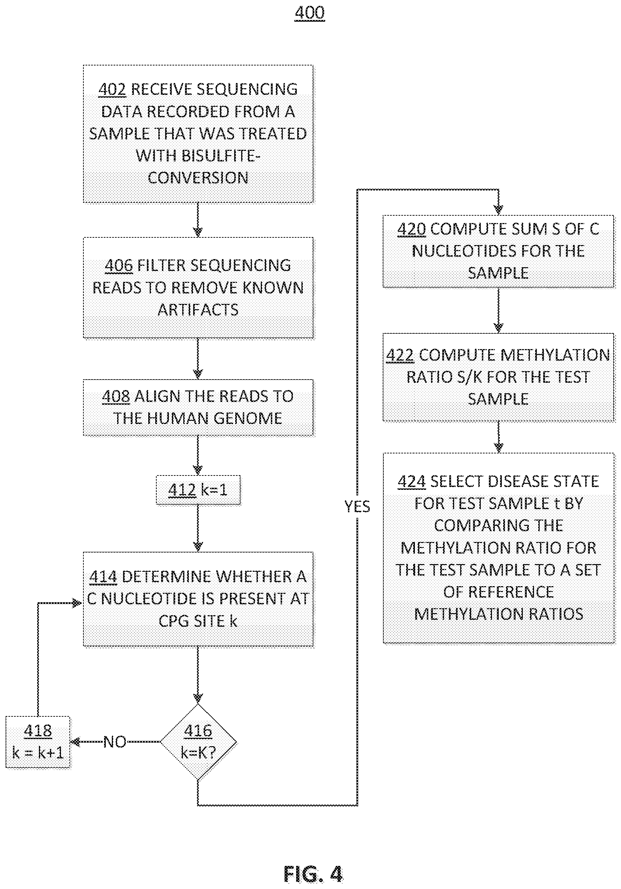

FIG. 4 is a flowchart of a process 400 for predicting a disease state of a test subject, according to an illustrative embodiment. In an example, the process 400 may be used to implement the steps 304 and 308 of the process 300 shown and described in relation to FIG. 3. As was described in relation to FIG. 3, a methylation ratio may be used to predict a disease state in a test subject that has an unknown disease state or a predisposition for a disease.

The process 400 includes the steps of receiving sequencing data recorded from a sample that was treated with bisulfite-conversion (step 402), filtering the sequencing reads to remove known artifacts (step 406), aligning the reads to the bisulfite converted human genome (step 408), setting a CpG site iteration parameter k to 1 (step 412), and determining whether a cytosine nucleotide is present a the k-th CpG site (step 414). When all K CpG sites have been considered, the process 400 further includes the steps of computing a sum S of the numbers of cytosine nucleotides determined at step 414 (step 420), computing a methylation ratio S/K for the test sample (step 422, where K corresponds to a total number of CpG sites), and selecting a disease state for the test sample by comparing the methylation ratio for the test sample to a set of reference methylation ratios (step 424).

At step 402, data recorded from a test sample is received. The test subject has an unknown disease state. The sample may be a nucleic acid sample isolated from the test subject and treated with bisulfite-conversion. The data may include sequencing data obtained from the nucleic acid samples. In an example, the sequencing data is obtained by using a population of MIPs to amplify a set of sites in the nucleic acid sample to produce a set of MIPs amplicons. The MIPs amplicons may then be sequenced to obtain the sequencing data received at step 402.

At step 406, the sequencing reads for the test sample are filtered to remove known artifacts. In one example, the data received at step 402 may be processed to remove an effect of probe-to-probe interaction. In some embodiments, the ligation and extension targeting arms of all MIPs are matched to the paired-end sequence reads. Reads that failed to match both arms of the MIPs are determined to be invalid and discarded. In some implementations, at most one base pair mismatch in each arm is allowed, but any reads that have more mismatches may be discarded. The arm sequences for the remaining valid reads are removed, and the molecular tags from both ligation and extension ends may be also removed from the reads.

At step 408, the resulting trimmed reads are aligned to the human genome. In some embodiments, an alignment tool may be used to align the reads to a reference human genome. In particular, an alignment score may be assessed for representing how well does a specific read align to the reference. Reads with alignment scores above a threshold may be referred to herein as primary alignments, and are retained. In contrast, reads with alignment scores below the threshold may be referred to herein as secondary alignments, and are discarded. Any reads that aligned to multiple locations along the reference genome may be referred to herein as multi-alignments, and are discarded.

At step 412, a CpG site iteration parameter k is initialized to one. The numbers and positions of CpG sites are known.

At step 414, the k-th CpG site is examined to determine whether a cytosine nucleotide is present. As is described in detail in relation to FIG. 6, the process of bisulfite-conversion converts un-methylated cytosine nucleotides to uracil nucleotides (which are later converted to thymine nucleotides during PCR), but does not have an effect on methylated cytosine nucleotides. Accordingly, after a sample has been treated with bisulfite-conversion, the presence of remaining cytosine nucleotides at a CpG site indicates that those cytosine nucleotides are methylated. After the k-th CpG site is examined at step 414, the CpG site iteration parameter k is incremented at step 418 until all K CpG sites have been considered. When all K CpG sites have been considered, the process 400 proceeds to step 420 to compute a sum S of the cytosine nucleotides for the test sample.

At step 422, a methylation ratio S/K is computed for the test sample. The methylation ratio corresponds to the total number of cytosine nucleotides present at the K CpG sites, normalized by K, and provides a proportional measure of the methylated cytosine nucleotides, compared to a total number of CpG sites.

At step 424, the methylation ratio for the test sample is then compared to a set of reference methylation ratios (that have been computed from reference subjects that have known disease states), and a statistical test is performed to select a predicted disease state for the test subject.

The order of the steps in FIG. 4 is shown for illustrative purposes only, and are not limiting.

Since methylation changes are not randomly distributed among the genome, the methylation ratio can be calculated, in some embodiments, by filtering out or isolating targets close to key elements of the genome. For example, to increase the sensitivity of detection of hypomethylation in cancer samples, the targets in proximity to CpG islands can be filtered out since they tend to become hypermethylated. In a second instance, the methylation ratio may be calculated with targets contained in the intergenic regions since they are known to show higher levels of hypomethylation.

In some embodiments, the level of hypomethylation of a test sample can be determined by comparing its methylation density to a set of control samples (5, 10, 50, 100, 500, 1000, 10,000 or more control samples). The methylation density is defined as the average percentage of methylated C in a CpG context for a defined region or for a defined bin size (1,000, 10,000, 100,000, 1,000,000, 10,000,000 or more bases). For each bin, a Z-score is calculated as follow and the percentage of $Z_{meth}$ over a defined threshold is determined.

$$Z_{meth} = \frac{MD_{test} - MD_{controls}}{MD_{SD\text{-}controls}} \qquad (12)$$

where:

**MD_{test}** is the methylation density for a defined bin for a test sample;
**MD_{controls}** is the average of the methylation density for a defined bin for a set of control samples; and
**MD_{SD-controls}** is the standard deviation of the methylation density for a set of control samples.

Also, CNVs, including CNAs, can be calculated the same way by replacing methylation density with read density.

A comparative analysis can also be performed to detect differential methylated CpG sites in a test sample group versus a control group. Methylkit is a R package for DNA methylation analysis (Altuna Akalin, Matthias Kormaksson, Sheng Li, Francine E. Garrett-Bakelman, Maria E. Figueroa, Ari Melnick, Christophe E. Mason. (2012). "methylKit: A comprehensive R package for the analysis of genome-wide DNA methylation profiles." Genome Biology, 13:R87.) Methylkit can be used to perform sample correlation and clustering, as well as, differential methylation analysis. CpG sites with differential methylation between the test group and the control group can be identified. Some CpG sites may show differential methylation status only in a subset of the test samples. Therefore, identifying a combination of CpG sites with a defined "weight" may be more appropriate to generate an algorithm allowing to evaluate if an unknown samples belong to the tested groups.

It will be understood by one of ordinary skill in the art that the compositions and methods described herein may be adapted and modified as is appropriate for the application being addressed and that the compositions and methods described herein may be employed in other suitable applications, and that such other additions and modifications will not depart from the scope hereof.

The embodiments referred to above will be better understood from the Experimental Details which follow. However, one skilled in the art will readily appreciate that the specific methods and results discussed are merely illustrative them.

## Examples

Example 1: MIP design and method for capturing target sequences of interest

*Probe design*

A single targeted capture probe is created for a semi-redundant site in the genome pertaining to any repeat regions. Additional criteria are designed to target short (~160bp) circulating nucleic acids and have the number of said repeat sites per bisulfite converted human genome of >150,000 sites with either exact primer match or 1 mismatch. The probe arm melting temperature is between 42°C and 50°C.

*Probe construction*

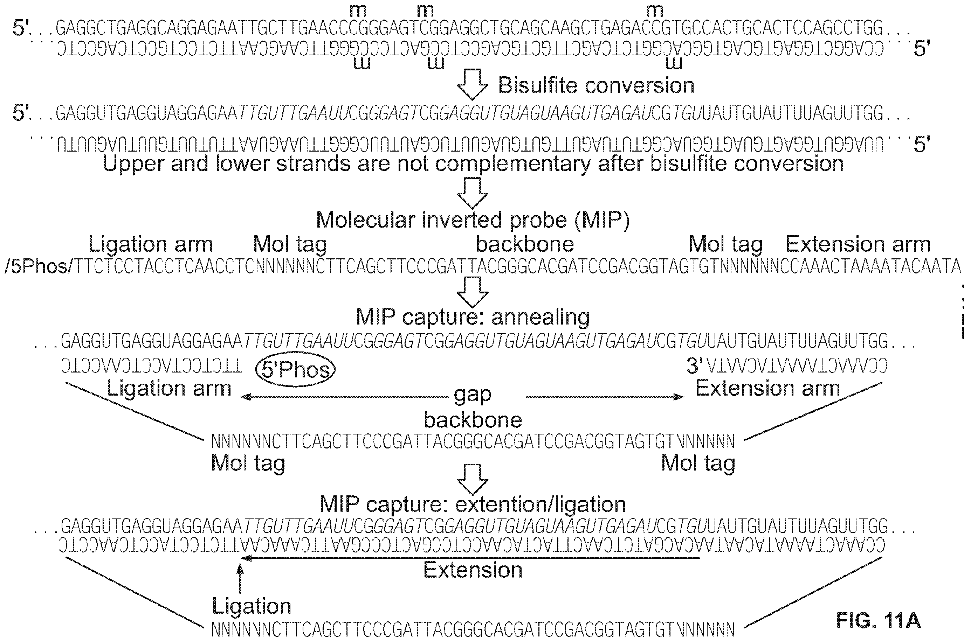

A single oligonucleotide ranging in size between 80-105bp (depending on the length of the repeat-targeting sequences) is synthesized such as that seen in FIG. 5 and FIG. 11A

*DNA Preparation*

DNA can be extracted from a variety of sources depending on the downstream use, including genomic DNA from whole blood, fragmented plasma DNA or DNA extracted from formalin-fixed paraffin embedded (FFPE) tissues.

After extraction, the DNA is bisulfite converted via a standard workflow such as: Input DNA → Denature DNA → Incubate→ Bisulfite Conversion → Incubate → Column-based sulphonation- hydrolytic deamination - desulphonatin →Elute to desired concentrations. Because the probe may be designed such that it is only amplifiable to the bisulfite converted genome, it allows for the capture of representative sites across the genome that have been bisulfite converted.

*Site capture reaction*

The capture probe (at an empirically determined concentration) is mixed with 10- 20 ng bisulfite treated DNA extracted from a variety of sources. The mixture is incubated in a thermocycler at temperatures that have been optimized for annealing of probes to the template (98 °C for 5 min → touchdown from 98 °C to 50 °C for 24 min → 50 °C for 120 min). During this incubation, the probe molecules anneal to the DNA template at specific chromosomal locations that are complementary to the probe sequence. The most easily predicted sites are those with sequences that are exactly complementary to the probe arms (invariant sites), but sites that have one or more variants in either arm are also targeted at somewhat lower efficiency. The optimal amount of probe for each reaction is dependent on four main considerations: 1) the number of genomes used as template, which can vary between samples, 2) the overall number of sites targeted by the specific probe, 3) the ratio of invariant sites to variant sites, and 4) the diversity of genomic CpG sites desired.

After the hybridization program is complete, a 5 μL mixture of enzymes and reagents is added and the mixture is incubated at 50 °C for 1 hr, then 72 °C for 20 min, then held at 4 °C. During this step, DNA polymerase (preferably a polymerase with no strand displacement properties) fills in the gap and the probe is covalently circularized by a DNA ligase using the phosphate at the 5' of the ligation arm (FIG. 6). Only probes that were annealed to the template and extended are circularized during this step, and linear probes are not amplified because of the gap between the primer binding sites. These linear probes are subsequently digested in a later step.

*Exonuclease digestion*

A cocktail of exonucleases is added. This removes remaining probes and genomic DNA by means of the exonuclease digestion. The exonucleases are then heat inactivated.

*Captured site indexing and amplification*

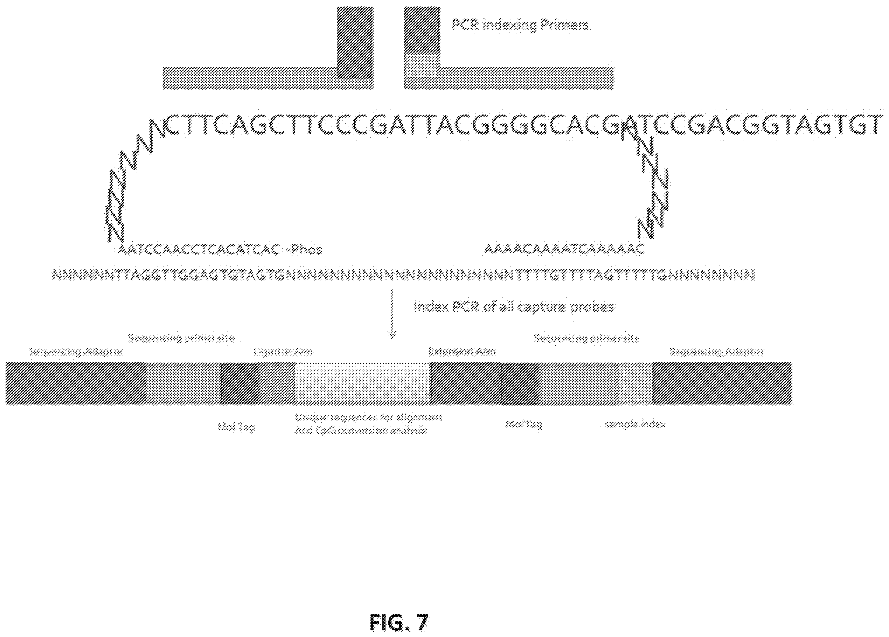

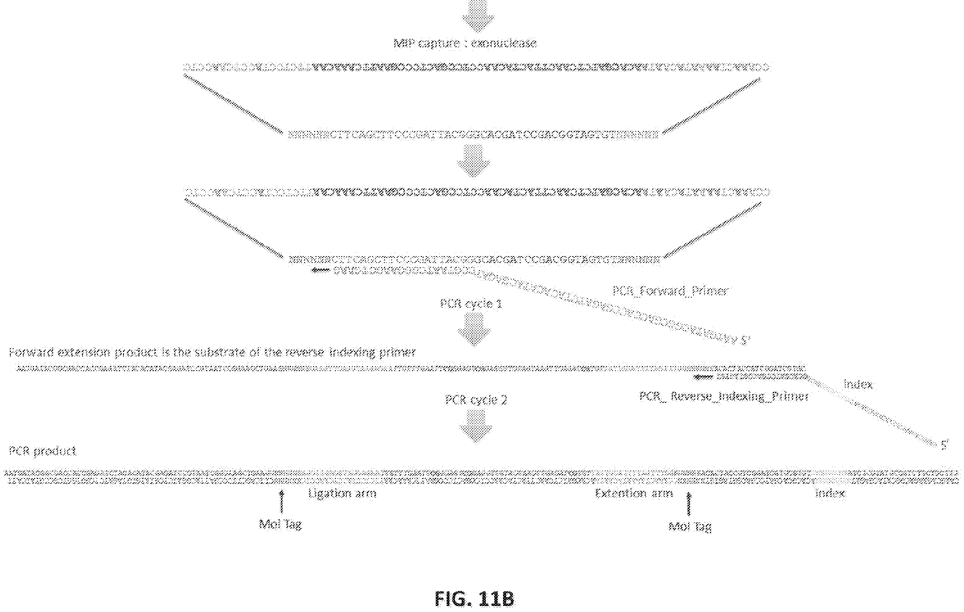

10 μL of the cleaned and captured probe mixture (i.e., replicons) is added to a 50 μL reaction mixture containing thermostable polymerase, dNTPS, PCR buffer, and universal primers that are complementary to the probe backbone (FIG. 7 and FIG. 11B). Each sample is amplified using a primer with a distinct sequencing barcode sequence allowing multiplex sequencing of a pooled sample library. The reaction is subjected to an empirically determined number of PCR cycles, which can be confirmed by the presence of a clean amplicon band observable by electrophoresis. The PCR product is purified using Ampure beads and quantitated using a Qubit fluorometer.

*Sequencing the captured sites*

Next, the purified PCR products are pooled into a library. The library is sequenced using either single-end or paired-end sequencing, using 75-100 cycles in order to determine the full sequence of the site-specific gap. If single-end sequencing is used, the read will consist of the ligation arm followed by the molecular tag and the unique gap sequence that was filled in during the extension/ligation step. Sequencing into the extension arm is unnecessary because the sequence is known from the probe. For example, massively parallel sequencing is used to identify both the methylated pattern in this area (hypo or hyper), the type and amount of DNA damage (e.g., mutational landscape) incurred, and the count of the sites to assay for large chromosomal abnormalities or genomic instability. Reads from the sequencer are aligned to an in silico-converted genome to determine positions where C nucleotides are observed instead of the expected T (the bisulfite-conversion produces a U nucleotide, which is read out as a T nucleotide by the sequencing methods). The methylation ratio is calculated as the number of C's observed in CpG sites, divided by the total number CpG dinucleotides in the target sequences of interest. This identifies the ratio of unconverted (i.e., methylated) cytosine nucleotides in the target region. The average methylation ratio of the sample is then reported.

*Removing PCR Duplicates*

Described below is an example of the importance of identifying and removing PCR duplicate previous to site specific methylation analysis. The use of unique molecular identifiers allows for each capture event to be characterized. More specifically, these identifiers are used to bin reads resulting from the same capture event, remove duplicates and report a single consensus read.

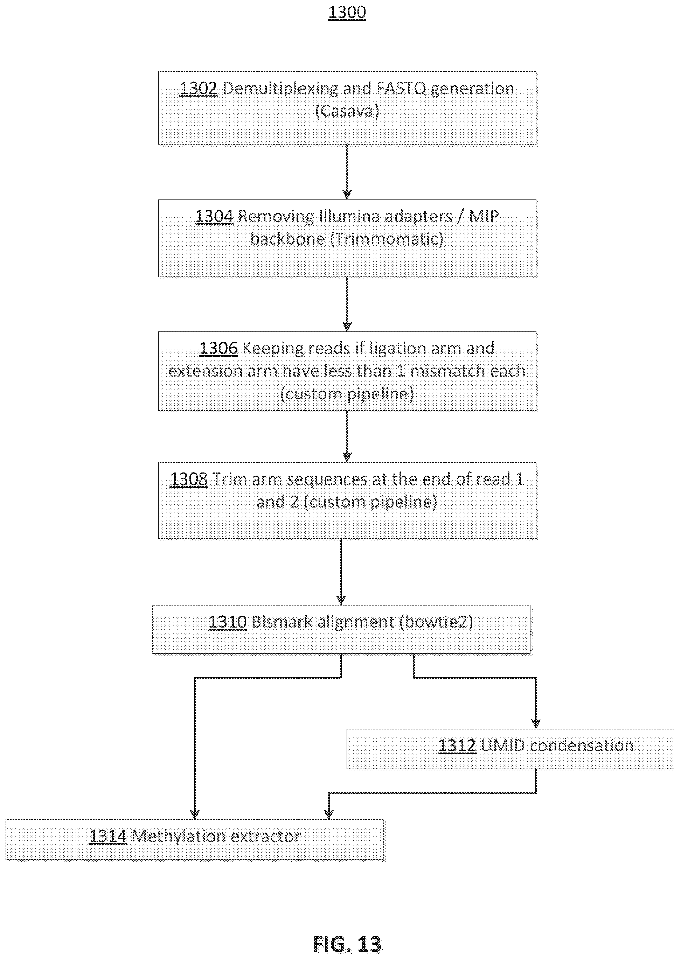

A mixture of human genomic DNA (gDNA) extracted from the blood of twelve individuals was obtained from Promega (cat #G3041). One, five, ten or twenty nano grams of blood gDNA and two hundred nano grams of salmon sperm DNA carrier were bisulfite converted using the EZ-96 DNA Methylation-Gold following the vendor's manual. The MIP capture was performed as described herein. The subsequent PCR was also performed as described in the present Example with the exception that 40ul of the exonuclease reaction was added instead of 10ul, and the PCR was assembled in a final volume of 100ul instead of 50ul. The libraries were pooled and sequenced on a HiSEQ 2500 using paired-end reagent V2 chemistry. The sequencing data was processed according to the pipeline described in FIG. 13 and throughout the specification. The pipeline included the step of condensing the unique molecular identifiers to remove PCR duplicates after Bismark alignment (1312). The bam files were imported into the Bismark Methylation Extractor, and the Bismark coverage files were uploaded into SeqMonk for further analysis (1314).

To determine the robustness of the assay at low input gDNA, the differences in the methylation at CpG sites from the different input gDNA libraries were determined by comparing them to the 20ng library control. First, the CpG sites with under 20x coverage were filtered out and the number of sites covered over 20x were determined (see Tables 4 and 5 below).

**Table 4**: Differential methylation observed between samples without PCR duplicate removal

| Input gDNA | # CpG covered >20x reads | #CpG shared at >20x reads with the 20ng control | Δ100 % | Δ80 % | Δ60 % | Δ50 % | Δ40 % | Δ30% |
|---|---|---|---|---|---|---|---|---|
| 1ng | 119,691 | 118,496 | 0 | 1 | 16 | 90 | 437 | 2062 |
| 5ng | 251,595 | 243,108 | 0 | 0 | 1 | 10 | 109 | 766 |
| 10ng | 289,955 | 271,608 | 0 | 0 | 0 | 3 | 66 | 438 |
| 20ng | 243,189 | 243,189 | 0 | 0 | 0 | 3 | 37 | 398 |
| 20ng | 295,235 | control | | | | | | |

**Table 5**: Differential methylation observed between samples without PCR duplicate removal

| Input gDNA | # CpG covered >20x reads | #CpG shared at >20x reads with the 20ng control | Δ100 % | Δ80 % | Δ60 % | Δ50 % | Δ40 % | Δ30 % |
|---|---|---|---|---|---|---|---|---|
| 1ng | 14,865 | 14,863 | 0 | 0 | 0 | 4 | 13 | 85 |
| 5ng | 193,526 | 191,513 | 0 | 0 | 0 | 0 | 18 | 230 |
| 10ng | 261,554 | 249,496 | 0 | 0 | 0 | 1 | 33 | 216 |
| 20ng | 243,078 | 235,473 | 0 | 0 | 0 | 2 | 24 | 284 |
| 20ng | 282,059 | control | | | | | | |

Table 4 shows the data without PCR duplicate removal and Table 5 shows the data after duplicate removal. When only 4.5% of the CpG sites were lost by eliminating PCR duplicates for the 20ng input samples (295,235 sites top table → 282,059 sites bottom table), 87.5% of the sites were filtered out in the 1ng samples (119,691 sites → 14,865 sites) showing the increase of PCR duplicates at low input.

Next, the CpG sites with over 20x coverage were identified in both the 20ng control samples and each corresponding input library. The number of sites shared are reported in column 3 of Tables 4 and 5. The CpG sites which lacked at least at 20x coverage in both the 20ng library control and the corresponding libraries (20, 10, 5 or 1ng) were filtered out. Finally, the number of CpG sites showing a differential in methylation over 100%, 80%, 60%, 50%, 40%, 30% between the control 20ng library and the 20, 10, 5 or 1ng were reported in columns 4-9. At 20ng input, few sites show differential methylation over a differential of 30% when compared to 20ng library control in both data sets with or without removing PCR duplicates. At 10ng, the PCR duplicate removal slightly improved the reproducibility of the assay by about 2 times. At 5ng and 1ng inputs, removing PCR duplicates becomes a key element of the analysis. The number of CpG sites with discordant methylation status with the 20ng control library was brought down by removing duplicates (top vs bottom table). However, eliminating PCR duplicates also greatly reduced the number of CpG sites with over 20x coverage in the low input DNA libraries.

In conclusion, detecting differential methylation in low input DNA can be improved by removing PCR duplicates prior to analysis, which can reduce the number of false positives.

*Mutational Landscape*

As described above, the PCR products generated using the compositions and methods described herein can be sequenced, and the sequence information can be informative of methylation status, CNV status and also provide a mutational landscape of the genome. All three of these measures can be hallmarks of different diseases and conditions, including cancer.

Cancer is a disease of deregulated cell growth caused by damage or alteration to a cell's DNA. As a cell evolves away from a state of regulated homeostasis, it acquires DNA alterations that disrupt key control pathways such as cell cycle regulation, cell death, and energy metabolism.

A more recently appreciated hallmark of cancer is the deregulation of genome stability and DNA repair processes. Deregulation of genome stability can occur via multiple pathways with different cancers having distinctive patterns of instability termed "mutational landscape". For example, a colorectal tumor in one individual may have a different mutational landscape than a colorectal tumor from someone else. This landscape includes the summation of all single nucleotide substitutions or variations, small insertions and deletions and larger aneuploidies and chromosomal rearrangements. This pattern of mutational landscape has been proposed to differentiate treatment effectiveness in response to immunotherapies. See Rizvi et al. "Mutational landscape determines sensitivity to PD-1 blockade in non-small cell lung cancer" Science; 03 Apr 2015: Vol. 348, Issue 6230, pp. 124-128.

The ability to detect and categorize the mutational landscape of tumors has clinical value, especially for improved prognosis. The compositions and methods described herein are particularly useful for determining mutational landscape. For example, after capturing and sequencing the DNA of interest, one can align the DNA to the genome using standard or custom methods, and proceed to apply a general variant caller. After application of the variant caller and additional methods to filter variants, the number of transitions, transversions, deletions and insertions can be binned into respective categories and enumerated per megabase of DNA analyzed. Since the location of DNA damage is spread across the genome, one does not need to focus on predetermined, targeted locations. Instead, a technology like that described herein that assays many repeat regions across the genome allows one to elucidate the mutational landscape in a single assay.

Example 2: Bioinformatics workflow

Raw sequencing data must be processed in order for it to be useful in detecting methylation status. To start, sequencing reads are filtered to remove known artifacts such as probe-to-probe interaction, backbone sequences or adapter sequences. The ligation and extension arms of the MIP (i.e., the first and second targeting polynucleotide arms) are then matched to the sequence reads, allowing a maximum of one base pair mismatch in each arm. Reads that fail to meet this criterion are treated as invalid and discarded. At the same time, the molecular tags from both the ligation and the extension ends are kept separately for counting of the capture events in a later step - although in some embodiments the tags are kept together. The trimmed reads are aligned to the bisulfite-converted human genome (hg19) with the Bismark Bisulfite Mapper by Babraham Bioinformatics. The uniquely aligned reads (in sam/bam format files) are examined to count the unique molecular tags for each targeted site with a unique probe gap sequence. These counts are the initial number of probe-to-target hybridization events that are sequenced in the Next Generation Sequencing (NGS) platform (e.g., an Illumina HiSeq 2500 flowcell). Alternatively or additionally, massively parallel sequencing or sequencing by synthesis may be used. The uniquely aligned reads (in bam format files) are finally run through the Bismark's Methylation Extractor to determine the methylation status of the sample. For this step, only the gap sequence is used (i.e., the ligation and extension arm sequences are not included in the calculation). The repeat methylation ratio (or score) is reported as the ratio of methylated C's in CpG context throughout the sample. For example, the number of methylated C's in CpG can be divided by the sum of the methylated C's in CpG plus unmethylated C's in CpG.

Targets or regions that display an aberrantly high level of either technical variation or population baseline variation are screened depending on the disease or condition to give a lower coefficient of variation than could be obtained by other random methods of capture and sequencing.

Example 3: Determination of methylation status in cancer cell lines

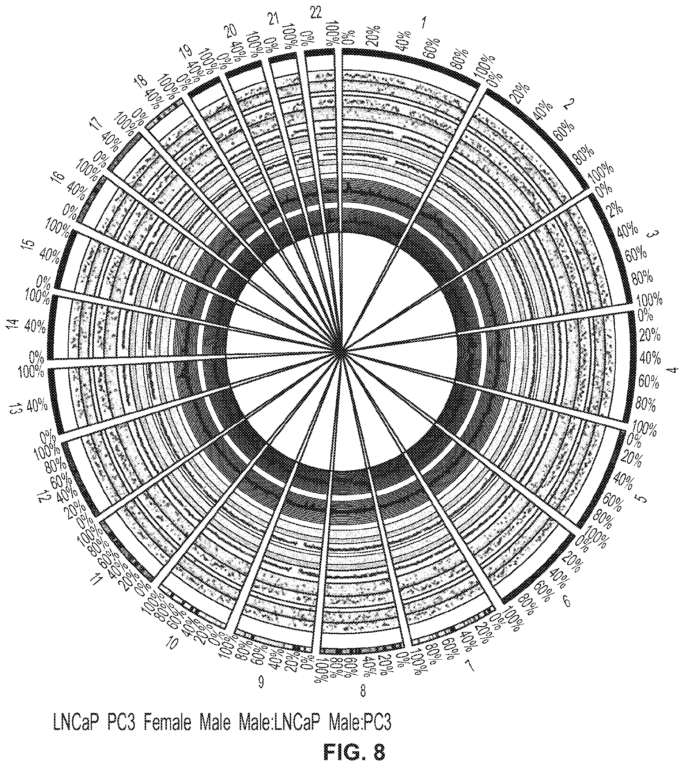

Using the methods described above, a total of 12 samples (the LNCaP and PC3 cancer cell lines, 5 healthy control female samples, and 5 healthy male samples) were processed. The methylation ratios were averaged into 1Mb bins for visualization to yield methylation densities. The methylation densities of the 5 female samples were averaged for each bin. Similarly, the 5 male samples' methylation densities were averaged for each bin. FIG. 8 depicts a graphical representation of the data obtained; the inner two tracks represent the ratio of unique capture events in each 1Mb bin between the averaged male samples and the respective cancer cell line, which can be used to analyze copy number variations in the samples. Also, there is a clear separation between the cancer cell line DNA and the non-cancer blood derived DNA samples in terms of their methylation status across the genome. Furthermore, the blood-derived DNA exists in a stable range of overall methylation as interrogated by the compositions and methods described herein.

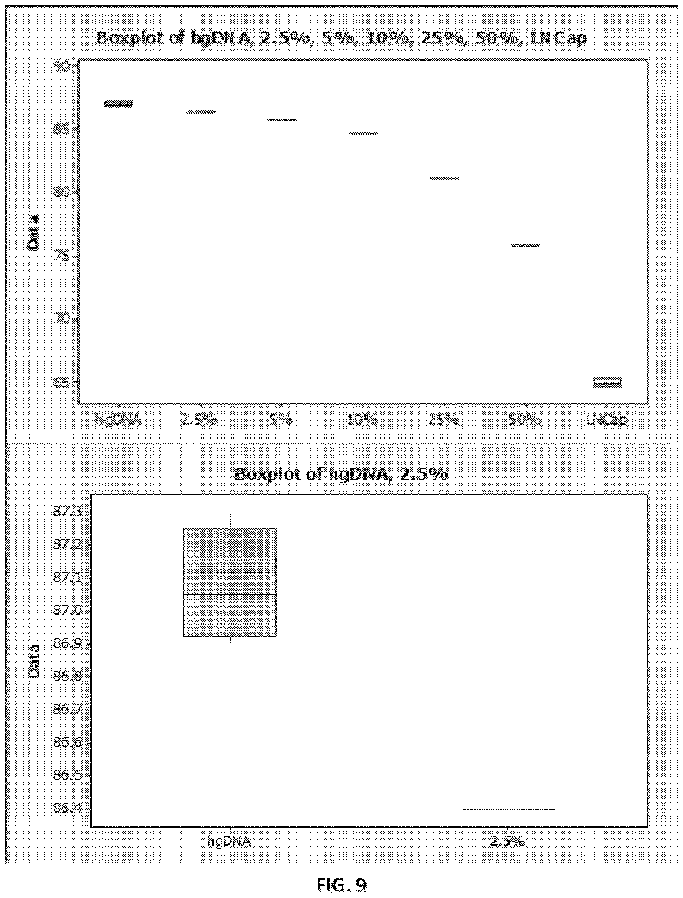

By then looking at the methylation index of the sample, differences were detected between the samples, as shown in the top plot of FIG. 9, where the Y axis represents the methylation score, and the X-axis represents percentage amounts of LNCap gDNA mixed into blood human genomic DNA at decreasing percentages as described below. The left most samples correspond to blood derived from healthy individuals. As the X axis increases towards the right, increasing percentages of cancer cell line DNA (LnCap) are added to DNA isolated from blood of healthy individuals from 2.5 to 50%. The bottom plot of FIG. 9 depicts a zoomed in version of the healthy samples and the 2.5% samples from the top plot of FIG. 9. The results shown in FIG. 9 indicate that shifts in methylation percentages can be distinguished at at least 2.5% because the repeat methylation scores for 2.5% are significantly below those for healthy individuals. Here we can detect mixtures of at least 2.5 or 3%. Thus, the MIPs described herein can be used to determine methylation across the genome or at desired intervals and presented as a score (index, ratio, percentage, density, etc.), which may be useful to clinicians comparing healthy tissue to diseased tissue as in the case of delineating cancerous versus non-cancerous specimens, aggressively growing cancer tissue versus non-aggressively growing tissue, or aiding in the diagnosis of diseases in which various types of non-normal tissue can be integrated in plasma DNA, including prediction of preterm labor, preeclampsia, and as a screening method for earlier cancer detection from a blood sample.

Based on these data, one can use a MIP to detect from a blood draw the presence of, for example, colorectal cancer. Small fragmented DNA from tumors exists in the plasma component of blood, and this DNA can be isolated. After isolation of this DNA from the blood, the DNA can be interrogated by the methods described herein. A population-derived baseline can be calculated using information obtained after interrogating a variety of non-cancerous individuals.

By further sequencing a variety of individuals with, for example, colorectal cancer, a second disease state score can be established. Upon subsequent sequencing, one will be able to determine the best fit of the individual sample to either the disease state or the non-diseased state. These methods are ideal for this purpose for cancer (e.g., colorectal cancer) as the score can be derived and simultaneously calculated for multiple phenomena in the cancer. In particular, methylation status is given as a repeat methylation score, which corresponds to the methylation ratio. In general, a chromosomal aneuploidy score or another score may be used. The hallmarks of cancer include genomic instability (e.g., copy number variation) and global hypomethylation, especially in repeat regions as opposed to non-repeat regions that may contain context-specific hyper methylation. Using the methods described herein is advantageous because they reduce the noise of targeting the whole genome, resulting in a lower sample-to-sample variation within the specific repeat targeted by the MIP while simultaneously using less reads and lowering costs.

## Example 4: Genomic Instability Analysis in Colorectal Samples

This example describes the use of compositions and methods of the invention to measure the methylation status of adenoma and adenocarcinoma isolated from the colon or the rectum. The same compositions and methods were also used to identify common CpG sites hypomethylated in the cancer samples. Finally, copy number alteration was detected from the same data set.

*Materials*

The following MIP capture probe and PCR primers were used:

Capture Probe:
:/5Phos/TTCTCCTACCTCAACCTCNNNNNNNCTTCAGCTTCCCGATTAC        GGGCACGATCCGACGGTAGT-GTNNNNNNCCAAACTAAAATACAAT A (SEQ ID NO: 16) (Integrated DNA technologies (IDT), 4 nmole Ultramer™ DNA oligo, standard desalting) 5' Phosphorylation (IDT)

PCR_Forward_Primer:
AATGATACGGCGACCACCGAGATCTACACATACGAGATCCGTAATC GGGAAGCTGAAG (SEQ ID NO: 19)
(IDT, 250nmoles, Standard desalting)
PCR_Reverse_Indexing_Primer:
CAAGCAGAAGACGGCATACGAGATNNNNNNNNACACGCACGATCC GACGGTAGTGT (SEQ ID NO:20) where
N can be A, T, C, or G, which is different combinations for each index. (IDT, 100nmoles, Standard desalting)

*Methods*

Human genomic DNA (hgDNA) was extracted from fresh frozen tissues from fourteen adenomas, and four adenocarcinomas as well as their corresponding normal adjacent tissues. Allprep DNA/RNA/Protein Mini Kit from Qiagen was used to extract hgDNA following the vendor's manual. The extracted hgDNA was quantified using a NanoDropTM spectrophotometer and 10ng of the samples and 200ng of salmon sperm carrier were used for bisulfite conversion using the EZ-96 DNA Methylation-Gold following the vendor's manual.

The bisulfite converted DNA was added to the first target capture reaction (described in Figures 11AB). A specially designed molecular inversion probe (MIP) was used to capture repetitive elements rich in CpG sites from bisulfite converted genome as described herein. First, the MIP was annealed to its targets on the bisulfite DNA. The reaction was assembled in a final volume of 20ul comprising a final concentration of 1x Ampligase buffer and 1 pmol of probe. The following cycling program was run: 98°C for 5 minutes, touchdown (decrease of 1°C every 30 seconds to reach 50°C), 50°C for 120 minutes.

Immediately after the capture, the annealed probe was extended at its 3' end by a high fidelity DNA polymerase (see FIG. 11A). The extension was stopped when the newly synthetized DNA met with the ligation arm of the MIP since the DNA polymerase lacks strand displacement activity. The new 3' end was ligated to the 5' end of the probe using the energy of the phosphate modification at this position creating a single-stranded circular molecule (or replicon). The extension/ligation reaction was assembled in a final volume of 40ul including the entire capture reaction and a final concentration of 1x Ampligase buffer, 150nM of dNTP, 1mM NAD+, 375mM of Betaine, 10 units of Ampligase and 1 unit of Phusion® High-Fidelity DNA Polymerase. The reaction was incubated at 50°C for 60 minutes, 72°C for 20 minutes.

Immediately after the extension/ligation step, the unligated probes and the gDNA were digested by exonucleases enzymes. This step is performed to avoid the formation of potential undesired products in the subsequent PCR amplification reaction. The exonuclease reaction was assembled in a final volume of 50ul comprising 40ul of the extension ligation reaction and final concentrations of 1x NEBuffer1 as well as 40 units of exonuclease I and 200 units of exonuclease III. This reaction was incubated at 37°C for 55 minutes. Exonuclease enzyme were then inactivated at 90°C for 40 minutes and held at 4°C or -20°C until processing to the PCR reaction.

The PCR reaction was assembled in a final volume of 50ul using 10ul of the exonuclease reaction and a final concentration of 1x Phusion® High-Fidelity buffer, 200nM dNTP, 2 units of Phusion® Hot start flex DNA polymerase, 500nM of PCR_Forward_Primer and 500nM of a unique PCR_Reverse_Indexing_Primer. The PCR was conducted as follow: 1 cycle of 95°C for 2 minutes follow by 21 cycles of 98°C 15 seconds, 65°C 15 seconds, 72°C 15 seconds and 1 cycle of 72°C 5 minutes and hold at 4°C. The PCR product was cleaned-up using AMPure XP beads at a ratio of 1.2x following the vendor's recommendations. The amplified libraries were quantified using Qubit® dsDNA HS Assay Kit. The libraries were pooled at an equimolar ratio at a final concentration of 4nM

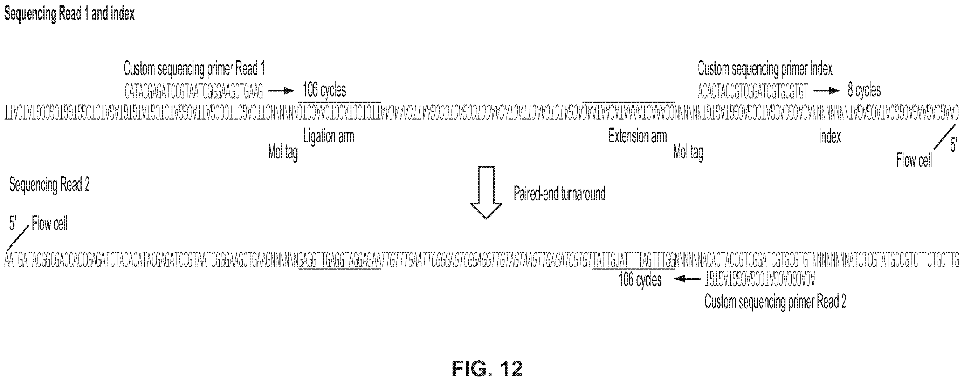

The libraries were sequenced on a HiSeq2500 using fast run mode and custom primers for read 1 and 2, as well as for indexing read at a final concentration of 570nM. A paired-end reads of 106 cycles were performed for read 1 and read 2 as well as an indexing read of 8 cycles. (See FIG. 12). Read 1 is defined as the read sequencing from the ligation arm toward the extension arm. The index is read after the read 1. After paired-end turnaround, read 2 sequences the reverse complement of the extension arm toward the reverse complement of the ligation arm (Figure 12).

The following steps were used for analysis as outlined in FIG. 13. Demultiplexing (retrieving sequencing data from each multiplexed indexed libraries) and FASTQ file generation were performed using Casava (Illumina) (1302). Illumina sequencing adapters as well as MIP backbone (see FIG. 11A for MIP structure) were trimmed using Trimmomatic (Bolger, A. M., Lohse, M., & Usadel, B. (2014) (1304). Trimmomatic: A flexible trimmer for Illumina Sequence Data. Bioinformatics). The reads with more than one mismatch on the ligation arm or the extension arm were filtered out (for probe structure see FIG. 11A) (1306). On read 1, the extension arm sequence was trimmed off using a custom pipeline. On read 2, the ligation arm sequence was trimmed off using a custom pipeline (1308). Alignment was performed using Bismark, a three letter aligner, (Felix Krueger, Babraham institute) with the bowtie2 option to generate SAM files, then BAM files using Samtools (1310) (Li H.*, Handsaker B.*, Wysoker A., Fennell T., Ruan J., Homer N., Marth G., Abecasis G., Durbin R. and 1000 Genome Project Data Processing Subgroup (2009) The Sequence alignment/map (SAM) format and SAMtools. Bioinformatics, 25, 2078-9). The bowtie2 output file contains uniquely aligned reads. A read (or read pair) align uniquely if the alignment has a unique best alignment score. In other words, the reads with multiple best alignment scores are discarded. Bismark minimum alignment score was defined by the option: --score-min L,0,-0.4, where L is the read length (~75bases after trimming). The minimum alignment score = 0 + -0.4 * 75 = -30 which correspond to 5 mismatches (-6 each) or combination of mismatch and indels up to a sum of -30 (Indel score: 1 bases: -8, 2 bases -11, 3 bases -14, 4 bases -17, 5 bases -20, 6 bases -23...) Bowtie 2: (Langmead B, Salzberg S. Fast gapped-read alignment with Bowtie 2. Nature Methods. 2012, 9:357-359).

Bismark Methylation Extractor was used with the following options: -p, -- no_overlap, --ignore 18, --ignore_r2 18, --comprehensive, -bedGraph,cytosine_report, where -p : paired-end analysis, --no_overlap: cytosine covered by read 1 and 2 are only reported once even if sequenced twice, --ignore 18: the sequence from the MIP in read 1 is excluded from the analysis, --ignore_r2 18: the sequence from the MIP in read 2 is excluded from the analysis, --comprehensive: display methylation status at CpG, CHG and CHH contexts merging all four possible strand-specific methylation info. Finally, the -bedGraph and --cytosine_report options were also used to generate coverage files (cov). The coverage output had the following features: <chromosome> <start position> <end position> <methylation percentage> <count methylated> <count unmethylated> and use a 1-based chromosome coordinates and report CpG context only. The BAM and the cov files were imported into Seqmonk (Simon Andrews, Babraham institute) for visualization of the methylation status and for further statistical analysis.

*Results*

The percentage methylation at CpG context was calculated and reported by Bismark Methylation Extractor as follows: % methylation at CpG = 100 * methylated Cs at CpG / (methylated Cs at CpG + unmethylated Cs at CpG). Table 1 shows the % methylation at CpG for the tumor and corresponding normal adjacent samples.

**Table 6**

| ID | Tissue type | Location | Age | Percent Methylated C in CpG context | |
| --- | --- | --- | --- | --- | --- |
| | | | | **Normal adjacent** | **Tumor** |
| 815 | Adenocarcinoma | Cecum | 74 | 85% | 73% |
| 861 | Adenocarcinoma | Ascending | 76 | 83% | 81% |
| G1-21 | Adenocarcinoma | Ascending | 57 | 86% | 82% |
| 781 | Adenocarcinoma | Descending | 52 | 84% | 81% |
| 227 | Adenoma | Cecum | 50 | 86% | 79% |
| 469 | Adenoma | Cecum | 86 | 84% | 82% |
| 726 | Adenoma | Ascending | 51 | 86% | 86% |
| 282 | Adenoma | Ascending | 66 | 84% | 79% |
| 735 | Adenoma | Ascending | 72 | 86% | 80% |
| 575 | Adenoma | Ascending | 73 | 85% | 74% |
| 300 | Adenoma | Transverse | 59 | 87% | 82% |
| 548 | Adenoma | Descending | 54 | 87% | 85% |
| 772 | Adenoma | Rectum | 79 | 86% | 82% |
| 236 | FAP | Ascending | 26 | 85% | 85% |
| 664 | FAP | Cecum | 52 | 86% | 82% |
| 610 | Lynch | Ascending | 59 | 85% | 82% |
| 245 | SSA | Ascending | 54 | 85% | 84% |
| 708 | SSA | Cecum | 76 | 85% | 84% |

The level of hypomethylation of the tumor samples was determined by comparing the methylation density of tumor samples and normal samples. The methylation density is defined as the average percentage of methylated C in a CpG context for a defined one megabase bin. To perform this analysis, the coverage files obtained from the Bismark Methylation Extractor were imported into SeqMonk. The methylation density was determined by averaging the methylation status at every megabase bins with a minimum of 25 different counts. Every time a CpG site was covered by a read, the information retrieved was considered as a count. A single read can generate multiple counts if it encompasses multiple CpG. On average, 2 CpG sites were covered per read. The bins on the Y chromosome were filtered out and a total of 2852 bins were analyzed. For each bin, Zmeth was calculated as follows:

Where ZDtumor is the methylation density in a bin of one megabase for a tumor sample;
MDnormal is the average of the methylation density from a bin of one megabase for all the normal samples (n=18); and
MDSD = the standard deviation of the methylation density from all the normal samples (n=18).

The $Z_{meth}$ was calculated for the valid 2852 bins. A bin was considered hypomethylated if the corresponding Zmeth was below -5. Table 2 depicts the percentage of the bins with significant hypomethylation.

**Table 7**

| ID | Tissue type | Location | Age | Percentage of bins with significant hypomethylation |
|---|---|---|---|---|
| 815 | Adenocarcinoma | Cecum | 74 | 60.8% |
| 861 | Adenocarcinoma | Ascending | 76 | 12.9% |
| G1-21 | Adenocarcinoma | Ascending | 57 | 8.0% |
| 781 | Adenocarcinoma | Descending | 52 | 14.0% |
| 227 | Adenoma | Cecum | 50 | 36.1% |
| 469 | Adenoma | Cecum | 86 | 8.7% |
| 726 | Adenoma | Ascending | 51 | 0.6% |
| 282 | Adenoma | Ascending | 66 | 35.9% |
| 735 | Adenoma | Ascending | 72 | 23.7% |
| 575 | Adenoma | Ascending | 73 | 62.1% |
| 300 | Adenoma | Transverse | 59 | 5.4% |
| 548 | Adenoma | Descending | 54 | 0.5% |
| 772 | Adenoma | Rectum | 79 | 10.3% |
| 236 | FAP | Ascending | 26 | 0.4% |
| 664 | FAP | Cecum | 52 | 9.7% |
| 610 | Lynch | Ascending | 59 | 11.5% |
| 245 | SSA | Ascending | 54 | 0.9% |
| 708 | SSA | Cecum | 76 | 0.8% |

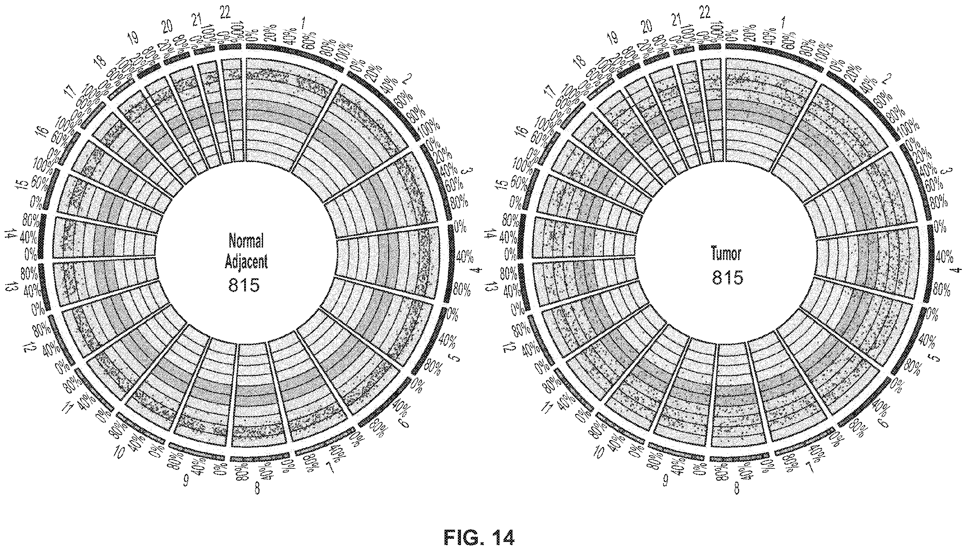

Circos plots can also be used to visualized the hypomethylated state of a sample. In FIG. 14, the methylation state was reported for all megabases. The circos plot on the left (normal) shows a high level of methylation across the genome with most of the bins (dots) in the red area. The plot on the right (tumor) displays a significant amount of bins (dots) with lower methylation in the grey and green zones. Thirteen tumor samples out of eighteen showed some level of hypomethylation. Again, using Seqmonk, the sites commonly hypomethylated in those tumor samples were identified. First, the CpG sites with a coverage of less than 30x were filtered out. A total of 89, 998 CpG were analyzed using a chisquare test p < 0.01. A total of 120 sites were identified. Interestingly, two CpG sites upstream of the gene TRPS1 were commonly hypomethylated in all thirteen samples with marked global hypomethylation, but not in the five without (samples 726, 548, 236, 245, 708) (Table 8).

**Table 8:** Common hypomethylated sites in thirteen samples with significant global hypomethylation

| ID | Tissue type | Location | Age | % C methylated at chr8: 116,160,339 | | % C methylated chr8: 116,369,046 | |
|---|---|---|---|---|---|---|---|
| | | | | Normal | Tumor | Normal | Tumor |
| 815 | Adenocarcinoma | Cecum | 74 | 83 | 17 | 78 | 28 |

(continued)

| ID | Tissue type | Location | Age | % C methylated at chr8: 116,160,339 | | % C methylated chr8: 116,369,046 | |
|---|---|---|---|---|---|---|---|
| | | | | Normal | Tumor | Normal | Tumor |
| 861 | Adenocarcinoma | Ascending | 76 | 74 | 44 | 90 | 47 |
| G1-21 | Adenocarcinoma | Ascending | 57 | 89 | 59 | 91 | 56 |
| 781 | Adenocarcinoma | Descending | 52 | 74 | 47 | 88 | 41 |
| 227 | Adenoma | Cecum | 50 | 84 | 43 | 92 | 59 |
| 469 | Adenoma | Cecum | 86 | 82 | 39 | 78 | 29 |
| 282 | Adenoma | Ascending | 66 | 75 | 33 | 88 | 51 |
| 735 | Adenoma | Ascending | 72 | 88 | 34 | 94 | 62 |
| 575 | Adenoma | Ascending | 73 | 87 | 26 | 95 | 27 |
| 300 | Adenoma | Transverse | 59 | 89 | 54 | 100 | 52 |
| 772 | Adenoma | Rectum | 79 | 83 | 40 | 80 | 42 |
| 664 | FAP | Cecum | 52 | 87 | 48 | 92 | 53 |
| 610 | Lynch | Ascending | 59 | 88 | 17 | 91 | 42 |
| 726 | Adenoma | Ascending | 51 | 82 | 79 | 77 | 86 |
| 548 | Adenoma | Descending | 54 | 77 | 76 | 79 | 82 |
| 236 | FAP | Ascending | 26 | 80 | 91 | 90 | 91 |
| 245 | SSA | Ascending | 54 | 81 | 74 | 73 | 59 |
| 708 | SSA | Cecum | 76 | 89 | 65 | 92 | 82 |

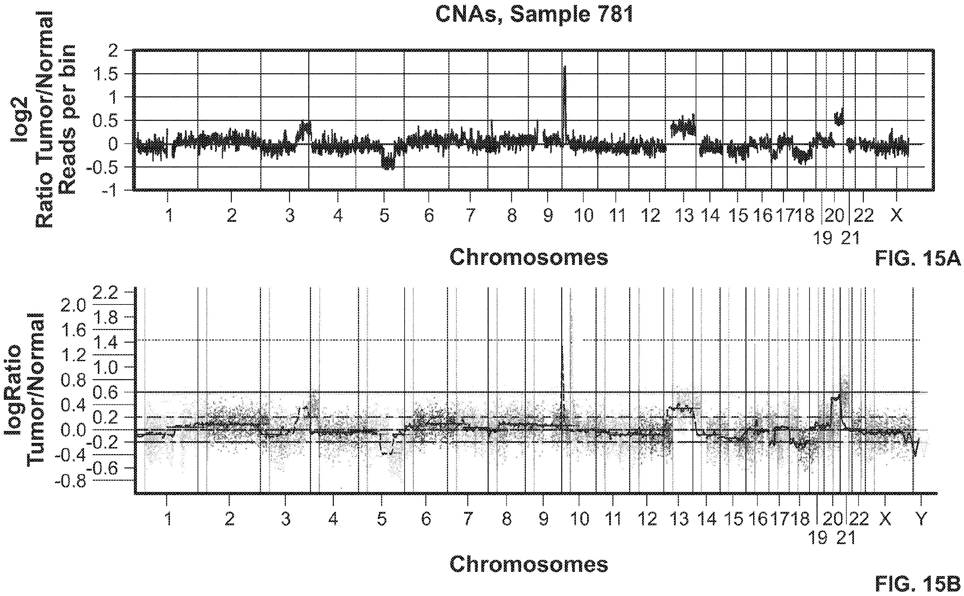

Copy number alterations present in the adenoma and the adenocarcinoma samples were determined by comparing the read density (RD) from tumor and normal samples. The read density is defined here as the total number of reads found in bins of one megabase. First, the reads were normalized for total number of reads to the samples with the highest total number of reads. A total of 3114 bins were created from the human genome hg19 (3,137,161,264 bases). The bins with less than 50 reads were removed from the analysis. A total of 2874 bins were preserved. The bins removed were mostly corresponding to the unknown bases in the hg19 assembly marked by 'N' (7.6 %) (UCSC). Note that the proportion of bins removed from the analysis (2874/3114*100 =7.7%) matched the unknown bases of the hg19 genome, suggesting that the assay target sites are well distributed throughout the usable hg19 genome. The bins on chromosome Y were also filtered out since some of the samples were females and had no reads mapping to chromosome Y. A total of 2867 bins were used to calculate and plot the log 2 ratio of the reads at every defined bin between the tumor and the normal adjacent samples (Figure 15, upper panel).

Alternatively, specialized software like Nexus 8.0 from BioDiscovery can be used to calculate the copy number variations based on read depth. FIG. 15 (lower panel) shows CNAs in one of the adenocarcinoma samples (sample 781). Gains are present at chr3q, chr10p, chr13q, chr20q and losses are visible in chr5q, chr17p and chr18q. Nexus 8.0 software shows in detail the CNAs events as well as identified cancer related genes positioned at CNAs events in sample 781 (See Table 4).

**Table 9:** Detailed description of CNAs events and associated cancer related genes

| Chr | Event | Length | Cytoband | # Probes | CancerGeneCensus-Sanger |
|---|---|---|---|---|---|
| chr3 | CN Gain | 41,921,869 | q25.2 - q29 | 235 | GMPS, MLF1, EVI1, PIK3CA, SOX2, ETV5, EIF4A2, BCL6, LPP, TFRC |
| chr5 | CN Loss | 48,154,516 | q14.3 - q31.1 | 188 | APC |

(continued)

| Chr | Event | Length | Cytoband | # Probes | CancerGeneCensus-Sanger |
|---|---|---|---|---|---|
| chr10 | High Copy Gain | 8,353,661 | p15.3 - p14 | 49 | GATA3 |
| chr13 | CN Gain | 96,145,424 | q11 - q34 | 435 | CDX2, FLT3, BRCA2, LHFP, LCP1, RB1, ERCC5 |
| chr17 | CN Loss | 5,493,187 | p13.3 - p13.2 | 84 | YWHAE, USP6 |
| chr17 | CN Loss | 1,616,776 | p13.1 | 24 | TP53, PER1 |
| chr18 | CN Loss | 47,300,916 | q11.2 - q22.2 | 202 | ZNF521, SS18, MALT1, BCL2 |
| chr20 | CN Gain | 35,525,521 | q11.1 - q13.33 | 294 | ASXL1, MAFB, TOP1, SDC4, GNAS, SS18L1 |

The genome instability can be reported as the percentage of bins with significant CNAs (gains or losses). This genome instability index is calculated by first determining the reads density at every megabase bin for the tumor and the normal samples as described above. For each bin, ZCNA is calculated as follow:

Where RDtumor is the read density in a bin of one megabase for a define tumor samples.

RDnormal is the average of the read density in a bin of one megabase for all the normal samples (n=18)

RDSD = the standard deviation of the read density from all the normal samples (n=18)

A total of 2867 different $Z_{CNA}$ were calculated. The $Z_{CNA}$ less than -3 and greater than 3 were judged significantly different than the normal samples. The percentage of bins with significant CNAs was reported in Table 5.

**Table 10:** Percentage of bins with significant CNAs

| ID | Tissue type | Location | Age | Percentage of bins with significant CNAs |
|---|---|---|---|---|
| 815 | Adenocarcinoma | Cecum | 74 | 14.4% |
| 861 | Adenocarcinoma | Ascending | 76 | 6.4% |
| G1-21 | Adenocarcinoma | Ascending | 57 | 6.5% |
| 781 | Adenocarcinoma | Descending | 52 | 12.9% |
| 227 | Adenoma | Cecum | 50 | 6.9% |
| 469 | Adenoma | Cecum | 86 | 14.7% |
| 726 | Adenoma | Ascending | 51 | 0.9% |
| 282 | Adenoma | Ascending | 66 | 0.4% |
| 735 | Adenoma | Ascending | 72 | 2.8% |
| 575 | Adenoma | Ascending | 73 | 1.9% |
| 300 | Adenoma | Transverse | 59 | 0.3% |
| 548 | Adenoma | Descending | 54 | 0.4% |
| 772 | Adenoma | Rectum | 79 | 9.6% |
| 236 | FAP | Ascending | 26 | 0.1% |
| 664 | FAP | Cecum | 52 | 1.1% |

(continued)

| ID | Tissue type | Location | Age | Percentage of bins with significant CNAs |
|----|-------------|----------|-----|------------------------------------------|
| 610 | Lynch | Ascending | 59 | 4.8% |
| 245 | SSA | Ascending | 54 | 0.4% |
| 708 | SSA | Cecum | 76 | 0.6% |

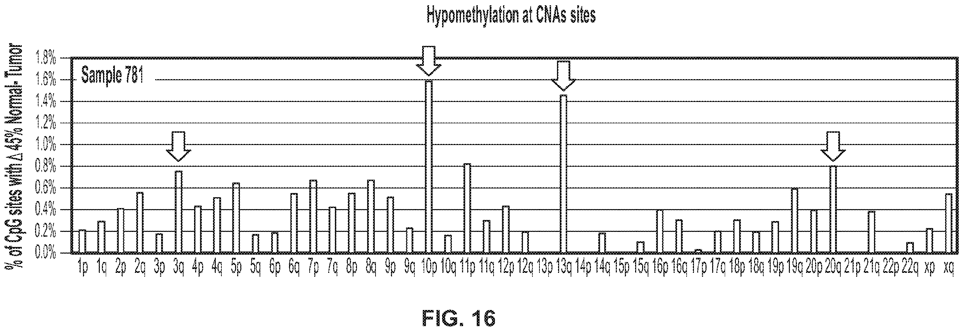

Different methylation status at specific bases can also be assessed between the tumor and the normal samples. For example, coverage files were imported into Seqmonk and the methylation status were analyzed for CpG sites with a coverage of at least 30x for the normal and the tumor samples. A total of 191,490 CpG sites met that criteria for sample 781. A total of 811 CpG sites exhibited significant difference of at least 45% between the normal and the tumor sample. Also the 811 sites were less methylated in the tumor sample than the normal sample. No site was more methylated in the tumor sample than the normal sample for a $\Delta$45%. Interestingly, most of the sites exhibiting hypomethylation in the tumor sample co-localized with the sites with a gain in copy number. The percentage of CpG sites with a $\Delta$45% between the normal and the tumor were plotted for every chromosome arms with at least one hundred CpG sites analyzed. See FIG. 16.

**Example 5: Determining Tissue-of-Origin**

The analysis of cell-free DNA (cfDNA) in plasma has been shown to be useful for different diagnostic purposes including, but not limited to, noninvasive prenatal testing and cancer detection; however, it has thus far proven difficult to determine the origin or location of different species of cfDNA in a mixture (e.g., to differentiate plasma cfDNA from hematopoietic cells vs. plasma cfDNA from liver cells) making the clinical utility of cfDNA assays somewhat limited.

Existing methods for determining the tissue-of-origin for cfDNA include the use of tissue-specific RNA expression patterns or tissue-specific methylation patterns. For example, Winston Koh et al. showed the RNA expression patterns of cell-free RNA in plasma can be correlated to certain tissue types (see Koh, et al. PNAS 2014 111 (20) 7361-7366). However, RNA is notoriously unstable, so when measured by RNASeq or RT-qPCR, it has so far proved and non-reliable for clinical use. Using tissue-specific methylation patterns to determine tissue-of-origin has previously relied on whole genome bisulfite sequencing. (See Sun et al. PNAS 2015 112 (40). This has shown tissue-of-origin can be determined from the methylation patterns of the cfDNA at specific loci in the genome. However, in order to get the required signal from whole genome bisulfite sequencing, deep sequencing is required which is time-consuming and expensive.

*Experiment and Results*

Using the compositions and methods described herein, one can determine the contributions of different tissues to a biological sample that includes a mixture of cell-free DNA from different tissues types, whereby one can analyze the methylation patterns of the DNA mixture (e.g., methylation levels at repeat sites in the genome) and determine the fractional makeup of various tissue types to the DNA mixture. In some embodiments, the methylation patterns of the tissue types that potentially contribute to the DNA mixture (candidate tissues) can be determined. Then, the methylation pattern of the DNA mixture of interest is determined. For example, methylation levels can be computed at various sites. Since the DNA mixture is composed of the DNA from the candidate tissues, the composition of the DNA mixture can be determined by comparing the methylation patterns of the DNA mixture and the candidate tissue types.

In some embodiments, a separation value (e.g., a subtracted difference or a ratio) in a contribution percentage of a particular tissue type in the DNA relative to a reference value can indicate a disease state. The reference value may correspond to a contribution percentage determined in a healthy individual, and a separation value greater than a threshold can determine a disease state, as the diseased tissue releases more cell-free DNA molecules than healthy tissue.

In a proof-of-concept study, the inventors were able to distinguish between DNA from the liver and from whole blood. Four samples with three replicates each of whole blood derived DNA and four sample with three replicates each of liver derived DNA were extracted. The methylation status for each of the samples was determined using the compositions and methods described herein. Specifically, the following MIP was used:

/5Phos/TTCTCCTACCTCAACCTCNNNNNNCTTCAGCTTCCCGATTACGGGCA

CGATCCGACGGTAGTGTNNNNNNCCAAACTAAAATACAATA (SEQ ID

NO:16)

*Analysis and Results*

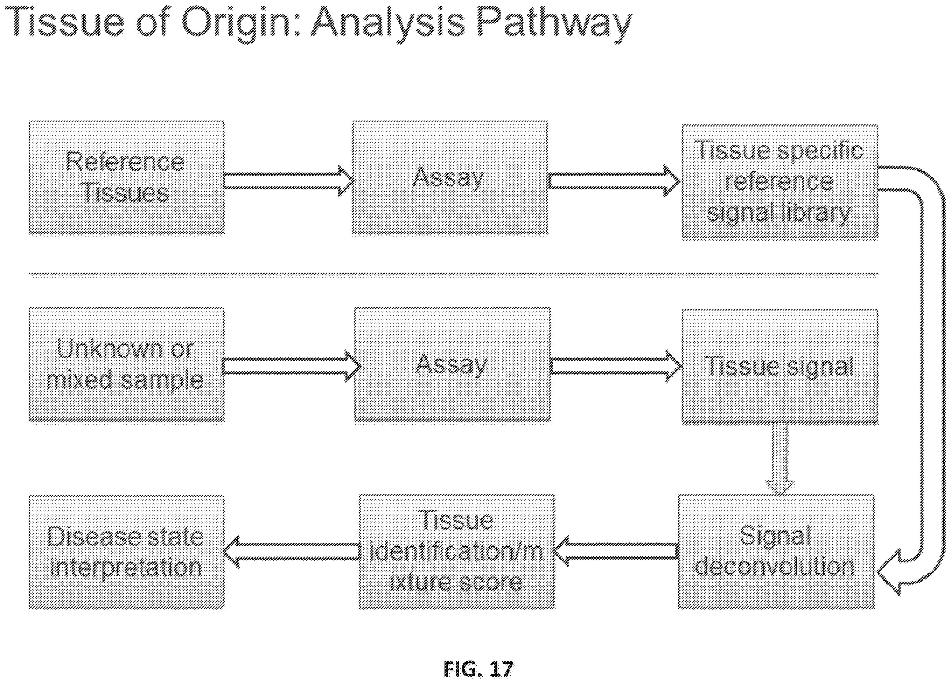

Generally speaking, the pathway of analysis to determine tissue-of-origin is similar independent of biology or measurement. See FIG. 17. First, a reference library is generated from an assay that has tissue-specific signals. Next, a deconvolution algorithm is used to interpret unknown samples and provide a percentage estimate of the unknown sample.

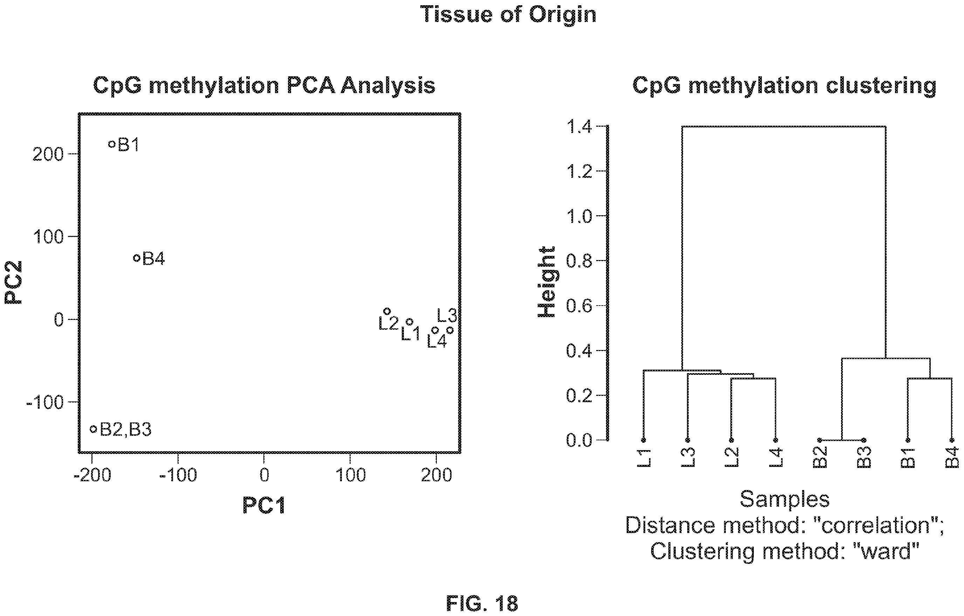

For the proof-of-concept study described here, a clustering analysis or principal component analysis of all of the CpG sites measured by the assay showed a distinct pattern of methylation between the different DNA species. As shown in FIG. 18, separation between blood-derived DNA (red) and liver DNA (blue) is greater between species than within a species.

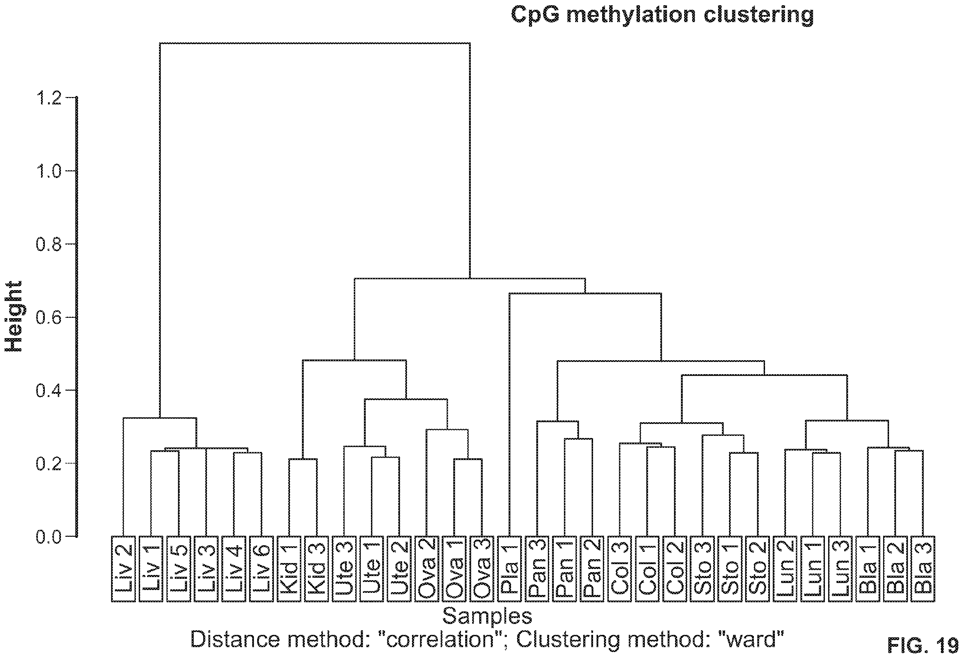

To see if the compositions and methods described herein could differentiate additional tissue types, DNA from differing tissue sources was obtained from different individuals. See FIG. 19, which shows, in order from left to right: liver (6), kidney(2), uterus(3), ovary (3), placenta (1), pancreas(3), colon(3), stomach (3), lung(3), bladder(3). Using the compositions and methods previously described herein, and a standard alignment and data processing package, the inventors were able to distinguish between the different tissue types. See the dendrogram provided in FIG. 19, which shows all samples from the same tissue source grouped together. The following MIP was used:

/5Phos/TTCTCCTACCTCAACCTCNNNNNNNNNNCTTCAGCTTCCCGATTACG

GGCACGATCCGACGGTAGTGTNNNNNNNNNNCCAAACTAAAATACAATA

(SEQ ID NO:21).

**Example 6: Determination of Methylation Age**

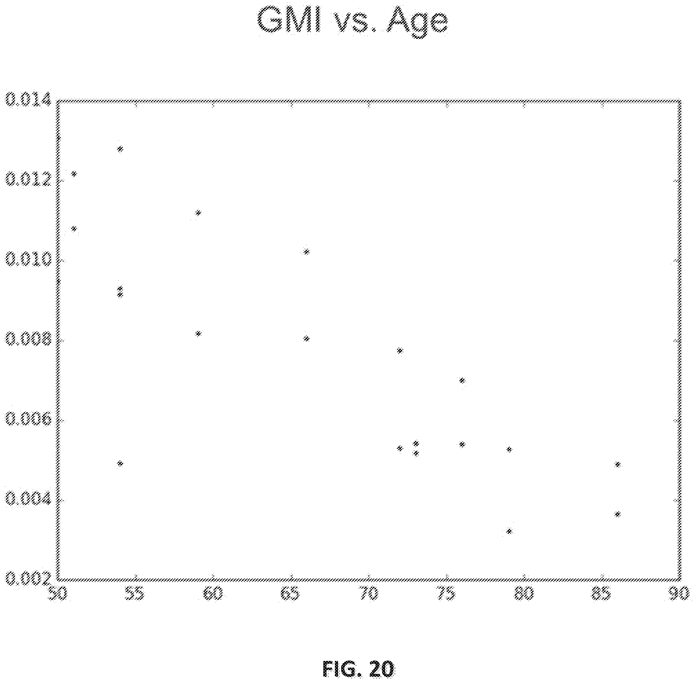

Methylation status is known to change over time with particular tissues becoming hyper- or hypomethylated at different rates depending on a range of factors, including exposure to environmental factors or the presence of disease. Therefore, determining the methylation status as described herein can provide a measure of "bio age", which may provide an early indication of the presence of age-related pathologies. For example, using the compositions and methods described herein with the tissue samples described in Example 4, it was shown that overall methylation decreases with age of the subject. See FIG. 20. Regional summaries of methylation were computed using a wavelet based sliding window analysis, and coefficients were examined for correlation to age of the subject. For each genomic region, the correlation between the region coefficient and sample age, as well as the associated p-value were computed. The p-values were corrected for multiple hypotheses using the Benjamini-Hochberg method, and significant correlations after correction were illustrated.

*Gestational Age as Predicted by Global Methylation Index (GMI)*

Using the compositions and methods described herein, 14 women over five time points during pregnancy had blood extracted into a Streck® tube. DNA from plasma was purified. Using the following MIP:

/5Phos/TTCTCCTACCTCAACCTCNNNNNNCTTCAGCTTCCCGATTAC

GGGCACGATCCGACGGTAGTGTNNNNNNCCAAACTAAAATACAATA (SEQ

ID NO:16), and

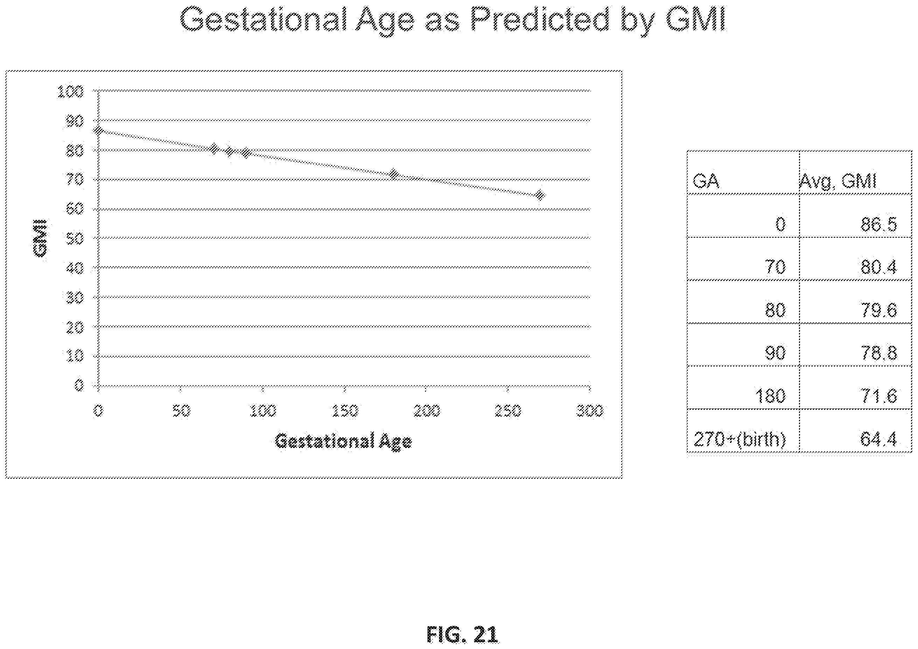

the methods described herein, the global methylation index (GMI) was determined for each sample at each of the five gestational age (GA) time points. As shown in FIG. 21, GMI decreases with GA in a linear manner until birth. The increase of hypomethylated DNA is expected as placental DNA increases in abundance as a percentage of total plasma DNA.

**Example 7: Detection of 5'hydroxymethylation**

5'hydroxymethylcytosine (5hmC) originates from the oxidation of 5' methylcytosine (5mC). The conversion of 5mC to 5hmC is an intermediate step in the active demethylation process. In cells, this reaction is catalyzed by the ten-eleven translocation enzyme family (TET). 5'hydroxymethylcytosine level is often dysregulated in cancer and may contribute to tumor development and progression.

Bisulfite conversion does not distinguish between 5hmC and 5mC. Both modifications prevent the conversion of cytosines to uracils. To discriminate 5hmC from 5mC using the compositions and methods described herein, gDNA is extracted from a sample and divided into 2 reactions: 1) regular bisulfite conversion and 2) denaturation of gDNA follow by oxidation of 5hmC to 5-formylcytosine using potassium perruthenate, followed by conversion of 5-formylcytosine to uracil with bisulfite. After MIP capture and sequencing as described herein, the sites of 5hmC are detected by comparing the data from reactions 1 and 2: In reaction 1, both 5hmC and 5mC are found as cytosines, whereas unmethylated cytosines are found as thymines. In reaction 2, 5mC are found as cytosines but the 5hmC and unmethylated cytosines are found as thymines. The hydroxymethylation status, as well as the hydroxymethylation density can be calculated as described in Example 4.

**Claims**

1. A computer implementedmethod of selecting a molecular inversion probe (MIP) from a plurality of candidate MIPs for using to detect methylation in a subject, the method comprising:

   a) receiving nucleic acid sequences of the plurality of candidate MIPs, wherein each of the MIPs in the plurality of candidate MIPs comprises in sequence the following components: first targeting polynucleotide arm - first unique molecular tag - polynucleotide linker - second unique molecular tag - second targeting polynucleotide arm;
   b) for each respective MIP in the plurality of candidate MIPs,

   i) computing a first number (A) of unique CpG sites predicted, with no mismatch on the binding arm sequence, to be captured by the respective MIP;
   ii) computing a second number (C) of unique CpG sites predicted, with one mismatch on the binding arm sequence, to be captured by the respective MIP;
   iii) computing a third number (E) of unique sites predicted, with no mismatch on the binding arm sequence, to be captured by the respective MIP across a genome;
   iv) computing a fourth number (G) of unique sites predicted, with one mismatch on the binding arm sequence, to be captured by the respective MIP across the genome;
   v) computing a fifth number (F) of non-unique sites predicted, with no mismatch on the binding arm sequence, to be captured by the respective MIP across the genome;
   vi) computing a sixth number (H) of non-unique sites predicted, with one mismatch on the binding arm sequence, to be captured by the respective MIP across the genome;
   vii) computing a seventh number (I) of CpG sites present on the first targeting polynucleotide arm;
   viii) computing an eighth number (J) of CpG sites present on the second targeting polynucleotide arm;
   ix) computing a performance metric for the respective MIP based at least in part on the first, second, third, fourth, fifth, sixth, seventh, and eighth numbers;

   c) selecting a MIP, based at least in part on the performance metric computed in step b)ix) for each MIP in the plurality of candidate MIPs, and wherein

   - the MIP at step c) is selected such that a sum of the seventh number (I) and the eighth number (J) is smaller than the corresponding sum for a remaining set of the candidate MIPs, and/or
   - a first sum is a sum of the first number (A) and the second number (C);
   a second sum is a sum of the third number (E), the fourth number (G), the fifth number (F), and the sixth number (H);
   and the MIP at step c) is selected such that a ratio between the first sum and the second sum is larger than the ratio for a remaining set of the candidate MIPs, and wherein preferably: a third sum is a sum of the third number (E) and the fourth number (G);
   a fourth sum is a sum of the third number (E), the fourth number (G), the fifth number (F), and the sixth number (H); and

the MIP at step c) is selected such that a ratio between the third sum and the fourth sum is larger than the ratio for a remaining set of the candidate MIPs.

2. A method of determining the methylation state of a nucleic acid in a subject, the method comprising:

a) obtaining a nucleic acid sample isolated from a blood sample from the subject, wherein the blood sample is preferably a whole blood sample, a plasma sample, or a serum sample, further preferably a plasma sample;

b) performing bisulfite conversion of the nucleic acid sample;

c) capturing a plurality of target sequences of interest in the nucleic acid sample obtained in step b) to produce a plurality of replicons by selecting one or more populations of molecular inversion probes (MIPs) according to the method of claim 1 and by using the selected MIPs in an amount dependent on the number of genomes used as a template, the overall number of sites, targeted by the specific probe, the ratio of invariant sites to variant sites, and the diversity of genomic CpG sites desired,

wherein each of the MIPs in the population of MIPs comprises in sequence the following components:

first targeting polynucleotide arm - first unique molecular tag - polynucleotide linker - second unique molecular tag - second targeting polynucleotide arm;

wherein the first targeting polynucleotide arm in each of the MIPs is substantially complementary to a first repeat region, and the second targeting polynucleotide arm in each of the MIPs is substantially complementary to a second repeat region, further wherein the first and second repeat regions, respectively, flank each sequence in the plurality of target sequences of interest;

wherein the first and second unique targeting molecular tags in each of the MIPs in combination are distinct in each of the MIPs;

wherein the target sequence of interest in the plurality of target sequences of interest has one or more CpG sites;

wherein each CpG site has a corresponding known position within the target sequence of interest;

d) sequencing a plurality of MIPs amplicons that are amplified from the replicons obtained in step c); and

e) determining the number of occurrences of cytosine nucleotides at each corresponding CpG site within the MIPs amplicons sequenced at step d) to determine the methylation state of a nucleic acid.

3. The method of claim 2, wherein the length of the first and/or second targeting polynucleotide arm is between 14 and 30 bases; wherein each of the targeting polynucleotide arms preferably has a melting temperature between 45 °C and 66 °C and/or each of the targeting polynucleotide arms has a GC content between 10% and 40%.

4. The method of any one of claims 2-3, wherein the length of the first and/or second unique molecular tag is between 4 and 15 bases.

5. The method of any one of claims 2-4, wherein the polynucleotide linker:

- is not substantially complementary to any genomic region of the subject, and/or
- has a length of between 14 and 50 bases, and/or
- has a melting temperature of between 45 °C and 85 °C, and/or
- has a GC content between 30% and 66%.

6. The method of any one of claims 2-5 wherein the polynucleotide linker comprises at least one amplification primer binding site, preferably a forward amplification primer binding site.

7. The method of claim 6, wherein the sequence of the forward amplification primer comprises the nucleotide sequence of CCGTAATCGGGAAGCTGAAG (SEQ ID NO:1).

8. The method of any one of claims 2-7, wherein:

- the sequence of a reverse amplification primer comprises the nucleotide sequence of GCACGATCCGACGGTAGTGT (SEQ ID NO:2); and/or
- the polynucleotide linker comprises the nucleotide sequence of CTTCAGCTTCCCGATTACGGGCACGATCCGACGGTAGTGT (SEQ ID NO:3); and/or

- the first targeting polynucleotide arm comprises the nucleotide sequence of CACTACACTCCAACCTAA (SEQ ID NO:4) or TTCTCCTACCTCAACCTC (SEQ ID NO:5); and/or
the second targeting polynucleotide arm comprises the nucleotide sequence of CAAAAAACTAAAACAAAA (SEQ ID NO:6) or CCAAACTAAAATACAATA (SEQ ID NO:7).

9. The method of any one of claims 1-8, wherein the MIP comprises the nucleotide sequence of CACTACACTCCAACCTAA(N1) CTTCAGCTTCCCGATTACGGGCACGATCCGACGGTAGTGT(N2) CAAAAAACTAAAACAAAA (SEQ ID NO:8) or TTCTCCTACCTCAACCTC(N1) CTTCAGCTTCCCGATTACGGGCACGATCCGACGGTAGTGT(N2) CCAAACTAAAATACAATA (SEQ ID NO:9), wherein (N1) represents the first unique molecular tag sequence of nucleotides and (N2) represents the second unique molecular tag sequence of nucleotides.

10. The method of any one of claims 2-9, wherein each of the MIPs replicons obtained in step c) is produced by:

   i) the first and second targeting polynucleotide arms, respectively, hybridizing to the first and second repeat regions in the nucleic acid sample, respectively, wherein the first and second repeat regions flank a target sequence of interest; and
   ii) after the hybridization, using a ligation/extension mixture to extend and ligate the gap region between the two targeting polynucleotide arms to form single-stranded circular nucleic acid molecules.

11. The method of any one of claims 2-10, wherein the sequencing step of d) comprises a next generation sequencing method, preferably a massively parallel sequencing method.

12. The method of any one of claims 2-11 wherein the method comprises, before the sequencing step of d), a PCR reaction to amplify the MIPs replicons for sequencing; wherein the PCR reaction is preferably an indexing PCR reaction that introduces into each of the MIPs amplicons the following components: a pair of sequencing adapters comprising a unique molecular tag for multiplexed sequencing, further preferably, wherein the barcoded MIPs amplicons comprise in sequence the following components:
a first sequencing adaptor - a first sequencing primer binding site - the first unique targeting molecular tag - the first targeting polynucleotide arm - captured nucleic acid - the second targeting polynucleotide arm - the second unique targeting molecular tag - a second sequencing primer binding site - a unique sample barcode - a second sequencing adaptor.

13. The method of any one of claims 2-12, said method further comprising obtaining sequencing information from the one or more populations of MIPs, wherein said sequencing information is preferably used in the determination of the methylation state of a nucleic acid in a subject, or used in the determination of whether the subject has the predisposition to the disease or condition, or used in diagnosing the disease or condition, or used in detecting the state of the disease or condition, or used in differentiating the nucleic acid species originating from the subject and the one or more additional individuals, or used in differentiating the nucleic acid species originating from the first tissue and the one or more additional tissues, or used in the determination of the methylation state of a nucleic acid and detection of copy number variation in the subject, or used in the determination of the methylation age of the subject, or used in the determination of the methylation age of the tissue, respectively.

14. A nucleic acid molecule comprising a nucleotide sequence of CACTACACTC CAACCTAA(N1) CTTCAGCTTCCCGATTACGGGCACGATCCGACGGTAGTGT(N2) CAAAAAACTAAAACAAAA (SEQ ID NO:8) or TTCTCCTAC-CTCAACCTC(N1) CTTCAGCTTCCCGATTACGGGCACGATCCGACGGTAGTGT(N2) CCAAACTAAAATACAA-TA (SEQ ID NO:9), wherein (N1) represents a first unique molecular tag sequence of nucleotides and (N2) represents a second unique molecular tag sequence of nucleotides.

15. The nucleic acid of claim 14, wherein the length of the first unique molecular tag is between 4 and 15 bases and/or wherein the length of the second unique molecular tag is between 4 and 15 bases.

**Patentansprüche**

1. Computerimplementiertes Verfahren zur Auswahl einer molekularen Inversionssonde (MIP) aus einer Vielzahl von MIP-Kandidaten zur Verwendung zum Detektieren von Methylierung in einem Subjekt, wobei das Verfahren umfasst:

   a) Erhalten von Nukleinsäuresequenzen der Vielzahl von MIP-Kandidaten, wobei jeder der MIPs in der Vielzahl

EP 3 377 647 B1

der MIP-Kandidaten in der Reihenfolge die folgenden Komponenten umfasst:
erster Targeting-Polynukleotidarm - erste spezifische molekulare Markierung - Polynukleotidlinker - zweite spezifische molekulare Markierung - zweiter Targeting-Polynukleotidarm;
b) für jedes jeweilige MIP der Vielzahl der Kandidaten-MIPs,

i) Berechnung einer ersten Zahl (A) spezifischer vorhersehbarer CpG-Stellen, ohne Mismatch an der Bindungsarmsequenz, die von dem jeweiligen MIP erfasst werden soll;
ii) Berechnung einer zweiten Zahl (C) spezifischer vorhersehbarer CpG-Stellen, mit einem Mismatch an der Bindungsarmsequenz, die von dem jeweiligen MIP erfasst werden soll;
iii) Berechnung einer dritten Zahl (E) spezifischer vorhersehbarer CpG-Stellen, ohne Mismatch an der Bindungsarmsequenz, die von dem jeweiligen MIP erfasst werden soll;
iv) Berechnung einer vierten Zahl (G) spezifischer vorhersehbarer Stellen, mit einem Mismatch an der Bindungsarmsequenz, die von dem jeweiligen MIP im Genom erfasst werden soll;
v) Berechnung einer fünften Zahl (F) nichtspezifischer vorhersehbarer Stellen, ohne Mismatch an der Bindungsarmsequenz, die von dem jeweiligen MIP im Genom erfasst werden soll;
vi) Berechnung einer sechsten Zahl (H) nichtspezifischer vorhersehbarer Stellen, mit einem Mismatch an der Bindungsarmsequenz, die von dem jeweiligen MIP im Genom erfasst werden soll;
vii) Berechnung einer siebten Zahl (I) von CpG-Stellen, die auf dem ersten Targeting-Polynukleotidarm vorhanden sind;
viii) Berechnung einer achten Zahl (J) von CpG-Stellen, die auf dem zweiten Targeting-Polynukleotidarm vorhanden sind;
ix) Berechnung einer Performance-Metrik für den jeweiligen MIP, die zumindest teilweise auf der ersten, zweiten, dritten, vierten, fünften, sechsten, siebten und achten Zahl basiert;

c) Auswahl eines MIPs, zumindest teilweise basierend auf der in Schritt b)ix) berechneten Performance-Metrik für jedes MIP in der Vielzahl von Kandidaten-MIPs, und wobei

- das MIP in Schritt c) so gewählt wird, dass eine Summe aus der siebten Zahl (I) und der achten Zahl (J) kleiner ist als die entsprechende Summe für einen verbleibenden Satz der Kandidaten-MIPs, und/oder
- eine erste Summe die Summe der ersten Zahl (A) und der zweiten Zahl (C) ist;
eine zweite Summe eine Summe der dritten Zahl (E), der vierten Zahl (G), der fünften Zahl (F) und der sechsten Zahl (H) ist;
und die MIP in Schritt c) so ausgewählt wird, dass ein Verhältnis zwischen der ersten Summe und der zweiten Summe größer als das Verhältnis für einen verbleibenden Satz der Kandidaten-MIPs ist, und wobei vorzugsweise:

eine dritte Summe eine Summe der dritten Zahl (E) und
der vierten Zahl (G) ist;
eine vierte Summe eine Summe aus der dritten Zahl (E), der vierten Zahl (G), der fünften Zahl (F) und der sechsten Zahl (H) ist; und
das MIP in Schritt c) so gewählt wird, dass das Verhältnis zwischen der dritten und der vierten Summe größer ist als das Verhältnis für einen verbleibenden Satz der MIP-Kandidaten.

2. Verfahren zur Bestimmung des Methylierungszustandes einer Nukleinsäure in einem Subjekt, wobei das Verfahren umfasst:

a) Erhalten einer Nukleinsäureprobe, die aus einer Blutprobe des Subjekts isoliert wurde, wobei die Blutprobe vorzugsweise eine Vollblutprobe, eine Plasmaprobe oder eine Serumprobe, weiter vorzugsweise eine Plasmaprobe, ist;
b) Durchführen einer Bisulfit-Umwandlung der Nukleinsäureprobe;
c) Einfangen einer Vielzahl von Zielsequenzen von Interesse in der in Schritt b) erhaltenen Nukleinsäureprobe, um eine Vielzahl von Replikons zu erzeugen, indem eine oder mehrere Populationen von molekularen Inversionssonden (MIPs) gemäß dem Verfahren nach Anspruch 1 ausgewählt werden und indem die ausgewählten MIPs in einer Menge verwendet werden, abhängig von der Anzahl der als Template verwendeten Genome, der Gesamtzahl der Stellen, auf die die spezifische Sonde abzielt, dem Verhältnis von invarianten Stellen zu Variantenstellen und der gewünschten Diversität von genomischen CpG-Stellen,
wobei jede der MIPs in der Population der MIPs nacheinander die folgenden Komponenten umfasst:
erster Targeting-Polynukleotidarm - erste spezifische molekulare Markierung - Polynukleotidlinker - zweite spezifische molekulare Markierung - zweiter Targeting-Polynukleotidarm;

wobei der erste Targeting-Polynukleotidarm in jedem der MIPs im Wesentlichen komplementär zu einer ersten Wiederholungsregion ist und der zweite Targeting-Polynukleotidarm in jedem der MIPs im Wesentlichen komplementär zu einer zweiten Wiederholungsregion ist, wobei ferner die erste und zweite Wiederholungsregion jeweils jede Sequenz in der Vielzahl der Zielsequenzen von Interesse flankiert;

wobei die ersten und zweiten spezifischen molekularen Zielmarkierungen in jeder der MIPs in Kombination in jeder der MIPs unterschiedlich sind;

wobei die Zielsequenz von Interesse in der Vielzahl der Zielsequenzen von Interesse eine oder mehrere CpG-Stellen aufweist;

wobei jede CpG-Stelle eine entsprechende bekannte Position innerhalb der Zielsequenz von Interesse hat;

d) Sequenzierung einer Vielzahl von MIPs-Amplikons, die aus den in Schritt c) erhaltenen Replikons amplifiziert werden; und

e) Bestimmung der Zahl des Vorkommens von Cytosin-Nukleotiden an jeder entsprechenden CpG-Stelle innerhalb der in Schritt d) sequenzierten MIP-Amplikons, um den Methylierungszustand einer Nukleinsäure zu bestimmen.

3. Verfahren nach Anspruch 2, wobei die Länge des ersten und/oder zweiten Targeting-Polynukleotidarms zwischen 14 und 30 Basen beträgt; wobei jeder der Targeting-Polynukleotidarme vorzugsweise eine Schmelztemperatur zwischen 45 °C und 66 °C aufweist und/oder jeder der Targeting-Polynukleotidarme einen GC-Gehalt zwischen 10% und 40% aufweist.

4. Verfahren nach einem der Ansprüche 2-3, wobei die Länge des ersten und/oder zweiten spezifischen molekularen Tags zwischen 4 und 15 Basen beträgt.

5. Verfahren nach einem der Ansprüche 2-4, wobei der Polynukleotidlinker:

   - nicht wesentlich komplementär zu irgendeiner genomischen Region des Subjekts ist, und/oder
   - eine Länge zwischen 14 und 50 Basen hat, und/oder
   - eine Schmelztemperatur zwischen 45 °C und 85 °C hat und/oder
   - einen GC-Gehalt zwischen 30% und 66% hat.

6. Verfahren nach einem der Ansprüche 2-5, wobei der Polynukleotidlinker mindestens eine Amplifikationsprimer-Bindungsstelle, vorzugsweise eine Vorwärts-Amplifikationsprimer-Bindungsstelle, umfasst.

7. Verfahren nach Anspruch 6, wobei die Sequenz des Vorwärts-Amplifikationsprimers die Nukleotidsequenz CCGTAATCGGGAAGCTGAAG (SEQ ID Nr.:1) umfasst.

8. Verfahren nach einem der Ansprüche 2-7, wobei:

   - die Sequenz eines Rückwärts-Amplifikationsprimers die Nukleotidsequenz GCACGATCCGACGGTAGTGT (SEQ ID Nr.:2) umfasst; und/oder
   - der Polynukleotidlinker die Nukleotidsequenz CTTCAGCTTCCCGATTACGGGCACGATCCGACGGTAGTGT (SEQ ID Nr.:3) umfasst; und/oder
   - der erste Targeting-Polynukleotidarm die Nukleotidsequenz CACTACACTCCAACCTAA (SEQ ID Nr.:4) oder TTCTCCTACCTCAACCTC (SEQ ID Nr.:5) umfasst; und/oder
   - der zweite Targeting-Polynukleotidarm die Nukleotidsequenz CAAAAAACTAAAACAAAA (SEQ ID Nr.:6) oder CCAAACTAAAATACAATA (SEQ ID Nr.:7) umfasst.

9. Verfahren nach einem der Ansprüche 1-8, wobei das MIP die Nukleotidsequenz CACTACACTCCAACCTAA(N1) CTTCAGCTTCCCGATTACGGGCACGATCCGACGGTAGTGT(N2) CAAAAAACTAAAACAAAA (SEQ ID Nr.:8) oder TTCTCCTACCTCAACCTC(N1) CTTCAGCTTCCCGATTACGGGCACGATCCGACGGTAGTGT(N2) CCAAACTAAAATACAATA (SEQ ID Nr.:9) umfasst, wobei (N1) die erste spezifische molekulare Zielsequenz von Nukleotiden und (N2) die zweite spezifische molekulare Zielsequenz von Nukleotiden darstellt.

10. Verfahren nach einem der Ansprüche 2-9, wobei jedes der in Schritt c) erhaltenen MIP-Replikons hergestellt wird durch:

   i) die ersten und zweiten Targeting-Polynukleotidarme, die jeweils mit den ersten und zweiten Wiederholungsregionen in der Nukleinsäureprobe hybridisieren, wobei die ersten und zweiten Wiederholungsregionen eine Zielsequenz von Interesse flankieren; und

ii) nach der Hybridisierung, unter Verwendung eines Ligation/Extension-Gemisches zur Verlängerung und Ligation der Lückenregion zwischen den beiden Targeting-Polynukleotidarmen, um einzelsträngige zirkuläre Nukleinsäuremoleküle zu bilden.

11. Verfahren nach einem der Ansprüche 2-10, wobei der Sequenzierungsschritt d) ein Sequenzierungsverfahren der nächsten Generation, vorzugsweise ein massiv paralleles Sequenzierungsverfahren, umfasst.

12. Verfahren nach einem der Ansprüche 2 bis 11, wobei das Verfahren vor dem Sequenzierungsschritt d) eine PCR-Reaktion zur Amplifikation der MIP-Replikons zur Sequenzierung umfasst; wobei die PCR-Reaktion vorzugsweise eine indizierende PCR-Reaktion ist, die in jedes der MIP-Amplikons die folgenden Komponenten einführt: ein Paar Sequenzierungsadapter, die eine spezifische molekulare Markierung zur multiplexen Sequenzierung umfassen, weiter vorzugsweise, wobei die barkodierten MIP-Amplikons in der Reihenfolge die folgenden Komponenten umfassen:

einen ersten Sequenzierungsadapter - eine erste Sequenzierungsprimer-Bindungsstelle - die erste spezifische molekulare Zielmarkierung - den ersten Targeting-Polynukleotidarm

- eingefangene Nukleinsäure - den zweiten Targeting-Polynukleotidarm - die zweite spezifische molekulare Zielmarkierung - eine zweite Sequenzierungsprimer-Bindungsstelle
- einen spezifischen Proben-Barcode - einen zweiten Sequenzierungsadapter.

13. Verfahren nach einem der Ansprüche 2 bis 12, wobei das Verfahren des Weiteren das Erhalten von Sequenzierungsinformationen von einer oder mehreren Populationen von MIPs umfasst, wobei die Sequenzierungsinformationen vorzugsweise bei der Bestimmung des Methylierungszustands einer Nukleinsäure in einem Subjekt verwendet werden, oder bei der Bestimmung, ob das Subjekt die Prädisposition für die Krankheit oder den Zustand hat, oder bei der Diagnose der Krankheit oder des Zustands verwendet werden, oder bei der Detektion des Zustands der Krankheit oder des Zustands verwendet werden, oder zur Differenzierung der Nukleinsäurespezies, die von dem Subjekt und einem oder mehreren zusätzlichen Individuen stammen, oder zur Differenzierung der Nukleinsäurespezies, die vom ersten Gewebe und einem oder mehreren zusätzlichen Geweben stammen, oder zur Bestimmung des Methylierungszustandes einer Nukleinsäure und zur Detektion der Kopienzahlvariation bei dem Subjekt, oder zur Bestimmung des Methylierungsalters des Subjekts, oder zur Bestimmung des Methylierungsalters des Gewebes verwendet werden.

14. Nukleinsäuremolekül, umfassend eine Nukleotidsequenz CACTACACTCCAACCTAA(N1) CTTCAGCTTCCCGATTACGGGCACGATCCGACGGTAGTGT (N2) CAAAAAACTAAAACAAAA (SEQ ID NO:8) or TTCTCCTACCTCAACCTC(N1) CTTCAGCTTCCCGATTACGGGCACGATCCGACGGTAGTGT(N2) CCAAACTAAAATACAATA (SEQ ID Nr.:9), wobei (N1) eine erste spezifische molekulare Zielsequenz von Nukleotiden und (N2) eine zweite spezifische molekulare Zielsequenz von Nukleotiden darstellt.

15. Nukleinsäure nach Anspruch 14, wobei die Länge der ersten spezifischen molekularen Markierung zwischen 4 und 15 Basen beträgt und/oder wobei die Länge der zweiten spezifischen molekularen Markierung zwischen 4 und 15 Basen beträgt.

**Revendications**

1. Procédé mis en œuvre par ordinateur consistant à sélectionner une sonde d'inversion moléculaire (MIP) parmi une pluralité de MIP candidates destinée à être utilisée pour détecter la méthylation chez un sujet, le procédé comprenant :

a) la réception de séquences d'acide nucléique de la pluralité de MIP candidates, chacune des MIP de la pluralité de MIP candidates comprenant dans l'ordre les composants suivants : premier bras polynucléotidique de ciblage - premier marqueur moléculaire unique - lieur polynucléotidique - second marqueur moléculaire unique - second bras polynucléotidique de ciblage ;

b) pour chaque MIP respective de la pluralité de MIP candidates,

i) le calcul par ordinateur d'un premier nombre (A) d'îlots CpG uniques prédits, sans mésappariement sur la séquence de bras de liaison, être capturés par la MIP respective ;

ii) le calcul par ordinateur d'un deuxième nombre (C) d'îlots CpG uniques prédits, avec un mésappariement sur la séquence de bras de liaison, être capturés par la MIP respective ;

iii) le calcul par ordinateur d'un troisième nombre (E) de sites uniques prédits, sans mésappariement sur la séquence de bras de liaison, être capturés par la MIP respective sur toute l'étendue d'un génome ;

iv) le calcul par ordinateur d'un quatrième nombre (G) de sites uniques prédits, avec un mésappariement sur la séquence de bras de liaison, être capturés par la MIP respective sur toute l'étendue du génome ;

v) le calcul par ordinateur d'un cinquième nombre (F) de sites non uniques prédits, sans mésappariement sur la séquence de bras de liaison, être capturés par la MIP respective sur toute l'étendue du génome ;

vi) le calcul par ordinateur d'un sixième nombre (H) de sites non uniques prédits, avec un mésappariement sur la séquence de bras de liaison, être capturés par la MIP respective sur toute l'étendue du génome ;

vii) le calcul par ordinateur d'un septième nombre (I) d'îlots CpG présents sur le premier bras polynucléotidique de ciblage ;

viii) le calcul par ordinateur d'un huitième nombre (J) d'îlots CpG présents sur le second bras polynucléotidique de ciblage ;

ix) le calcul par ordinateur d'une mesure de performance pour la MIP respective sur la base au moins en partie des premier, deuxième, troisième, quatrième, cinquième, sixième, septième et huitième nombres ;

c) la sélection d'une MIP, sur la base au moins en partie de la mesure de performance calculée par ordinateur dans l'étape b)ix) pour chaque MIP de la pluralité de MIP candidates, et dans lequel

- la MIP à l'étape c) est sélectionnée de façon telle qu'une somme du septième nombre (I) et du huitième nombre (J) est plus petite que la somme correspondante pour un ensemble restant des MIP candidates et/ou
- une première somme est une somme du premier nombre (A) et du deuxième nombre (C) ;
une deuxième somme est une somme du troisième nombre (E), du quatrième nombre (G), du cinquième nombre (F) et du sixième nombre (H) ; et
la MIP à l'étape c) est sélectionnée de façon telle qu'un rapport entre la première somme et la deuxième somme est plus grand que le rapport pour un ensemble restant des MIP candidates et dans lequel de préférence :

une troisième somme est une somme du troisième nombre (E) et du quatrième nombre (G) ;
une quatrième somme est une somme du troisième nombre (E), du quatrième nombre (G), du cinquième nombre (F) et du sixième nombre (H) ; et
la MIP à l'étape c) est sélectionnée de façon telle qu'un rapport entre la troisième somme et la quatrième somme est plus grand que le rapport pour un ensemble restant des MIP candidates.

2. Procédé de détermination de l'état de méthylation d'un acide nucléique chez un sujet, le procédé comprenant :

a) l'obtention d'un échantillon d'acide nucléique isolé à partir d'un échantillon de sang provenant du sujet, l'échantillon de sang étant de préférence un échantillon de sang total, un échantillon de plasma ou un échantillon de sérum, de préférence encore un échantillon de plasma ;
b) la mise en œuvre d'une conversion au bisulfite de l'échantillon d'acide nucléique ;
c) la capture d'une pluralité de séquences présentant de l'intérêt cibles dans l'échantillon d'acide nucléique obtenu dans l'étape b) pour produire une pluralité de réplicons par la sélection d'une ou plusieurs populations de sondes d'inversion moléculaires (MIP) selon le procédé de la revendication 1 et par l'utilisation des MIP sélectionnées en une quantité qui est fonction du nombre de génomes utilisés en tant que matrice, du nombre global de sites, ciblés par la sonde spécifique, du rapport des sites invariants aux sites variants et de la diversité des îlots CpG génomiques souhaitée,

chacune des MIP de la population de MIP comprenant dans l'ordre les composants suivants : premier bras polynucléotidique de ciblage - premier marqueur moléculaire unique - lieur polynucléotidique - second marqueur moléculaire unique - second bras polynucléotidique de ciblage ;
le premier bras polynucléotidique de ciblage dans chacune des MIP étant en grande partie complémentaire à une première région de répétition et le second bras polynucléotidique de ciblage dans chacune des MIP étant en grande partie complémentaire à une seconde région de répétition, en outre les première et seconde régions de répétition, respectivement, flanquant chaque séquence de la pluralité de séquences présentant de l'intérêt cibles ;
les premier et second marqueurs moléculaires de ciblage uniques dans chacune des MIP en association étant distincts dans chacune des MIP ;

la séquence présentant de l'intérêt cible de la pluralité de séquences présentant l'intérêt cibles ayant un ou plusieurs îlots CpG ;

chaque îlot CpG ayant une position connue correspondante au sein de la séquence présentant de l'intérêt cible ;

d) le séquençage d'une pluralité d'amplicons des MIP qui sont amplifiés à partir des réplicons obtenus dans l'étape c) ; et

e) la détermination du nombre d'occurrences de nucléotides de cytosine au niveau de chaque îlot CpG correspondant au sein des amplicons des MIP séquencés à l'étape d) pour déterminer l'état de méthylation d'un acide nucléique.

3. Procédé selon la revendication 2, dans lequel la longueur du premier bras polynucléotidique de ciblage et/ou du second bras polynucléotidique de ciblage est comprise entre 14 et 30 bases ; dans lequel chacun des bras polynucléotidiques de ciblage a de préférence une température de fusion comprise entre 45 °C et 66 °C et/ou chacun des bras polynucléotidiques de ciblage a une teneur en GC comprise entre 10 % et 40 %.

4. Procédé selon l'une quelconque des revendications 2-3, dans lequel la longueur du premier marqueur moléculaire unique et/ou du second marqueur moléculaire unique est comprise entre 4 et 15 bases.

5. Procédé selon l'une quelconque des revendications 2-4, dans lequel le lieur polynucléotidique :

- n'est pas en grande partie complémentaire à une quelconque région génomique du sujet et/ou
- a une longueur comprise entre 14 et 50 bases et/ou
- a une température de fusion comprise entre 45 °C et 85 °C et/ou
- a une teneur en GC comprise entre 30 % et 66 %.

6. Procédé selon l'une quelconque des revendications 2-5, dans lequel le lieur polynucléotidique comprend au moins un site de liaison d'amorce d'amplification, de préférence un site de liaison d'amorce d'amplification sens.

7. Procédé selon la revendication 6, dans lequel la séquence de l'amorce d'amplification sens comprend la séquence de nucléotides CCGTAATCGGGAAGCTGAAG (SEQ ID n° : 1).

8. Procédé selon l'une quelconque des revendications 2-7, dans lequel :

- la séquence d'une amorce d'amplification antisens comprend la séquence de nucléotides GCACGATCCGACGGTAGTGT (SEQ ID n° : 2) ; et/ou
- le lieur polynucléotidique comprend la séquence de nucléotides CTTCAGCTTCCCGATTACGGGCACGATCCGACGGTAGTGT (SEQ ID n° : 3) ; et/ou
- le premier bras polynucléotidique de ciblage comprend la séquence de nucléotides CACTACACTCCAACCTAA (SEQ ID n° : 4) ou TTCTCCTACCTCAACCTC (SEQ ID n° : 5) ; et/ou
- le second bras polynucléotidique de ciblage comprend la séquence de nucléotides CAAAAAACTAAAACAAAA (SEQ ID n° : 6) ou CCAAACTAAAATACAATA (SEQ ID n° : 7).

9. Procédé selon l'une quelconque des revendications 1-8, dans lequel la MIP comprend la séquence de nucléotides CACTACACTCCAACCTAA-(N1)-CTTCAGCTTCCCGATTACGGGCACGATCCGACGGTAGTGT-(N2)-CAAAAAACTAAAACAAAA (SEQ ID n° : 8) ou TTCTCCTACCTCAACCTC-(N1)-CTTCAGCTTCCCGATTACGGGCACGATCCGACGGTAGTGT-(N2)-CCAAACTAAAATACAATA (SEQ ID n° : 9), (N1) représentant la séquence de nucléotides du premier marqueur moléculaire unique et (N2) représentant la séquence de nucléotides du second marqueur moléculaire unique.

10. Procédé selon l'une quelconque des revendications 2-9, dans lequel chacun des réplicons des MIP obtenus dans l'étape c) est produit par :

i) l'hybridation des premier et second bras polynucléotidiques de ciblage, respectivement, aux première et seconde régions de répétition présentes dans l'échantillon d'acide nucléique, respectivement, les première et seconde régions de répétitions flanquant une séquence présentant de l'intérêt cible ; et

ii) après l'hybridation, l'utilisation d'un mélange de ligature/extension pour allonger et ligaturer la région de brèche entre les deux bras polynucléotidiques de ciblage pour former des molécules d'acide nucléique circulaire simple brin.

**11.** Procédé selon l'une quelconque des revendications 2-10, dans lequel l'étape de séquençage de d) comprend un procédé de séquençage de nouvelle génération, de préférence un procédé de séquençage massivement parallèle.

**12.** Procédé selon l'une quelconque des revendications 2-11, le procédé comprenant, avant l'étape de séquençage de d), une réaction de PCR pour amplifier les réplicons des MIP pour le séquençage ; la réaction de PCR étant de préférence une réaction de PCR à indexation qui introduit dans chacun des amplicons des MIP les composants suivants : une paire d'adaptateurs de séquençage comprenant un marqueur moléculaire unique pour le séquençage multiplex, de préférence encore, les amplicons des MIP portant des codes à barres comprenant dans l'ordre les composants suivants :
un premier adaptateur de séquençage - un premier site de liaison d'amorce de séquençage - le premier marqueur moléculaire de ciblage unique - le premier bras polynucléotidique de ciblage - l'acide nucléique capturé - le second bras polynucléotidique de ciblage - le second marqueur moléculaire de ciblage unique - un second site de liaison d'amorce de séquençage - un code à barres d'échantillon unique - un second adaptateur de séquençage.

**13.** Procédé selon l'une quelconque des revendications 2-12, ledit procédé comprenant en outre l'obtention d'informations de séquençage à partir de la ou les populations de MIP, dans lequel lesdites informations de séquençage sont de préférence utilisées dans la détermination de l'état de méthylation d'un acide nucléique chez un sujet, ou utilisées dans la détermination du fait de savoir si le sujet a la prédisposition à la maladie ou affection, ou utilisées en diagnostic de la maladie ou affection, ou utilisées en détection du stade de la maladie ou affection, ou utilisées en différenciation des espèces d'acide nucléique provenant du sujet et du ou des individus supplémentaires, ou utilisées en différenciation des espèces d'acide nucléique provenant du premier tissu et du ou des tissus supplémentaires, ou utilisées dans la détermination de l'état de méthylation d'un acide nucléique et la détection d'une variation de nombre de copies chez le sujet, ou utilisées dans la détermination de l'âge de méthylation du sujet, ou utilisées dans la détermination de l'âge de méthylation du tissu, respectivement.

**14.** Molécule d'acide nucléique comprenant une séquence de nucléotides CACTACACTCCAACCTAA-(N1)-CTTCAGCTTCCCGATTACGGGCACGATCCGACGGTAGTGT-(N2)-CAAAAAACTAAAACAAAA (SEQ ID n° : 8) ou TTCTCCTACCTCAACCTC-(N1)-CTTCAGCTTCCCGATTACGGGCACGATCCGACGGTAGTGT-(N2)- CCAAACTAAAATACAATA (SEQ ID n° : 9), dans laquelle (N1) représente une séquence de nucléotides de premier marqueur moléculaire unique et (N2) représente une séquence de nucléotides de second marqueur moléculaire unique.

**15.** Acide nucléique selon la revendication 14, dans lequel la longueur du premier marqueur moléculaire unique est comprise entre 4 et 15 bases et/ou dans lequel la longueur du second marqueur moléculaire unique est comprise entre 4 et 15 bases.

100

FIG. 1

200

```
┌─────────────────────────────────────────┐
│  202 DETERMINE SET OF CONSTRAINTS        │
└─────────────────────────────────────────┘
                    │
                    ▼
┌─────────────────────────────────────────┐
│  204 IDENTIFY AMPLICON SITES USING THE   │
│          SET OF CONSTRAINTS              │
└─────────────────────────────────────────┘
                    │
                    ▼
┌─────────────────────────────────────────┐
│  206 PERFORM OPTIMIZATION TECHNIQUE      │
│   TO MAXIMIZE A NUMBER OF CAPTURED       │
│  CPG SITES, TO MAXIMIZE THE NUMBER OF    │
│ UNIQUE CAPTURE SITES, AND TO MINIMIZE    │
│     A NUMBER OF CPG SITES ON THE         │
│     EXTENSION AND LIGATION ARMS          │
└─────────────────────────────────────────┘
                    │
                    ▼
┌─────────────────────────────────────────┐
│  208 SELECT PROBE BASED ON               │
│       OPTIMIZATION TECHNIQUE             │
└─────────────────────────────────────────┘
```

FIG. 2

300

302 RECEIVE SEQUENCING DATA FOR TEST
SUBJECT, WHERE THE SAMPLE FROM THE
TEST SUBJECT HAS BEEN TREATED WITH
BISULFITE-CONVERSION

304 COMPUTE METHYLATION RATIO FOR
THE TEST SUBJECT

306 RECEIVE METHYLATION RATIOS FOR A
SET OF REFERENCE SUBJECTS

308 PREDICT DISEASE STATE IN TEST
SUBJECT BASED ON COMPARISON OF
METHYLATION RATIO FOR TEST SUBJECT TO
METHYLATION RATIO FOR THE REFERENCE
SUBJECTS

FIG. 3

400

402 RECEIVE SEQUENCING
DATA RECORDED FROM A
SAMPLE THAT WAS TREATED
WITH BISULFITE-
CONVERSION

406 FILTER SEQUENCING
READS TO REMOVE KNOWN
ARTIFACTS

408 ALIGN THE READS TO
THE HUMAN GENOME

412 k=1

414 DETERMINE WHETHER A
C NUCLEOTIDE IS PRESENT AT
CPG SITE k

418
k = k+1

NO

416
k=K?

YES

420 COMPUTE SUM S OF C
NUCLEOTIDES FOR THE
SAMPLE

422 COMPUTE METHYLATION
RATIO S/K FOR THE TEST
SAMPLE

424 SELECT DISEASE STATE
FOR TEST SAMPLE t BY
COMPARING THE
METHYLATION RATIO FOR
THE TEST SAMPLE TO A SET
OF REFERENCE
METHYLATION RATIOS

FIG. 4

/5Phos/CACTACACTCCAACCTAA-Ns-CTTCAGCTTCCCGATTACGGGCACGATCCGACGGTAGTGT-Ns-CAAAAAACTAAAACAAAA

Ligation Arm       6-10N           Backbone          6-10N     Extension
                               (index PCR template)            Arm

**FIG. 5**

/5phos/CACTACACACTCCAACCTAA-Nx-CTTCAGCTTCCCGATTACGGGGCACGATCCGACGGTAGTGT-N8-CAAAAAACTAAAACAAAA

Ligation Arm        6-10N    Backbone (index PCR template)            6-10N      Extension Arm

Bisulfite DNA combined and hybridisation

NCTTCAGCTTCCCGATTACGGGGCACGATCCGACGGTAGTGT

AATCCAACCTCACATCAC -Phos                              AAAACAAAATCAAAAAC

NNNNNNTTAGGTTGGAGTGTAGTGNNNNNNNNNNNNNNNNNNNNTTTTGTTTTAGTTTTTGNNNNNNNNN

Polymerase and Ligase

NCTTCAGCTTCCCGATTACGGGGCACGATCCGACGGTAGTGT

Unique Gap Sequences with CPG sites

AATCCAACCTCACATCAC ⬅━━━━━━━━━━ AAAACAAAATCAAAAAC

NNNNNNTTAGGTTGGAGTGTAGTGNNNNNNNNNNNNNNNNNNNNTTTTGTTTTAGTTTTTGNNNNNNNNN

Captured Probes

FIG. 6

FIG. 7

FIG. 8

FIG. 9

73

## Sample 1

xxxxxxCGxxxxxxCGxxxxxxxxxxxxxxxxCGxxxxxxxxxxxxxxxx

Methylation score = 100 * $\dfrac{\text{Methylated Cs at CpG (25)}}{\text{Methylated Cs at CpG (25) + Unmethylated Cs at CpG (5)}}$

**MI for sample 1 : 83%**

## Sample 2

xxxxxxCGxxxxxxCGxxxxxxxxxxxxxxxxCGxxxxxxxxxxxxxxxx

Methylation score = 100 * $\dfrac{\text{Methylated Cs at CpG (20)}}{\text{Methylated Cs at CpG (20) + Unmethylated Cs at CpG (9)}}$

**MI for sample 2 : 69%**

FIG. 10

m m m
5'...GAGGCTGAGGCAGGAGAATTGCTTGAACCCGGGAGTCGGAGGCTGCAGCAAGCTGAGACCGTGCCACTGCACTCCAGCCTGG...
...CCACGGCTGGAGTCGCAGGTCCTCTCTTAAGCAACTTGGGCCCTCAGCCTCCGACGTCGTTCGACTCTGGCACGGTGACGTGAGGTCGGACC...5'
m m m

⬇ Bisulfite conversion

5'...GAGGUTGAGGUAGGAGAA*TTGUTTGAAUUCGGGAGTCGGAGGUTGUAGUAAGUTGAGAUCGTGUUAUTGUAUTUUAGUUTGG*...

...UUAGGUTGAGGUTGGUGGUTUUTUTTAAGGAAGUTTGGGCGGUUTGAGGUTTGGAGUGTGGTTGGAGUTGTGGTCGGUCGGUGACGGUGAGGUTGGUGACGGUGGTUGGAGUU...5'

**Upper and lower strands are not complementary after bisulfite conversion**

⬇

**Molecular inverted probe (MIP)**

Ligation arm Mol tag backbone Mol tag Extension arm

/5Phos/TTCTCCTACCTCAACCTCNNNNNNCTTCAGCTTCCCGATTACGGGCACGATCCGACGGTAGTGTNNNNNNCCAAACTAAAATACAATA

⬇

**MIP capture: annealing**

...GAGGUTGAGGUAGGAGAA*TTGUTTGAAUUCGGGAGTCGGAGGUTGUAGUAAGUTGAGAUCGTGUUAUTGUAUTUUAGUUTGG*...

CTCCTACCTCAACCTC TTCTCCTACCTCAACCTC (5'Phos) 3'ATAACATTTATTTGG...CC

Ligation arm ⟵ gap ⟶ Extension arm

backbone

NNNNNNCTTCAGCTTCCCGATTACGGGCACGATCCGACGGTAGTGTNNNNNN

Mol tag Mol tag

⬇

**MIP capture: extention/ligation**

...GAGGUTGAGGUAGGAGAA*TTGUTTGAAUUCGGGAGTCGGAGGUTGUAGUAAGUTGAGAUCGTGUUAUTGUAUTUUAGUUTGG*...

CTCCTACCTCAACCTCAACGAATCTACTTACTTCAACTCTAGGCGCCCTCAGCCTCCGACGTCGTTCGACTCTGCTTAGATGAATGAAATCAACC

⟵ Extension

↑

Ligation

NNNNNNCTTCAGCTTCCCGATTACGGGCACGATCCGACGGTAGTGTNNNNNN

**FIG. 11A**

MIP capture : exonuclease

...CTTCAGCTTCCCGATTACGGGCACGATCCGACGGTAGTGT...

...CTTCAGCTTCCCGATTACGGGCACGATCCGACGGTAGTGT...

PCR_Forward_Primer

PCR cycle 1

Forward extension product is the substrate of the reverse indexing primer

PCR_Reverse_Indexing_Primer

Index

PCR cycle 2

PCR product

Ligation arm          Extention arm          Index

Mol Tag          Mol Tag

**FIG. 11B**

Sequencing Read 1 and index

Custom sequencing primer Read 1
CATACGAGATCCGTAATCGGGAAGCTGAAG ⟶ 106 cycles

Custom sequencing primer Index
ACACTACCGTCGGATCGTGCGTGT ⟶ 8 cycles

Mol tag  Ligation arm

Extension arm  Mol tag  index  Flow cell  5'

Paired-end turnaround

Sequencing Read 2

5' Flow cell

106 cycles ⟵

Custom sequencing primer Read 2

**FIG. 12**

<u>1300</u>

```
┌─────────────────────────────────────────────┐
│ 1302 Demultiplexing and FASTQ generation     │
│               (Casava)                        │
└─────────────────────────────────────────────┘
                      │
                      ▼
┌─────────────────────────────────────────────┐
│ 1304 Removing Illumina adapters / MIP         │
│         backbone (Trimmomatic)                │
└─────────────────────────────────────────────┘
                      │
                      ▼
┌─────────────────────────────────────────────┐
│ 1306 Keeping reads if ligation arm and        │
│ extension arm have less than 1 mismatch each  │
│              (custom pipeline)                │
└─────────────────────────────────────────────┘
                      │
                      ▼
┌─────────────────────────────────────────────┐
│ 1308 Trim arm sequences at the end of read 1  │
│           and 2 (custom pipeline)             │
└─────────────────────────────────────────────┘
                      │
                      ▼
┌─────────────────────────────────────────────┐
│ 1310 Bismark alignment (bowtie2)              │
└─────────────────────────────────────────────┘
         │                        │
         │                        ▼
         │          ┌──────────────────────────────┐
         │          │ 1312 UMID condensation        │
         │          └──────────────────────────────┘
         │                        │
         ▼                        ▼
┌─────────────────────────────────────────────┐
│ 1314 Methylation extractor                    │
└─────────────────────────────────────────────┘
```

FIG. 13

FIG. 14

CNAs, Sample 781

FIG. 15A

FIG. 15B

Hypomethylation at CNAs sites

FIG. 16

# Tissue of Origin: Analysis Pathway

FIG. 17

FIG. 18

CpG methylation clustering

FIG. 19

Distance method: "correlation"; Clustering method: "ward"

Samples

EP 3 377 647 B1

FIG. 20

# Gestational Age as Predicted by GMI

| GA | Avg, GMI |
|---|---|
| 0 | 86.5 |
| 70 | 80.4 |
| 80 | 79.6 |
| 90 | 78.8 |
| 180 | 71.6 |
| 270+(birth) | 64.4 |

FIG. 21

EP 3 377 647 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 186636
- US 20070020640 A1
- US 027039
- US 20080269068 A1
- US 6858412 B
- US 5817921 A
- US 6558928 B
- US 7320860 B
- US 7351528 B
- US 5866337 A
- US 6027889 A
- US 6852487 B
- US 7960120 B

- US 7835871 B
- US 7232656 B
- US 7598035 B
- US 6911345 B
- US 6833246 B
- US 6828100 B
- US 6306597 B
- US 6210891 B
- US 20110009278 A
- US 20070114362 A
- US 20060292611 A
- US 20060024681 A

**Non-patent literature cited in the description**

- **MOTULSKY.** Intuitive Biostatistics. Oxford University Press, Inc, 1995
- **LODISH et al.** Molecular Cell Biology. W. H. Freeman & Co, 2000
- **GRIFFITHS et al.** Introduction to Genetic Analysis. W. H. Freeman & Co, 1999
- **GILBERT et al.** Developmental Biology. Sinauer Associates, Inc, 2000
- The McGraw-Hill Dictionary of Chemical Terms. McGraw-Hill, 1985
- **TIMP et al.** *Genome Medicine,* 2014, vol. 6, 61
- **LUO et al.** *BioMed research international,* 2014
- **DING, L. et al.** Somatic mutations affect key pathways in lung adenocarcinoma. *Nature,* 2008, vol. 455, 1069-1075
- **KANDOTH ; CYRIAC et al.** Mutational landscape and significance across 12 major cancer types. *Nature,* 2013, 333-339
- **TURNER E H et al.** *Nat Methods,* April 2009, vol. 6, 1-2
- **HARDENBOL et al.** Multiplexed genotyping with sequence-tagged molecular inversion probes. *Nature Biotechnology,* 2003, vol. 21 (6), 673-678
- **HARDENBOL et al.** Highly multiplexed molecular inversion probe genotyping: Over 10,000 targeted SNPs genotyped in a single tube assay. *Genome Research,* 2005, vol. 15, 269-275
- **BURMESTER et al.** DMET microarray technology for pharmacogenomics-based personalized medicine. *Methods in Molecular Biology,* 2010, vol. 632, 99-124

- **SISSUNG et al.** Clinical pharmacology and pharmacogenetics in a genomics era: the DMET platform. *Pharmacogenomics,* 2010, vol. 11 (1), 89-103
- **DEEKEN.** The Affymetrix DMET platform and pharmacogenetics in drug development. *Current Opinion in Molecular Therapeutics,* 2009, vol. 11 (3), 260-268
- **WANG et al.** High quality copy number and genotype data from FFPE samples using Molecular Inversion Probe (MIP) microarrays. *BMC Medical Genomics,* 2009, vol. 2, 8
- **WANG et al.** Analysis of molecular inversion probe performance for allele copy number determination. *Genome Biology,* 2007, vol. 8 (11), R246
- **JI et al.** Molecular inversion probe analysis of gene copy alternations reveals distinct categories of colorectal carcinoma. *Cancer Research,* 2006, vol. 66 (16), 7910-7919
- **WANG et al.** Allele quantification using molecular inversion probes (MIP). *Nucleic Acids Research,* 2005, vol. 33 (21), e183
- **BREWSTER et al.** Copy number imbalances between screen- and symptom-detected breast cancers and impact on disease-free survival. *Cancer Prevention Research,* 2011, vol. 4 (10), 1609-1616
- **GEIERSBACH et al.** Unknown partner for USP6 and unusual SS18 rearrangement detected by fluorescence in situ hybridization in a solid aneurysmal bone cyst. *Cancer Genetics,* 2011, vol. 204 (4), 195-202

- **SCHIFFMAN et al.** Oncogenic BRAF mutation with CDKN2A inactivation is characteristic of a subset of pediatric malignant astrocytomas. *Cancer Research,* 2010, vol. 70 (2), 512-519
- **SCHIFFMAN et al.** Molecular inversion probes reveal patterns of 9p21 deletion and copy number aberrations in childhood leukemia. *Cancer Genetics and Cytogenetics,* 2009, vol. 193 (1), 9-18
- **PRESS et al.** Ovarian carcinomas with genetic and epigenetic BRCA1 loss have distinct molecular abnormalities. *BMC Cancer,* 2008, vol. 8, 17
- **DEEKEN et al.** A pharmacogenetic study of docetaxel and thalidomide in patients with castration-resistant prostate cancer using the DMET genotyping platform. *Pharmacogenomics,* 2009, vol. 10 (3), 191-199
- **CALDWELL et al.** CYP4F2 genetic variant alters required warfarin dose. *Blood,* 2008, vol. 111 (8), 4106-4112
- **MCDONALD et al.** CYP4F2 Is a Vitamin K1 Oxidase: An Explanation for Altered Warfarin Dose in Carriers of the V433M Variant. *Molecular Pharmacology,* 2009, vol. 75, 1337-1346
- **MEGA et al.** Cytochrome P-450 Polymorphisms and Response to Clopidogrel. *New England Journal of Medicine,* 2009, vol. 360 (4), 354-362
- **DUMAUAL et al.** Comprehensive assessment of metabolic enzyme and transporter genes using the Affymetrix Targeted Genotyping System. *Pharmacogenomics,* 2007, vol. 8 (3), 293-305
- **DALY et al.** Multiplex assay for comprehensive genotyping of genes involved in drug metabolism, excretion, and transport. *Clinical Chemistry,* 2007, vol. 53 (7), 1222-1230
- **MAN et al.** Genetic Variation in Metabolizing Enzyme and Transporter Genes: Comprehensive Assessment in 3 Major East Asian Subpopulations With Comparison to Caucasians and Africans. *Journal of Clinical Pharmacology,* 2010, vol. 50 (8), 929-940
- Microarrays: A Practical Approach. IRL Press, 2000
- **ALTUNA AKALIN ; MATTHIAS KORMAKSSON ; SHENG LI ; FRANCINE E ; GARRETT-BAKELMAN, MARIA E ; FIGUEROA, ARI MELNICK ; CHRISTOPHER E. MASON.** methylKit: A comprehensive R package for the analysis of genome-wide DNA methylation profiles. *Genome Biology,* 2012, vol. 13, R87
- **RIZVI et al.** Mutational landscape determines sensitivity to PD-1 blockade in non-small cell lung cancer. *Science,* 03 April 2015, vol. 348 (6230), 124-128
- **BOLGER, A. M. ; LOHSE, M. ; USADEL, B.** Trimmomatic: A flexible trimmer for Illumina Sequence Data. *Bioinformatics,* 2014, vol. 1304
- **LI H.* ; HANDSAKER B.* ; WYSOKER A. ; FENNELL T. ; RUAN J. ; HOMER N. ; MARTH G. ; ABECASIS G.** Durbin R. and 1000 Genome Project Data Processing Subgroup (2009) The Sequence alignment/map (SAM) format and SAMtools. *Bioinformatics,* vol. 25, 2078-9
- **LANGMEAD B ; SALZBERG S.** Fast gapped-read alignment with Bowtie 2. *Nature Methods,* 2012, vol. 9, 357-359
- **KOH et al.** *PNAS,* 2014, vol. 111 (20), 7361-7366
- **SUN et al.** *PNAS,* 2015, vol. 112 (40